# EUROPEAN PATENT SPECIFICATION

(11) **EP 2 875 078 B1**
(45) Date of publication and mention of the grant of the patent: **20.12.2017**
(21) Application number: 13739595.0
(22) Date of filing: 12.07.2013
(51) Int. Cl.: C09D 5/16, B08B 17/00, B05D 3/10, B05D 5/08, A61L 29/08, A61L 33/00, A61L 29/16, A61L 31/08, A61L 31/16, C08L 83/00

(54) **MODIFICATION OF SURFACES FOR SIMULATANEOUS REPELLENCY AND TARGETED BINDING OF DESIRED MOIETIES**
MODIFIZIERUNG VON OBERFLÄCHEN ZUR GLEICHZEITIGEN ABWEISUNG UND GEZIELTEN BINDUNG GEWÜNSCHTER GRUPPEN
MODIFICATION DE SURFACES POUR UNE RÉPULSION ET UNE LIAISON CIBLÉE SIMULTANÉES DE FRACTIONS DÉSIRÉES

(30) Priority: 18.07.2012 US 201261673071 P; 18.07.2012 US 201261673154 P; 30.05.2013 US 201361829068 P
(43) Date of publication of application: 27.05.2015
(73) Proprietor: President and Fellows of Harvard College, Cambridge, MA 02138 (US)
(72) Inventor: INGBER, Donald, Boston, MA 02118 (US); SUPER, Michael, Lexington, MA (US); LESLIE, Daniel, C., Brookline, MA 02445 (US); DIDAR, Tohid, Brighton, MA 02135 (US); WATTERS, Alexander, L., Melrose, MA 02176 (US); BERTHET, Julia, Bellows, Brookline, MA 02446 (US); WATERHOUSE, Anna, Brookline, MA 02446 (US)
(74) Representative: HGF Limited
(86) International application number: PCT/US2013/050405
(87) International publication number: WO 2014/014788

(56) References cited:
- EP-A2- 0 166 998
- EP-A2- 0 497 204
- WO-A2-2011/090954
- WO-A2-2011/091037
- US-A- 4 678 660
- US-A1- 2007 166 344
- US-A1- 2010 174 351
- TAK-SING WONG ET AL: "Bioinspired self-repairing slippery surfaces with pressure-stable omniphobicity", NATURE, vol. 477, no. 7365, 1 January 2011 (2011-01-01), pages 443-447, XP055025132, ISSN: 0028-0836, DOI: 10.1038/nature10447

## Description

### CROSS-REFERENCES TO RELATED APPLICATIONS

This application claims the benefit of the earlier filing date of U.S. Patent Application No. 61/673, 154, filed on July 18,2012; U.S. Patent Application No. 61/673,071, filed on July 18, 2012; and U.S. Patent Application No. 61/829,068, filed on May 30, 2013, the contents of which are incorporated by reference herein in their entireties.

### STATEMENT CONCERNING GOVERNMENT RIGHTS IN

### FEDERALLY-SPONSORED RESEARCH

This invention was made with government support under Grant No. NS073474 awarded by the National Institutes of Health and under Grant No. N66001-11-1-4180 awarded by the U.S. Department of Defense/DARPA. The government has certain rights in this invention.

### TECHNICAL FIELD

The present disclosure relates generally to surfaces that are able to reduce friction, adhesion, adsorption, and deposition from liquids, semi-solids, and solids while simultaneously allowing targeted binding of desired moieties. In some embodiments, the surfaces, devices, and methods described herein are designed to be portable and disposable.

### BACKGROUND

Articles that allow targeted binding of desired moieties on surfaces such as microbe-targeting molecule or a microbe-binding molecule are known. For example, a microbe-targeting molecule is engineered by fusing the carbohydrate recognition domain and neck region of a carbohydrate-binding protein (*e.g*., mannose-binding lectin) to the C-terminal of a Fc fragment of human IgG1. Further, the microbe-targeting molecules can be engineered, *e.g*., by inserting an AKT tripeptide to the N-terminal of the Fc fragment for site-specific biotinylation, such that their carbohydrate recognition domains orient away from the substrate to which they attach, thus increasing the microbe-binding capacity. The microbe-targeting molecules can be attached to various substrates, *e.g*., a magnetic microbead. Non-leaching anti-microbial coating compositions suitable for surfaces of medical devices is known (for example US 2007/0166344 A1).

However, many of these techniques suffer from poor signal-to-noise ratio in selectively binding only the desired moieties while repelling the undesired moieties. Moreover, there has been limited success in developing such diagnostic tools having good signal-to-noise ratios.

Moreover, sepsis is a major cause of morbidity and mortality in humans and other animals. In the United States, sepsis is the second leading cause of death in intensive care units among patients with non-traumatic illnesses. It is also the leading cause of death in young livestock, affecting 7.5-29% of neonatal calves, and is a common medical problem in neonatal foals. Despite the major advances of the past several decades in the treatment of serious infections, the incidence and mortality due to sepsis continues to rise.

Sepsis results from the systemic invasion of microorganisms into blood and can present two distinct problems. First, the growth of the microorganisms can directly damage tissues, organs, and vascular function. Second, toxic components of the microorganisms can lead to rapid systemic inflammatory responses that can quickly damage vital organs and lead to circulatory collapse (*i.e.,* septic shock) and, often times, death.

Sepsis is a systemic reaction characterized by arterial hypotension, metabolic acidosis, decreased systemic vascular resistance, tachypnea and organ dysfunction. Sepsis can result from septicemia (*i.e.,* organisms, their metabolic end-products or toxins in the blood stream), bacteremia (*i.e.,* bacteria in the blood), toxemia (*i.e.,* toxins in the blood), endotoxemia (*i.e.,* endotoxin in the blood). Sepsis can also result from fungemia (*i.e.,* fungi in the blood), viremia (*i.e.,* viruses or virus particles in the blood), and parasitemia (*i.e.,* helminthic or protozoan parasites in the blood). Thus, septicemia and septic shock (acute circulatory failure resulting from septicemia often associated with multiple organ failure and a high mortality rate) may be caused by various microorganisms.

There are three major types of sepsis characterized by the type of infecting organism. For example, gram-negative sepsis is the most common type (with a case fatality rate of about 35%). The majority of these infections are caused by Escherichia coli, Klebsiella pneumoniae and Pseudomonas aeruginosa. Gram-positive pathogens such as the Staphylococci and Streptococci are the second major cause of sepsis. The third major group includes fungi, with fungal infections causing a relatively small percentage of sepsis cases, but with a high mortality rate; these types of infections also have a higher incidence in immunocomprised patients.

Some of these infections can be acquired in a hospital setting and can result from certain types of surgery (*e.g.,* abdominal procedures), immune suppression due to cancer or transplantation therapy, immune deficiency diseases, and exposure through intravenous catheters. Sepsis is also commonly caused by trauma, difficult newborn deliveries, and intestinal torsion (especially in dogs and horses). Infections in the lungs (pneumonia), bladder and kidneys (urinary tract infections), skin (cellulitis), abdomen (such as appendicitis), and other areas (such as meningitis) can spread and also lead to sepsis. In some circumstances, ingestion of microbe-contaminated water, fluid or food, or contact with microbe-covered environmental surfaces can cause infections that lead to sepsis.

Many patients with septicemia or suspected septicemia exhibit a rapid decline over a 24-48 hour period. Thus, rapid and reliable treatment methods are essential for effective patient care. Hence, there remains a strong need for development of portable and efficient devices for treating infectious diseases, blood-borne infections, sepsis, or systemic inflammatory response syndrome. The ability of a portable device to remove infectious pathogens in food, water, and to remove microbes from a test sample would also have great value for preventing infections and sepsis in the population, and also facilitating detection and diagnosis of a microbial infection or contamination.

### SUMMARY

In accordance with the presentinvention, an article is described that includes a substrate; anchoring molecules comprising a head group attached to the substrate and a tail group directly or indirectly attached to the head group, wherein the tail group has an affinity with a lubricating liquid; a lubricating liquid layer immobilized over the substrate surface comprising said lubricating liquid having an affinity with the tail group of said anchoring molecules, wherein the anchoring molecules and the lubricating liquid are held together by non-covalent attractive forces; and a binding group directly or indirectly secured to the substrate and extending over the surface of the lubricating layer or retained within the lubricating layer having an affinity with a target moiety; wherein the anchoring molecules and the lubricating layer form a slippery surface configured and arranged for contact with a material that is immiscible with the lubricating liquid, wherein said slippery surface resists adhesion and repels the material that is immiscible with the lubricating liquid but selectively binds the target moiety to the binding group.

In accordance with certain embodiments, a method of preventing adhesion, adsorption, surface-mediated clot formation, or coagulation of a material while selectively binding a target moiety thereon is disclosed. The method includes providing the above described slippery surface wherein the anchoring molecules and the lubricating layer form a slippery surface configured and arranged for contact with a material that is immiscible with the lubricating liquid; and contacting said immiscible material to the slippery surface and selectively binding said target moiety to the binding group.

In accordance with certain embodiments, a method of making an article having the above described slippery surface that selectively binds a target moiety is disclosed.

In accordance with certain embodiments, a device for capturing a microbe and/or microbial matter is disclosed. The device includes a chamber with an inlet and an outlet; and a slippery articles described herein.

In accordance with certain embodiments, a shunt system for capturing a microbe is disclosed. The system includes: a shunt having a first and second end; a hollow passageway extending between the first and the second end; and at least one device for capturing a microbe and/or microbial matter disposed in the hollow passageway.

In accordance with certain embodiments a diagnostic device is disclosed. The diagnostic device includes a substrate; anchoring molecules comprising a head group attached to the substrate and a tail group directly or indirectly attached to the head group, wherein the tail group has an affinity with a lubricating liquid; a lubricating layer immobilized over the substrate surface comprising said lubricating liquid having an affinity with the tail group of said anchoring molecules, wherein the anchoring molecules and the lubricating liquid are held together by non-covalent attractive forces, areas of said substrate comprising a binding group directly or indirectly secured to the substrate and extending over the surface of the lubricating layer or retained within the lubricating layer having an affinity with a target moiety; wherein the anchoring molecules and the lubricating layer form a slippery surface configured and arranged for contact with a material that is immiscible with the lubricating liquid; wherein said diagnostic tool provides a high signal-to-noise ratio for capture of the target moieties over other non-target moieties.

Embodiments described herein relate to microbe-binding devices for capturing a microbe and/or microbial matter present in a bodily fluid, e.g., blood, a fluid obtained from an environmental source, e.g., a pond, a river or a reservoir, or a test sample. In one aspect, provided herein is a device for capturing a microbe and/or microbial matter comprising (i) a chamber with an inlet and an outlet, (ii) at least one capture element disposed in the chamber between the inlet and outlet, wherein the capture element has on its surface at least one microbe-binding molecule described herein (e.g., FcMBL). An exemplary capture element can include, but is not limited to, a mixing element (e.g., a static mixer or a spiral mixer).

In some embodiments, the surface of the capture element can be modified to reduce non-specific binding of a microbe and/or microbial matter on the capture element material surface. In one embodiment, the surface of the capture element can be coated with a combination of perflurocarbon-containing silanes and amino-containing silanes. In another embodiment, the surface of the capture element can be modified to create a slippery liquid-infused porous surface (SLIPS). In such embodiments, a fluid such as blood can flow through the device in the absence of anticoagulant without blood adhesion or coagulation.

The devices described herein can be used in any applications where removal of a microbe is described. In some embodiments, the devices can be designed for use with a shunt for cleansing blood of a patient with a microbial infection. The devices can be designed to be portable such that they can be used in emergency conditions. In other embodiments, the devices can be used for removal of a microbe from an environmental source (e.g., a pond or a reservoir). In some embodiments, the devices can be integrated into a system for capturing and/or detecting a microbe for diagnostic applications.

### BRIEF DESCRIPTION OF THE DRAWINGS

The following figures are provided for the purpose of illustration only and are not intended to be limiting.
**FIGS. 1A** and **1B** are schematics of ultra-slippery surface with a reservoir, which replenishes the surface with lubricating liquid, housed below the repellant surface, having selective binding molecules formed over the lubricating liquid layer in accordance with certain embodiments;
**FIGS. 1C** through **1H** are schematics of patterned surface having ultra-slippery regions and binding regions that can bind desired target molecules in accordance with certain embodiments;
**FIG. 11** is a schematic of an ultra-slippery surface with a reservoir, which replenishes the surface with lubricating liquid, housed below the repellant surface, having selective binding molecules formed over the lubricating liquid layer in accordance with certain embodiments;

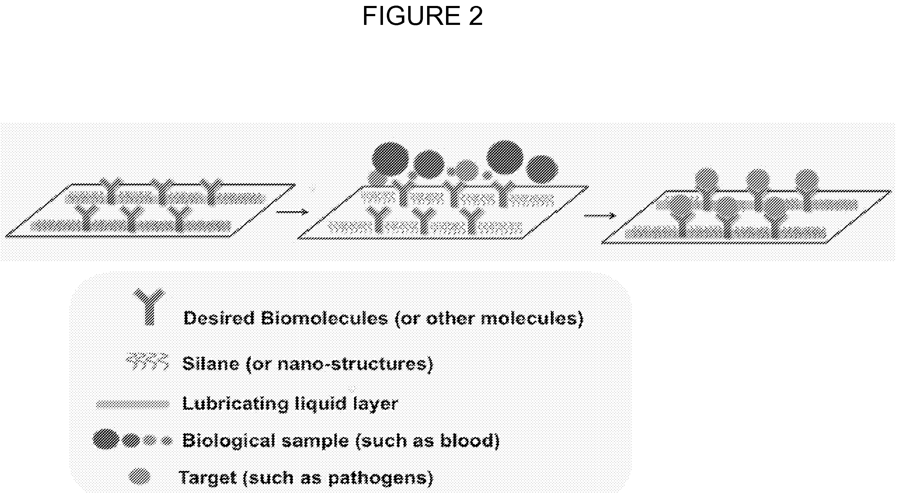

**FIG. 2** schematically illustrates the deposition of anchoring molecules in the slippery regions in accordance with certain embodiments;

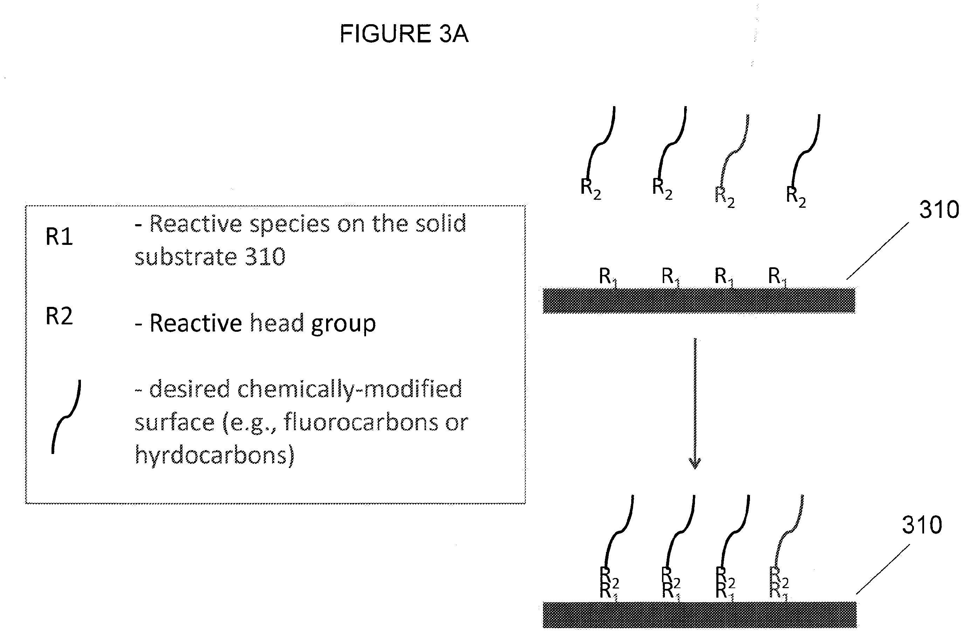

**FIGS. 3A** through **3D** show exemplary schematics of cross-linking chemistries that can be used provide anchoring molecules and/or binding molecules onto a solid substrate in accordance with certain embodiments;

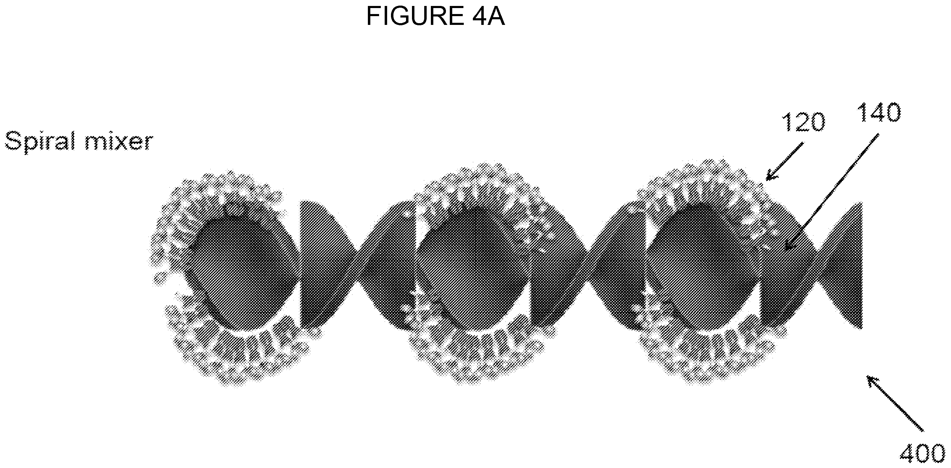

**FIG. 4A** is a diagrammatic view of a solid substrate capture element (e.g., a spiral mixer) with its surface treated to become a slippery liquid-infused porous surface (SLIPS) and coated with a plurality of microbe-binding molecules (e.g., FcMBL molecules) in accordance with certain embodiments;

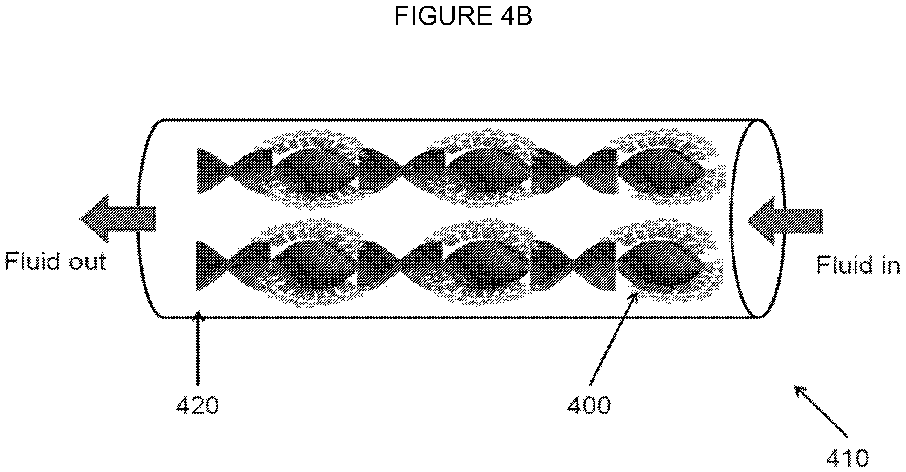

**FIG. 4B** shows diagrammatic view of a shunt device comprising one or more microbe-binding devices disposed therein, wherein the microbe-binding devices are oriented in parallel to flow direction of a fluid in accordance with certain embodiments;

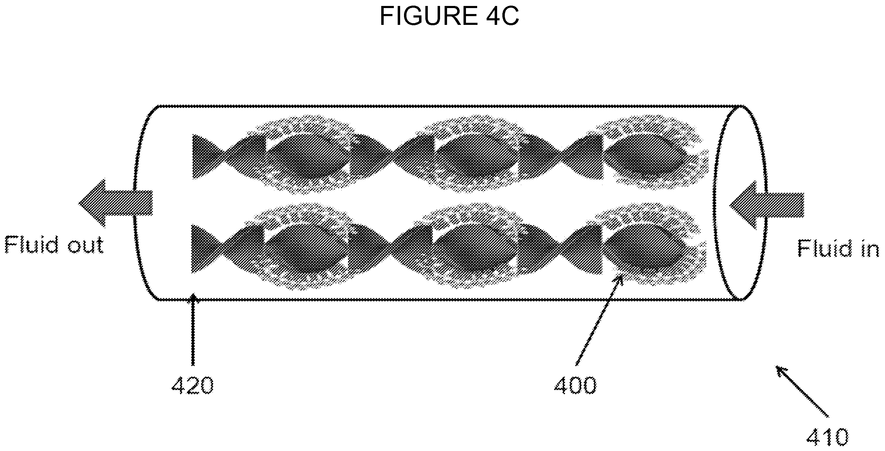

**FIG. 4C** shows another diagrammatic view of a shunt device comprising a plurality of microbe-binding devices disposed therein, wherein the microbe-binding devices are oriented transverse to flow direction of a fluid in accordance with certain embodiments;

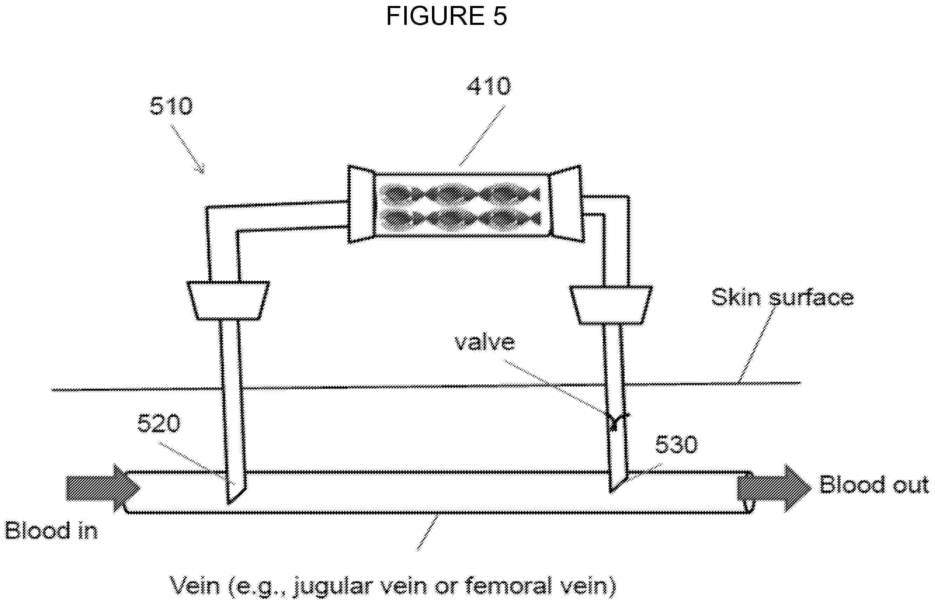

**FIG. 5** **is a** diagrammatic illustration to show a shunt device externally connected to a vein (e.g., **a jugular** vein or femoral vein) of a patient with an infectious disease, e.g., sepsis. Infected blood flows into the shunt device, where microbes in the blood bind to microbe-binding molecules coated on the microbe-binding device and filtered blood then returns to the patient's vein in accordance with certain embodiments;

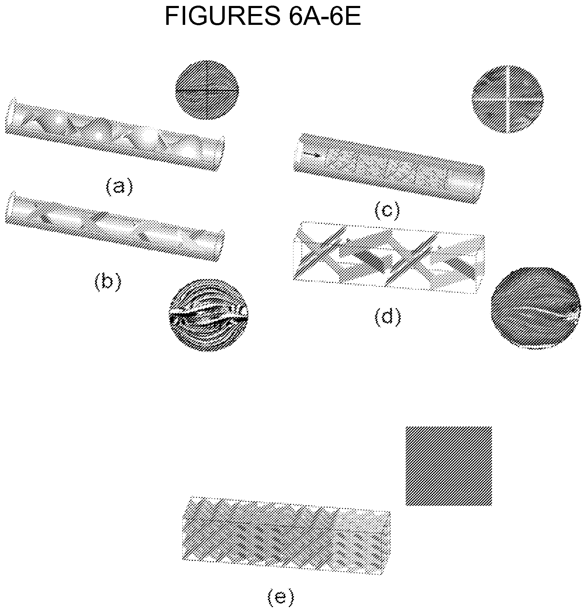

**FIGS. 6A-6E** are diagrammatic illustrations of different static mixers that can be used in various embodiments described herein in accordance with certain embodiments;

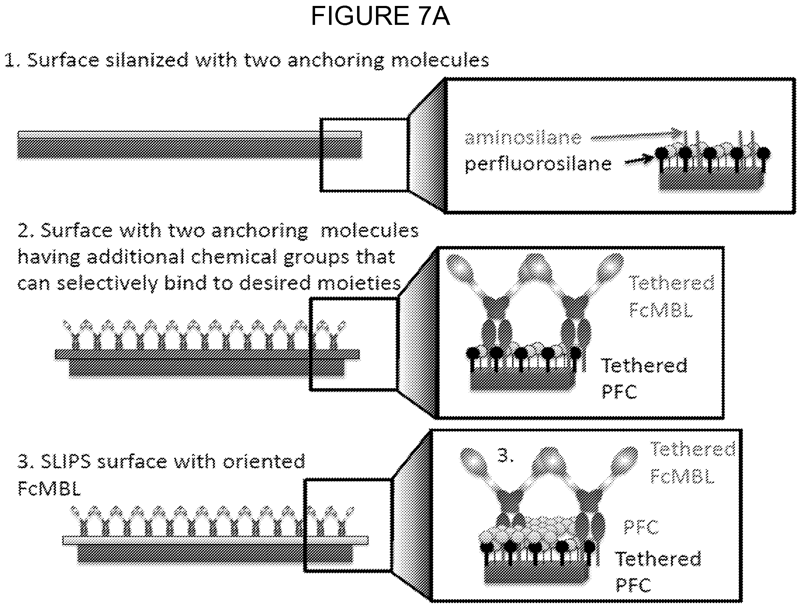

**FIG. 7A** shows a schematic of the surfaces that can simultaneously function as an ultra-slippery surface and selectively bind to a desired moiety in accordance with certain embodiments;

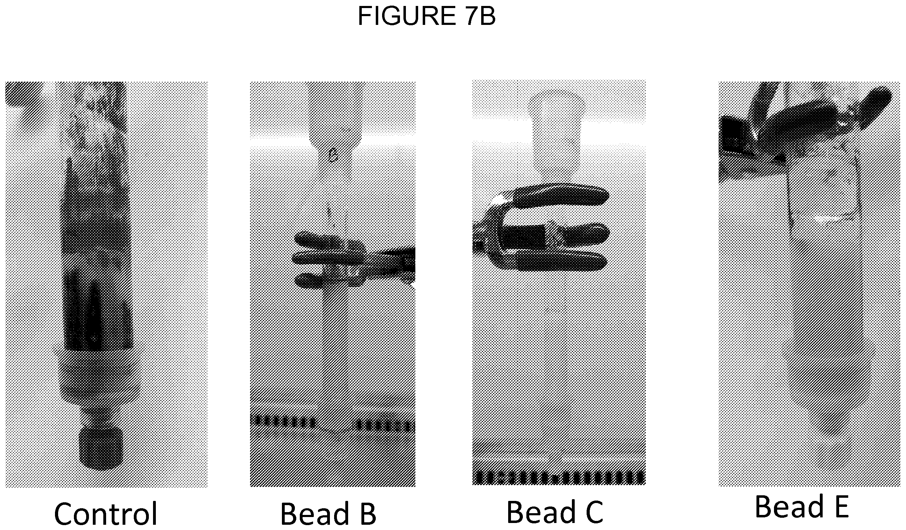

**FIG. 7B** shows a series of images which shows that different glass beads that can prevent adhesion and adsorption of blood with greater efficacy with increasing functionalized amounts of perfluorocarbon silanes relative to aminosilanes. Glass E has the highest ration of perfluorocarbon silanes relative to aminosilanes in accordance with certain embodiments;

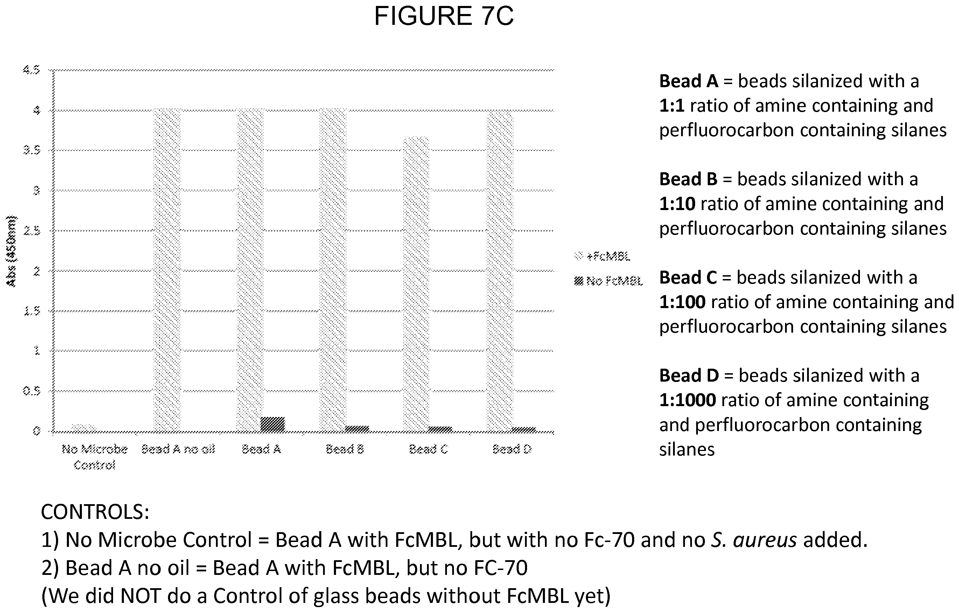

**FIG. 7C** shows the absorbance spectra when the different beads are further functionalized with FcMBL through the aminosilanes and introduced to *S. aureus,* demonstrating that the surfaces can simultaneously function as an ultra-slippery surface and selectively bind to a desired moiety in accordance with certain embodiments;

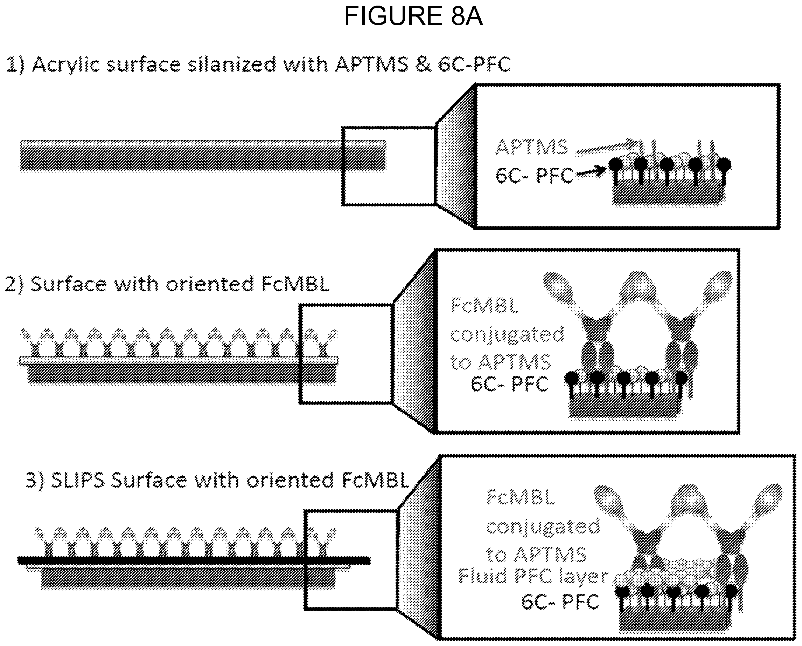

**FIG. 8A** shows a schematic of the surfaces that can simultaneously function as an ultra-slippery surface and selectively bind to a desired moiety in accordance with certain embodiments;

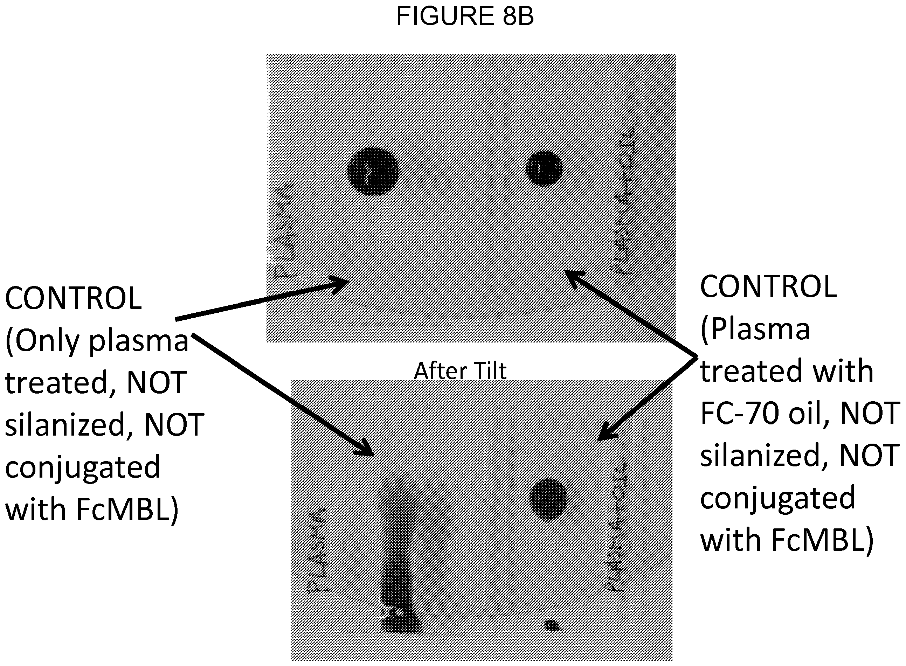

**FIGS. 8B** to **8E** show images of acrylic surfaces before and after blood has been applied on the surface and tilted to demonstrate slippery characteristics of surfaces that can simultaneously function as an ultra-slippery surface and selectively bind to a desired moiety in accordance with certain embodiments;

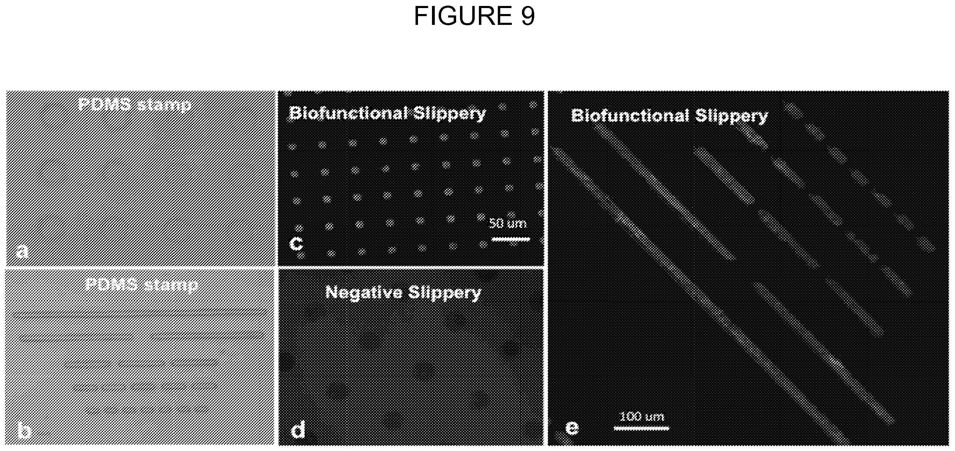

**FIGS. 9A** and **9B** show optical microscope images of the fabricated PDMS stamps for use in creating patterned ultra-slippery surfaces in accordance with certain embodiments;
**FIGS. 9C** to **9E** show images of patterned ultra-slippery surfaces that have been produced using certain stamps in accordance with certain embodiments;

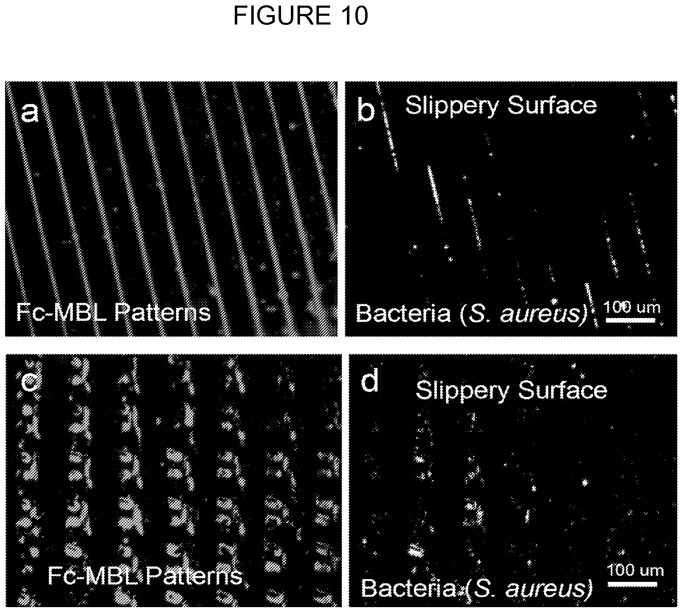

**FIGS. 10A** to **10D** show capture and detection of S. aureus on bio-functional slippery surfaces from whole blood in accordance with certain embodiments;

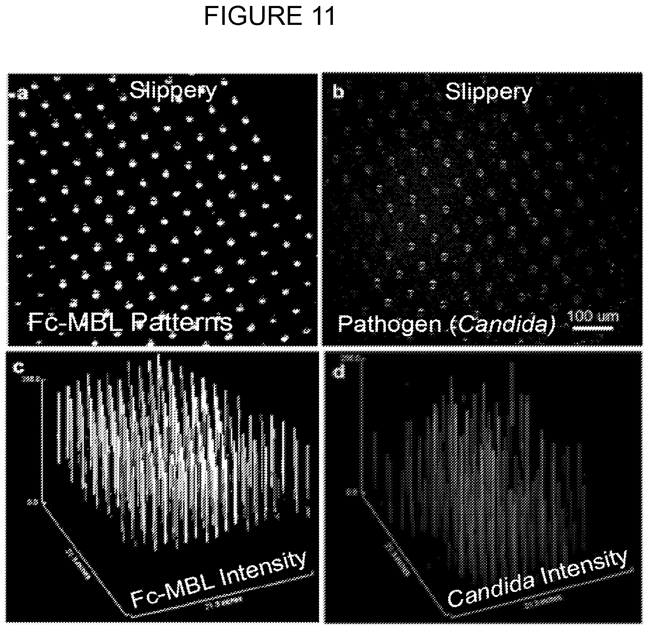

**FIGS. 11A** to **11D** show capture and detection of Candida on bio-functional slippery surfaces from whole blood in accordance with certain embodiments;

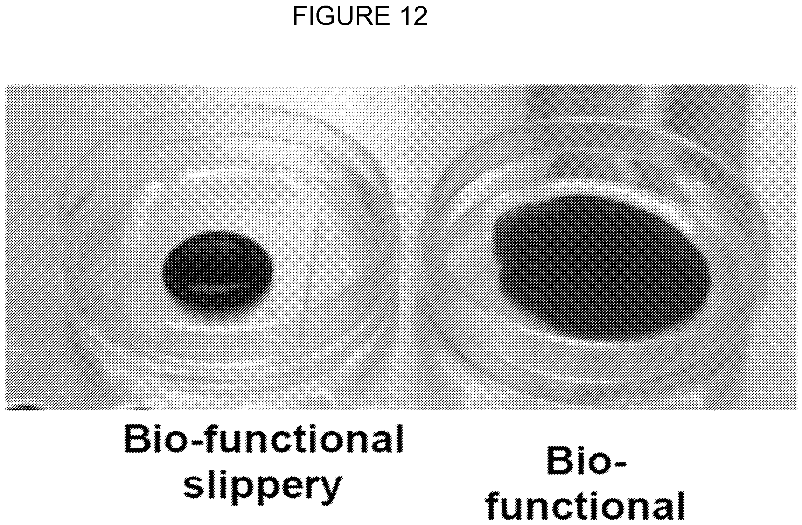

**FIG. 12** shows images of pathogen capture from whole blood on bio-functional slippery surface versus bio-functional surface in accordance with certain embodiments;

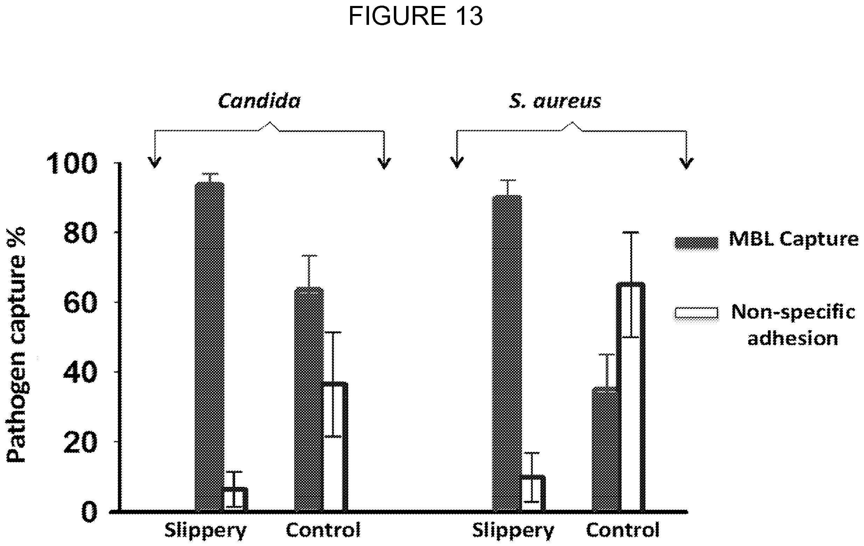

**FIG. 13** shows a graph comparing the non-specific adhesion/capture of pathogens on bio-functional slippery surfaces versus bio-functional (control) surfaces in accordance with certain embodiments;

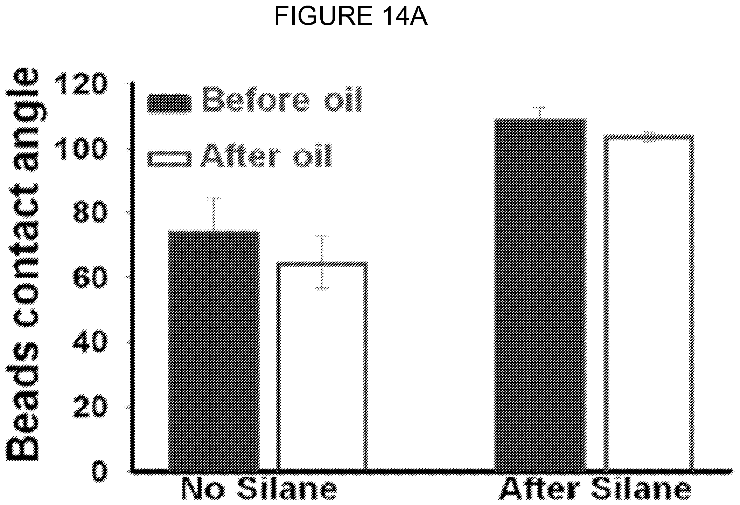

**FIG. 14A** shows a graph of contact angle measurement of surfaces produced with and without silanization in accordance with certain embodiments;

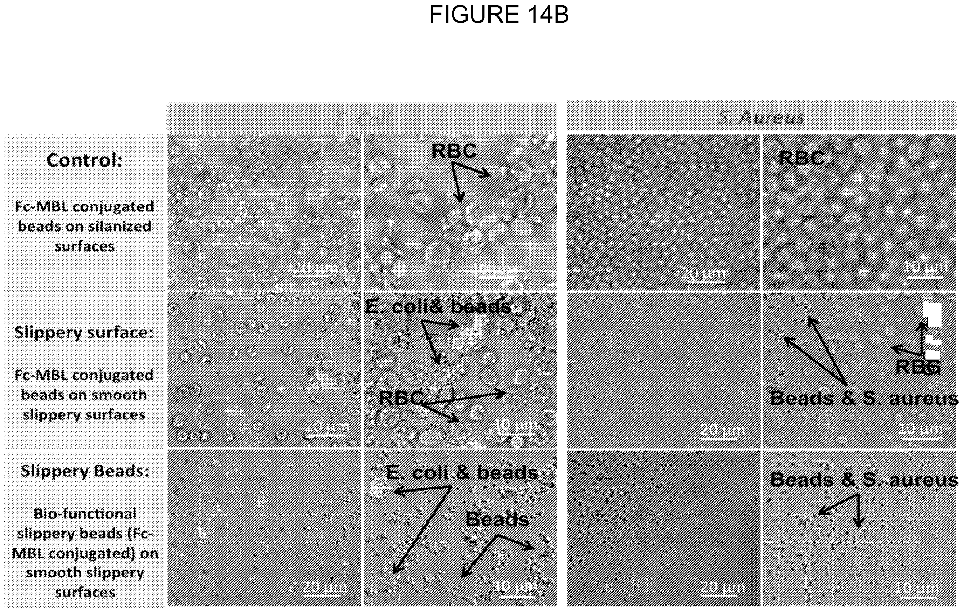

**FIG. 14B** shows the fluorescent microscope images of the produced surfaces after exposure to E. coli (left columns) and S. aureus (right columns) in accordance with certain embodiments; and

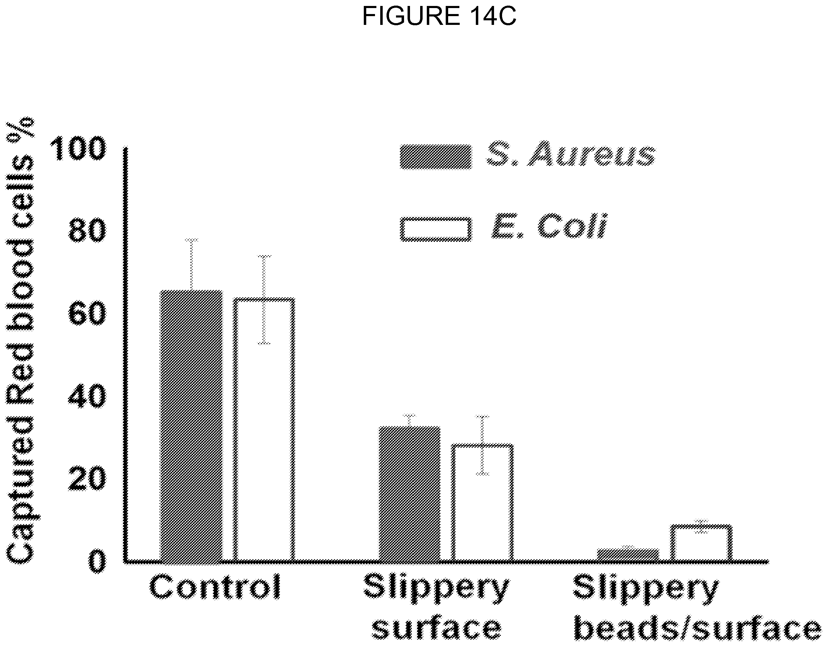

**FIG. 14C** shows a graph comparing the percentage of captured red blood cells for S. aureus and E. coli for the three different surfaces in accordance with certain embodiments.

### DETAILED DESCRIPTION

Methods for making most solid surfaces ultra-repellant to liquids, molecules or particulates contained within liquids, and dry solids are described. However, the ultra-repellant surface can selectively bind any desired target molecules while remaining ultra-repellant to other materials. A lubricating liquid is locked onto and/or into a substrate to form a lubricating layer over the substrate. A binding molecule is provided over or in the lubricating layer to selectively bind a desired target molecule while the surface remains ultra-slippery to other moieties.

### Definitions

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments of the aspects described herein, and are not intended to limit the claimed invention, because the scope of the invention is limited only by the claims. Further, unless otherwise required by context, singular terms shall include pluralities and plural terms shall include the singular.

As used herein, a "subject" means a human or animal. Usually the animal is a vertebrate such as a primate, rodent, domestic animal or game animal. Primates include chimpanzees, cynomologous monkeys, spider monkeys, and macaques, *e.g*., Rhesus. Rodents include mice, rats, woodchucks, ferrets, rabbits and hamsters. Domestic and game animals include cows, horses, pigs, deer, bison, buffalo, feline species, *e.g*., domestic cat, canine species, *e.g*., dog, fox, wolf, avian species, *e.g*., chicken, emu, ostrich, and fish, *e.g*., trout, catfish and salmon. Patient or subject includes any subset of the foregoing, *e.g.,* all of the above, but excluding one or more groups or species such as humans, primates or rodents. In certain embodiments of the aspects described herein, the subject is a mammal, *e.g*., a primate, *e.g.,* a human. The terms, "patient" and "subject" are used interchangeably herein.

In some embodiments, the subject is a mammal. The mammal can be a human, non-human primate, mouse, rat, dog, cat, horse, or cow, but are not limited to these examples. Mammals other than humans can be advantageously used as subjects that represent animal models of disorders.

A subject can be one who has been previously diagnosed with or identified as suffering from or having a disease or disorder caused by any microbes or pathogens described herein. By way of example only, a subject can be diagnosed with sepsis, inflammatory diseases, or infections.

The term "antibody" as used herein refers to immunoglobulin molecules and immunologically active portions of immunoglobulin molecules (molecules that contain an antigen binding site which specifically binds an antigen), including monoclonal antibodies (including full length monoclonal antibodies), polyclonal antibodies, multispecific antibodies (for example, bispecific antibodies), chimeric antibodies, humanized antibodies, human antibodies, and single chain antibodies (scFvs).

The term "peptide" refers to a polymer of amino acids, or amino acid analogs, regardless of its size or function. In some embodiments, the term "peptide" refers to small polypeptides, *e.g*., a polymer of about 15-25 amino acids.

The term "oligonucleotide" as used herein refers to a short nucleic acid polymer, typically with twenty or fewer bases.

"Functional group" as used herein to indicate that the group has a certain desired characteristics, such as chemical reactivity to other groups, affinity to other molecules or compounds, desired polarity, desired charge, repellency of other molecules or compounds, and the like.

"Binding group" as used herein to indicate that the group has a certain desired characteristics, such as physical/chemical reactivity to desired target moieties, affinity to desired target moieties, and the like.

"Anchoring molecules" as used herein to indicate that the group has a certain desired characteristics, such as affinity with the lubricating liquid. Moreover, anchoring molecules can have a functional group that can anchor itself onto the solid substrate. As used herein, "anchor" need not mean complete immobilization but can include physical adsorption/absorption, chemical reactivity, electrostatic attraction, and the like to the solid substrate.

The term "microbial matter" as used herein refers to any matter or component that is derived, originated or secreted from a microbe. For example, microbial matter or a component derived or secreted from a microbe that can bind to an engineered microbe-targeting or microbe-binding molecule can include, but are not limited to, a cell wall component, an outer membrane, a plasma membrane, a ribosome, a microbial capsule, a pili or flagella, any fragments of the aforementioned microbial components, any nucleic acid (*e.g.,* DNA, including 16S ribosomal DNA, and RNA) derived from a microbe, and microbial endotoxin (*e.g.,* lipopolysaccharide). In addition, microbial matter can encompass non-viable microbial matter that can cause an adverse effect (e.g., toxicity) to a host or an environment

### Overview

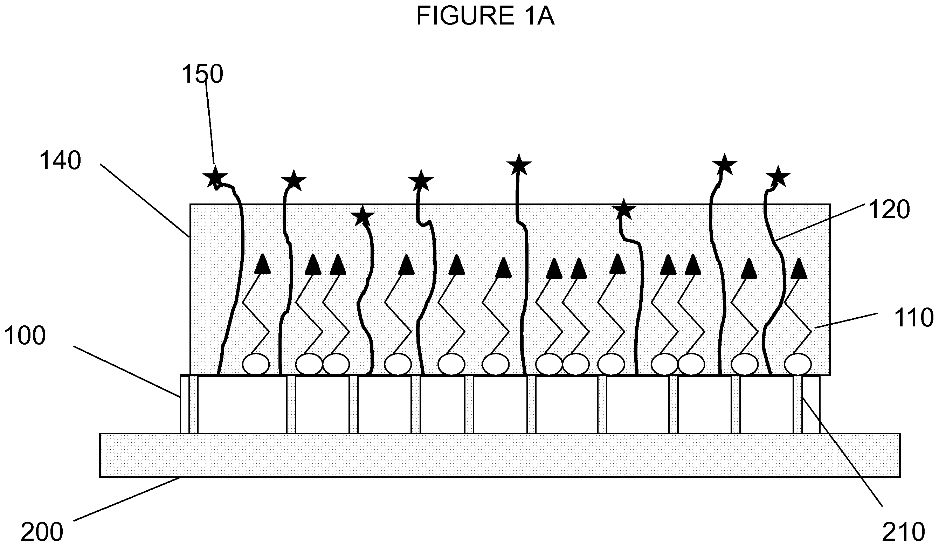

Many different methods to form such ultra-repellent surface that have specific binding properties to target molecules can be formed. In certain embodiments, a mixture of anchoring molecules that attract a lubricating liquid can be provided binding molecules. Referring to **FIG. 1A****,** two different type of anchoring and binding molecules can be provided over a solid surface **100.** The solid surface **100** may be smooth or roughened. Anchoring molecules **110** are provided over the solid substrate surface **100** that provides a chemical affinity toward a lubricating liquid to form a lubricating layer **140.** Binding molecules **120** are provided that have binding groups **150** that can selectively bind to certain target molecules. As shown in **FIG. 1A****,** the binding group **150** extends over the lubricating layer **140.**

The lubricating layer **140,** which has an affinity for the anchoring molecules **110** (and also possibly binding molecules **120)** can be formed by immobilizing the anchoring molecules **110** on the surface **100** and applying a lubricating liquid to the surface containing the immobilized anchoring molecules **110.** The surface layer **110** and lubricating layer **140** are held together by non-covalent attractive forces. Together, the solid substrate and lubricating layer **140** on the solid substrate form a slippery surface that resists adhesion by molecules and particles, and repels certain immiscible fluids. This allows the passage of materials at high flow rates without allowing the material to adhere to, attach, foul the surface, or, in the case of biological fluids such as blood, coagulate. However, the binding groups **150** can selectively bind to any desired moieties while the remaining surface remains slippery to most other species. Thus, these surfaces can be used in a wide variety of environments, such as laboratories, on medical devices, medical equipment, in drug formulations, for medical applications including anticoagulation and anti-biofilm formation, industrial applications, commercial applications, point-of-care applications, and other practical applications.

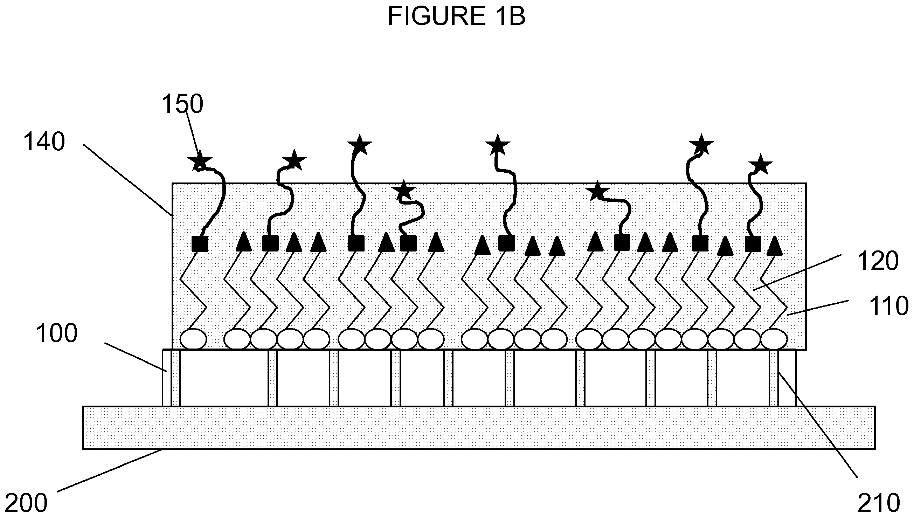

In some other embodiments, the binding groups **150** can be provided onto other anchoring molecules. As shown in **FIG. 1B****,** two or more anchoring molecules **110** and **120,** both having an affinity for the lubricating liquid can be provided over the solid substrate **100.** Anchoring molecule 110 may be provided with a different tail group (shown in triangle) as compared to the tail groups (shown in square) of the anchoring molecule **120.** In addition, as depicted in **FIG. 1B****,** the functional group depicted in square can be selectively reactive with a molecule containing a different chemical binding group **150** that selectively binds a desired moiety, denoted as a star. The different chemical binding group **150** can be provided so that the chemical group resides above or below the lubricating liquid to be applied over the anchoring layer. The tail regions of the immobilized molecular anchoring layer, including the functional groups depicted in triangle and square as well as possibly the additional chemical groups, can alter the surface properties of the solid substrate to provide a desired property. For example, depending on the nature of lubricating liquid, the immobilized molecular anchoring layer can increase the lipophilicy, hydrophilicy, or omniphobicity of the surface. The tail region interacts with lubricating liquid applied to the surface. Thus, the tail region retains the lubricating liquid by non-covalent attachment. The tail region and the lubricating liquid can be arranged on the surface to form a lubricating layer **140** on the surface.

The lubricating layer **140,** which has an affinity for the tail regions of the anchoring molecules **110** and **120,** can be formed by immobilizing the anchoring molecules **110** and **120** on the surface **100** and applying a lubricating liquid to the surface. The anchoring molecules **110** and **120** and the lubricating layer **140** are held together by non-covalent attractive forces. Together, the solid substrate and lubricating layer on the solid substrate form a slippery surface that resists adhesion by molecules and particles, and repels certain immiscible fluids. However, the additional anchoring molecules containing additional chemical binding groups **150** can simultaneously and selectively bind to certain desired target moieties. This allows the passage of materials at high flow rates without allowing most materials to adhere to, attach, foul the surface, or, in the case of biological fluids such as blood, coagulate while simultaneously binding certain desired target moieties. Thus, these surfaces can be used in a wide variety of environments, such as laboratories, on medical devices, medical equipment, for medical applications including anticoagulation and anti-biofilm formation, industrial applications, commercial applications, and other practical applications.

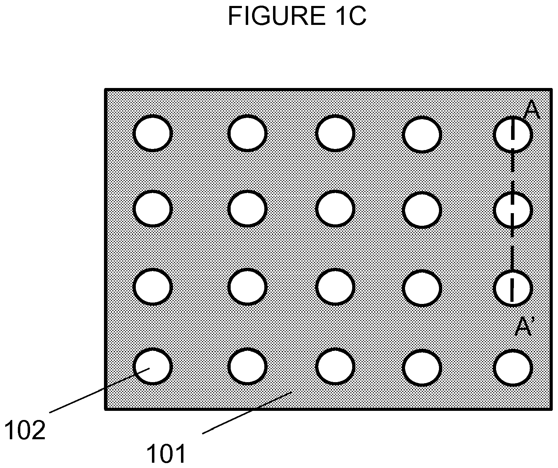

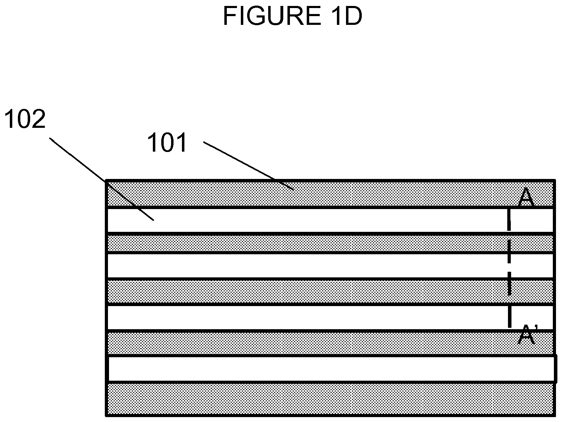

In some embodiments, solid slippery surfaces that simultaneously allow binding of targeted moieties in certain regions while repelling non-targeted moieties in other regions can be made. **FIG. 1C** shows one exemplary structure where regions **101** may be regions that repel non-targeted moieties while regions **102** may allow binding of targeted moieties. Other patterns are possible where a linear pattern of slippery regions **101** and binding regions **102** are shown in **FIG. 1D****.** In certain embodiments, the patterns may be microns in size, such as a few to several hundred microns. In some other embodiments, the patterns can be nanometers in size, such as tens to several hundred nanometers. Other suitable patterns would be readily apparent to one of ordinary skill in the art.

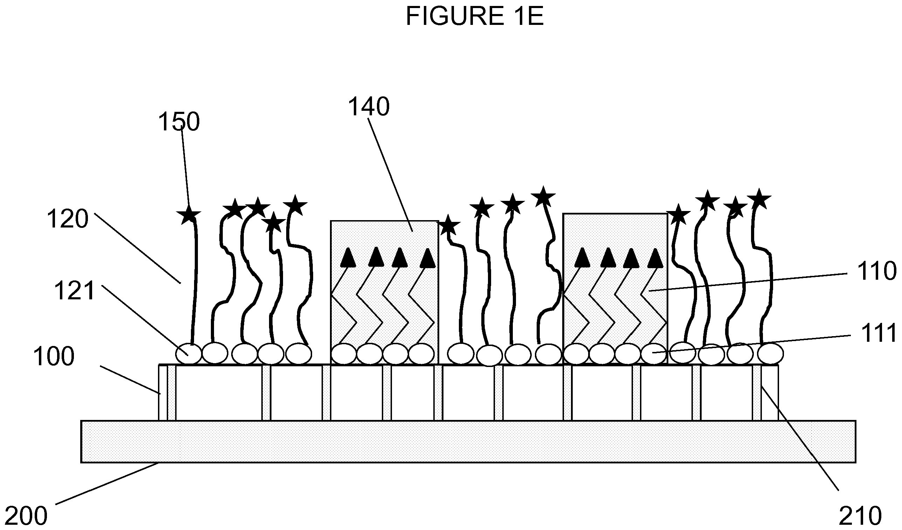

Viewing along the cross-section direction of the line A-A' in **FIGS. 1C** and **1D****,** a patterned surface having regions of both slippery and binding regions is schematically illustrated in **FIG. 1E****.** As shown, the slippery regions **101** can preferentially be wetted with a lubricating liquid **140** while the binding regions **102** are not wetted by any of the lubricating liquid **140.** In the slippery regions **101,** anchoring molecules can be attached onto the underlying surface. For example, the anchoring molecules can be adsorbed or even chemically bound (e.g., covalent bonds, ionic bonds, and the like) to the underlying surface. In some embodiments, certain anchoring molecules can be adsorbed onto the underlying surface while some other anchoring molecules can be chemically bound to the underlying surface. As exemplified in **FIG. 1E****,** the immobilized anchoring molecules in the slippery regions **101** include anchoring molecules **110** having a head region **111** that provides a chemical linkage or adhesive forces to the solid substrate **100.** The immobilized anchoring molecules also includes a tail region that can interact with a lubricating liquid **140** applied to the surface. Thus, the tail region can retain the lubricating liquid **140** by non-covalent attachment

The lubricating liquid **140,** which has an affinity for the tail region of the anchoring molecules **110,** is formed by immobilizing the anchoring molecules on the substrate surface **100** and applying a lubricating liquid to the surface. Together, the solid substrate and lubricating layer on the solid substrate form a slippery surface that resists adhesion by molecules and particles, and repels certain immiscible fluids. This allows the passage of materials at high flow rates without allowing the material to adhere to, attach, foul the surface, or, in the case of biological fluids such as blood, coagulate.

In the binding regions **102,** **FIG. 1E** shows a set of binding molecules having a head group **121** and tail groups. Particularly, the tail groups further includes binding groups **150** that can bind to desired moieties. Moreover, as shown in **FIG. 1E****,** the tail group of the binding molecules **120** may have a characteristic such that it repels the lubricating liquid **140.**

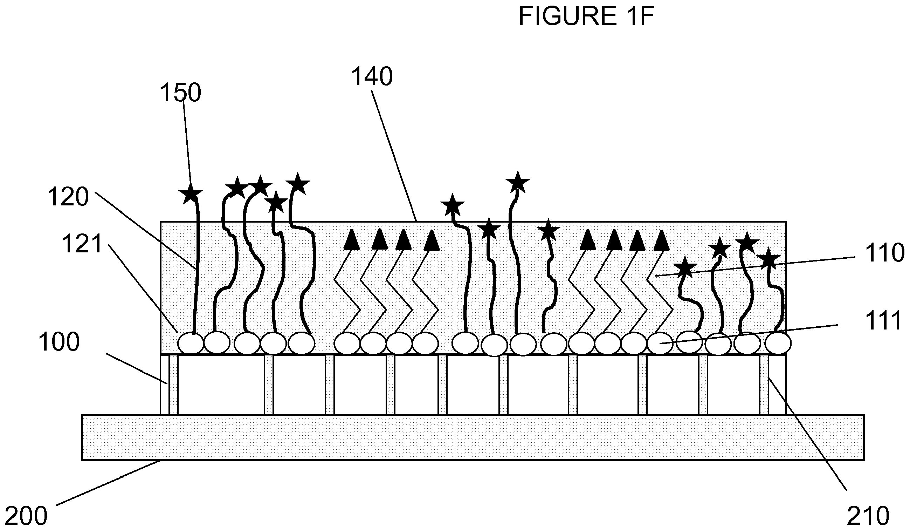

In certain embodiments, as shown in **FIG. 1F****,** the binding molecules **120** having the binding group **150** may also have groups that have an affinity with the lubricating liquid. In such instances, the lubricating liquid **140** may wet the entire surface, including both the slippery regions **101** and binding regions **102.** However, the binding molecules may be designed so that the binding groups **150** reside on at the binding regions **102** and reside above the height of the lubricating liquid **140.**

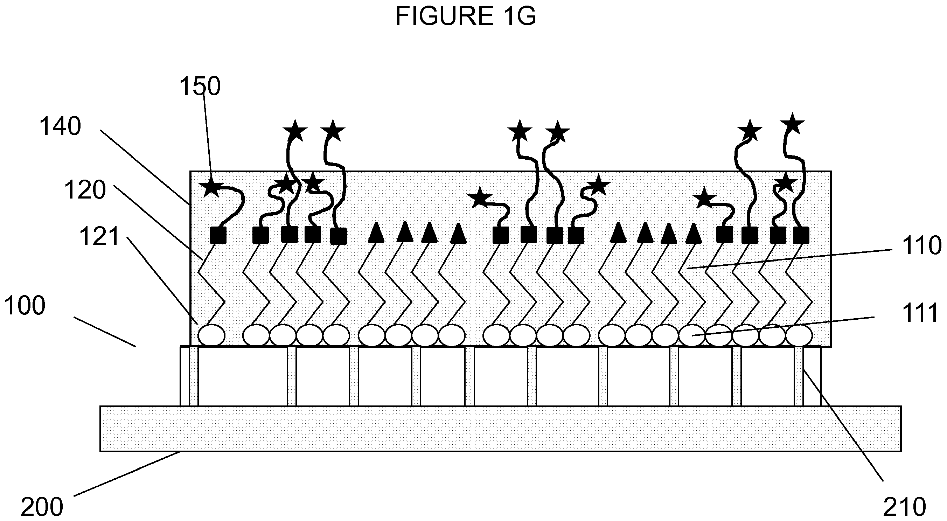

In certain embodiments, as shown in **FIG. 1G****,** binding groups **150** can be attached onto anchoring molecules **120.** The tail regions of anchoring molecules **110** in the slippery regions **101** may have different functional groups as compared to the anchoring molecules **120** in the binding regions **102.** For example, the slippery regions may have anchoring molecules that have functional groups depicted in triangle while the binding regions may have anchoring molecules that have functional groups depicted in squares. The square functional groups may selectively react with molecules that have binding groups **150.** While such designs are within the scope of the present disclosure, molecules or combination of different molecules that reside in the binding regions **102** will be referred to as binding molecules throughout this disclosure for simplicity.

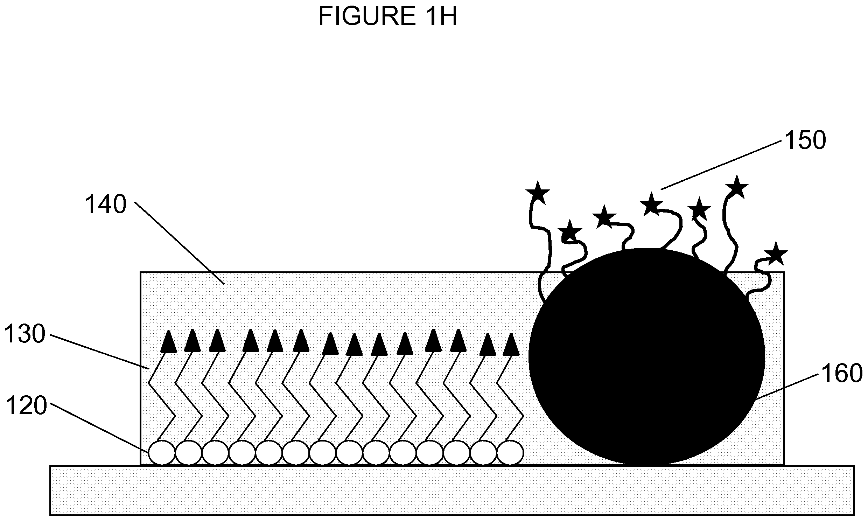

**FIG. 1H** shows yet another exemplary method to obtain a patterned surface containing slippery regions **101** and binding regions **102.** In certain embodiments, each of the patterned regions may contain additional materials, such as colloidal particles, posts, protrusions, and the like. In certain embodiments, such additional materials may be provided to ensure that the desired type of anchoring molecules are situated above the lubricating liquid. For example, as shown in **FIG. 1H****,** binding regions **102** can include colloidal particles **160** that are bound to the surface while the slippery regions **101** include anchoring molecules having tail groups **110** that have affinity with a lubricating liquid **140.** The colloidal particles **160** may further include desired binding groups **150** that can bind desired target molecules. Hence, by providing a lubricating liquid layer **140** over the substrate at a level that is below the colloidal particles, exposure of the binding groups **150** that can bind desired target molecules can be ensured. Moreover, an increase in the surface to volume ratio of the desired binding groups **150** can be increased by providing additional surface onto which binding groups **150** can be bound onto.

Such patterned surfaces can be utilized to bind certain target molecules, such as pathogens, to only the binding regions **102** while all other non-target molecules are repelled from the surface. For example, as shown in **FIG. 2****,** such target molecules can co-exist in a complex fluid, such as blood, with many other molecules. These other molecules would repel away from the surface due to the ultra slippery surface while only the target molecule binds to the binding groups **150,** such as the desired biomolecules (or other desired molecules).

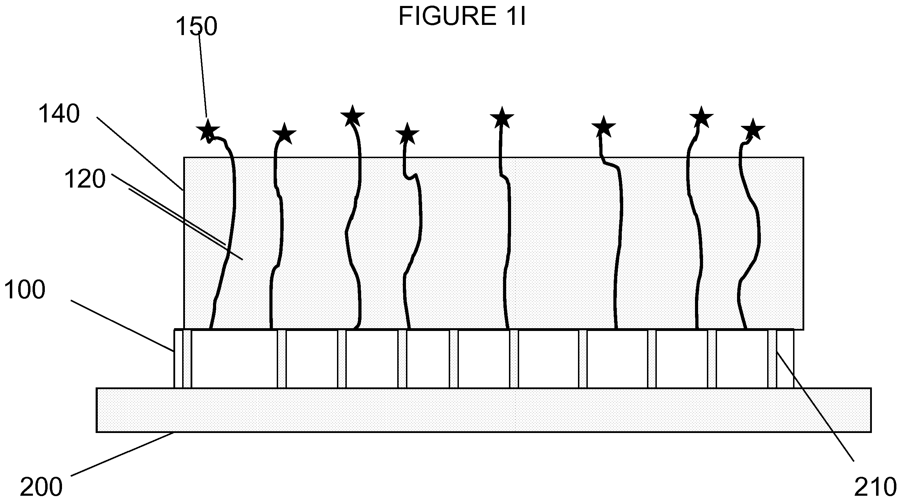

**FIG. 1I** shows yet another embodiment for forming an ultra-repellent surface having the ability to selective bind desired target moieties. As shown, only the binding molecules may be provided over the solid surface where the lubricating liquid is locked in with underlying substrate. In certain embodiments, the lubricating liquid can be locked in with the underlying substrate through capillary forces to form a lubricating liquid layer over the solid substrate. Solid substrate **100** may be a microporous structure, such as a fabric or a filter, or even a roughened surface that has the ability to retain a lubricating liquid without additional anchoring molecules. In other embodiments, the solid substrate may be a solid crosslinked polymer where the lubricating liquid has an energetically favorably enthalpy of mixing with the crosslinked polymer. Binding molecules **120** containing the desired binding groups **150** can be provided over any desired areas of the solid substrate **100.**

### Solid Substrate

Many types of solid substrates can be used in accordance with this disclosure. In certain embodiments, solid substrates having chemically reactive surfaces (or surfaces that can be activated to provide chemically reactive surfaces) can be used to repel liquids that are immiscible with the anchoring layer and the lubricating layer applied to the surface. In one embodiment, the surface is smooth. In other embodiments, the surface is not limited to any degree of surface roughness. In other embodiments, the surface is porous.

In certain embodiments, the solid substrate can be a smooth surface, such as those described in U.S. Patent Application No. 61/585,059, filed on January 10, 2012, U.S. Patent Application No. 61/692,079, filed on August 22, 2012, and PCT Application No. PCT/US2013/021056, filed on January 10, 2013, the contents of which are incorporated by reference herein in their entireties.

The liquid repellant surfaces disclosed herein have properties that are independent of the geometry of the underlying solid substrate. Thus, the geometry of the solid substrate can be any shape, form, or configuration to suit the configuration of a variety of materials. Non-limiting examples of shapes, forms, and configurations that liquid repellant surfaces can take include generally spherical (e.g., beads), tubular (e.g., for a cannula, connector, catheter, needle, capillary tube, or syringe), planar (e.g., for application to a microscope slide, plate, wafer, film, or laboratory work surface), or arbitrarily shaped (e.g., well, well plate, Petri dish, tile, jar, flask, beaker, vial, test tube, column, container, cuvette, bottle, drum, vat, or tank). The solid substrate can be flexible or rigid.

The solid substrate material can be any material that is capable of surface modification to form the immobilized molecular anchoring layer. Many solid substrate materials are commercially available, or can be made by a variety of manufacturing techniques known in the art Non-limiting examples of surfaces that can be functionalized for liquid repellency include, *e.g*., glass, polymers (e.g., polysulfone, polystyrene, polydimethylsiloxane ("PDMS"), polycarbonate, polymethylmethacrylate, polyethylene terephthalate, polyvinyl chloride, poly(lactic-co-glycolic acid), etc.), polymers with plasticizers, (e.g. polyvinyl chloride with bis(2-ethylhexyl) phthalate, etc.), metals, metal alloys, metalloids, paper, plastics, various forms of carbon (e.g, diamond, graphite, fullerene, graphene, carbon nanotubes, black carbon, etc.), metal oxides, metalloid oxides, nonmetals, nonmetal oxides, and other ceramic materials, and the like.

In certain environments, the solid substrate is selected to be compatible with the intended use of the device. For example, in medical applications such as medical devices, it is preferred that the solid material comply with FDA standards for safety and biocompatibility.

Suitable solid materials contain reactive surface moieties in its native form, or can be treated to provide suitable reactive moieties for linking with a surface-treating compound. Exemplary reactive surface moieties include oxygen-containing surface groups such as oxides, hydroxides, carboxyl, carbonyl, phenol, epoxy, quinone and lactone groups and the like; nitrogen-containing surface groups such as amino, C=N groups, azides, amides, nitrile groups, pyrrole-like structure and the like, sulfur-containing moieties such as thiols, and the like, and reactive carbon containing surface groups such as alkynes and alkenes.

Surfaces can be treated to activate the surface and render it amenable to surface modification using well-understood techniques. Exemplary surface treatments include acid or base (e.g., sodium hydroxide) treatment, oxidization, ammonization, plasma (e.g., introduction of hydroxyl groups by oxygen plasma treatment, followed by grafting a desired functional group (X) to the surface using a X-silane), heat, ion, electron, electromagnetic, photon, such as UV-induced grafting (e.g., introduction of an initiator such as benzophenone, followed by polymerization of a functional group or polymer initated at grafting sites), microwave treatment, and any combinations thereof.

In some embodiments, the solid substrate may be a roughened surface. In certain embodiments, the solid substrate may be a porous substrate. Some suitable roughened or porous substrates are described in PCT Patent Application No. PCT/US2012/21928, filed on January 19, 2012, the contents of which are incorporated by reference herein in its entirety.

In some embodiments, the solid substrate is flexible, such as for example, a flexible tube used in medical applications. In certain embodiments, the solid substrate can be a crosslinked polymer. For example, flexible PDMS tubing that has been treated according to one or more embodiments of the invention can be made ultra-repellant. For example, flexible PVC tubing that has been treated according to one or more embodiments of the invention can be made ultra-repellant.

### Anchoring Layer

As used herein, anchoring layers will generally refer to the layer that forms by depositing anchoring molecules on the substrate. Anchoring can be selectively located on the surface to create the slippery regions. However, as discussed above with respect to **FIG. 1G****,** anchoring layer may be formed across the entire surface.

Generally, most solid substrates can be modified for liquid repellency. These substrates are modified to have an affinity for and to retain a lubricating liquid to form a lubricating layer on the solid substrate with an anchoring layer. Materials known to have strong omniphobic properties do not adhere to or spread out well on most hydrophilic or hydrophobic substrates. Similarly, materials known to have strong hydrophobic properties do not adhere to or spread out well on most hydrophilic or omniphobic substrates, and materials known to have strong hydrophilic properties do not adhere to or spread out well on most hydrophobic or omniphobic substrates. The selection of the appropriate immobilized molecular anchoring layer can improve the wetting properties of such liquids and thereby provide a surface with excellent liquid repelling properties.

Modification of the substrate can be achieved by a variety of methods. Generally, the substrate is modified with an anchoring layer, which comprises a head group that covalently attaches to, or is adsorbed onto the substrate, and a functional group that non-covalently binds the lubricating layer to retain the lubricating layer on the surface. This anchoring layer forms at least a monomolecular layer on the substrate. In some embodiments, this layer forms more than a monomolecular layer on the substrate.

In some embodiments, the anchoring layer is formed on the underlying substrate by adhesion. Adhesion is the tendency of dissimilar particles and/or surfaces to cling to one another. Non-limiting adhesive forces that may be employed to form the anchoring layer include one or more of mechanical, van der Waals or electrostatic forces.

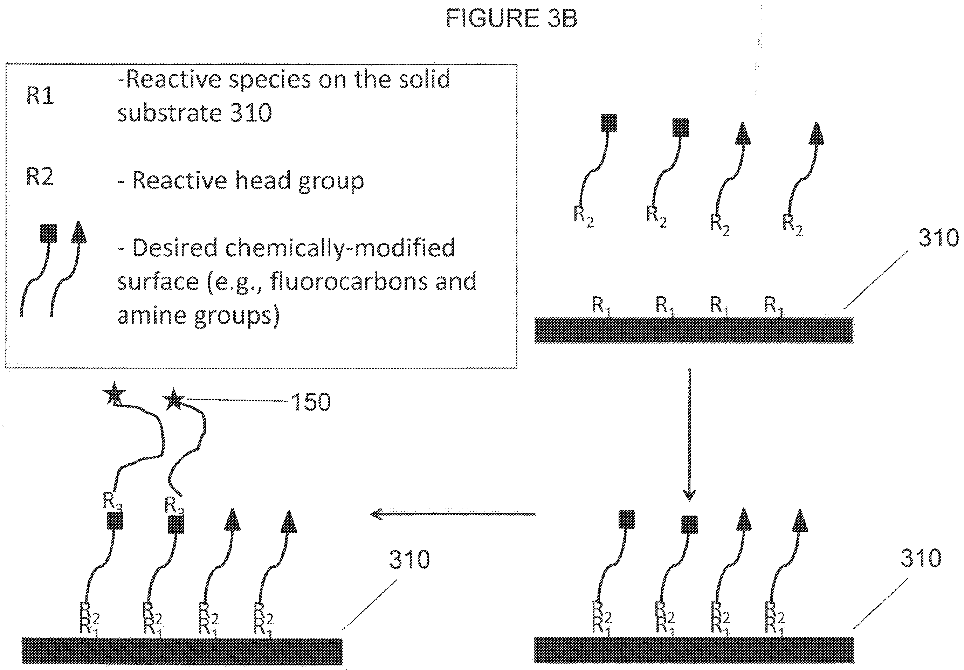

In some embodiments, the anchoring layer forms a covalent interaction with the underlying substrate. The anchoring layer is typically prepared by reaction of a reactive head group ("R2" in **FIG. 3A** and **FIG. 3B**) of the bifunctional molecule bearing the functional tail, with a reactive species ("R1" in **FIG. 3A** and **FIG. 3B**) on the surface of the solid substrate **310.** The reaction of R2 and R1 result in a linking moiety **310** that retains the functional group on the surface of the solid substrate. For example, reactive oxygen moieties on the surface ("R1") react with the silane moieties ("R2") of a perfluorocarbon silane and/or an aminosilaane, rendering a modified surface of exposed perfluorocarbon tails and/or an aminosilane.

By way of example, the reactive head group is a group that reacts with oxygen-containing surface groups such as oxides, hydroxides, carboxyl, carbonyl, phenol, epoxy, quinone and lactone groups and the like; nitrogen-containing surface groups such as amino, C=N groups, amides, azides, nitrile groups, pyrrole-like structure and the like, sulfur-containing moieties such as thiols, and the like that are on the surface of the solid substrate, and reactive carbon containing surface groups such as alkynes and alkenes. Non-limiting examples include carboxylic acids, amines, halides, silanols, thiols, carbonyls, alcohols, inorganic oxides, reactive metals (e.g., gold, platinum, silver), azides, alkenes and alkynes. For example, the surfaces with hydroxyl groups (i.e., -OH) can be functionalized with various commercially available substances such as fluorosilanes (e.g., tridecafluoro-1,1,2,2-tetrahydroocty1-trichlorosilane, heptadecafluoro-1,1,2,2-tetra-hydrodecyl trichlorosilane, etc.), alkanesilanes, aminosilanes (e.g., (3-aminopropyl)-triethoxysilane, 3-(2-aminoethyl)-aminopropyltrimethoxysilane), glycidoxysilanes (e.g., (3-glycidoxypropyl)-dimethyl-ethoxysilane), and mercaptosilanes (e.g., (3-mercaptopropyl)-trimethoxysilane). In certain embodiments, a variety of materials having native oxides, such as silicon, glass, and alumina, can be activated to contain -OH functional groups using techniques such a plasma treatment. After activation, either vapor or solution deposition techniques can be used to attach silanes to the substrates.

In other embodiments, crosslinking agents can be used to link the reactive surface with the anchoring layer molecules. **Table 1** shows additional examples of cross linking chemicals. A non-limiting list of exemplary crosslinking reagents with the same or different reactive groups at either end are shown. The reagents are classified by which chemical groups crosslink (left column) and their chemical composition (right column).

**Table 1**

| **Crosslinking Target** | **Crosslinker Reactive Groups, Features** |
|---|---|
| Amine-to-Amine | NHS esters |
| | Imidoesters |
| Sulfhydryl-to-Sulfhydryl | Maleimides |
| Nonselective | Aryl azides |
| Amine-to-Sulfhydryl | NHS ester / Maleimide |
| | NHS ester / Pyridyldithiol |
| | NHS esters / Haloacetyl |
| Amine-to-Nonselective | NHS ester / Aryl Azide |
| | NHS ester / Diazirine |
| Amine-to-Carboxyl | Carbodiimide |
| Sulfhydryl-to-Carbohydrate | Maleimide / Hydrazide |
| | Pyridyldithiol / Hydrazide |
| Amine-to-DNA | NHS ester / Psoralen |

The functional group used in the anchoring layer can be selected based on the ability of the functional group to non-covalently bind molecules of the lubricating layer and retain the lubricating layer on the surface. For example, functional groups comprising hydrocarbons such as alkanes, alkenes, alkynes, and aromatic compounds, and combinations thereof can be used to create a hydrophobic surface that has an affinity for lubricating liquids that are also hydrophobic or lypophilic. The combined surface layer and lubricating liquid is useful for repelling hydrophilic or omniphobic fluids. In another embodiment, hydrophilic functional groups can be used to create a hydrophilic surface that has an affinity for hydrophilic liquids. Exemplary hydrophilic groups include charged polypeptides, polyanions (e.g., heparin sulfate, oligonucleotides, dextran sulfate), polycations (e.g. chitosan, chitin, hexadimethrine bromide, diethylaminoethyl cellulose) polar polymers (polyacrylamide, polyethylene glycol, polypropylene glycol), polysaccharides (dextran, agarose, inulin, sepharose), amines (e.g. aminopropyl, diethylaminoethanol), carboxylic acids, guanidine, alcohols, sulfhydryls, carboxamides, metal oxides The combined surface layer and lubricating liquid is useful for repelling hydrophobic or omniphobic fluids. In still another embodiment, functional groups comprise perfluorocarbons to create an omniphobic surface for repelling hydrophilic or hydrophobic fluids.

The substrate can be coated with the anchoring layer by methods well known in the art, including plasma-assisted chemical vapor deposition, physical vapor deposition, chemical functionalization, chemical solution deposition, chemical vapor deposition, chemical cross linking, and atomic layer deposition. For example, chemical vapor deposition can be carried out by exposing the substrate to silane vapors. For chemical solution deposition, the deposition can be carried out by immersing the substrate in a silane solution followed by rinsing and drying.

The anchoring layer can be applied in a thickness sufficient to cover the surface of the substrate. The actual thickness of the applied layer may be a function of the method of application. The anchoring layer applied in a typical thickness is assumed to be a monomolecular layer, however, the layer may not completely cover the entire surface but still be sufficient to modify the surface properties of the solid substrate. Similarly, the layer may be more than one monomolecular layer.

Other methods to chemically anchor the anchoring molecules to the substrate can be employed. For example, use of hydroxyl groups, thiol groups, amine, carboxyl, cabonyl, sulfate, phosphate, halides, silanols, azides, alkenes and alkynes or other conventional techniques can be utilized.

In some embodiments, the solid substrate surface can be modified with different functional groups such as amine (NH₂), caboxylate (COOH), octadecyl (C-18), polymer such as poly(ethylene glycol) (PEG) or derivatives thereof, perflurocarbon silane and derivative thereof, amino silane and derivatives thereof, and any combinations thereof.

In some embodiments, the solid substrate surface can be modified for liquid repellency and/or reduced background binding (e.g., non-specific/random or non-desirable binding of biological molecules present in a sample) by smooth surface silane-perfluorocarbon chemisty and different embodiments of the methods described in U.S. Provisional Application No. 61/585,059 - "Modification of surfaces for liquid repellency" filed January 10, 2012, the content of which is incoporated herein by reference.

Using the silane-perfluorocarbon chemistry, in one embodiment, the surface of a solid substrate can be coated with perfluorocarbon silane or derivatives thereof, amino silane or derivatives thereof, or a combination thereof. An exemplary perfluorocarbon silane that can be used in the silane-perfluorocarbon reaction includes, but not limited to, trichlor(1H, 1H, 2H, 2H- perfluorooctyl)silane). An exemplary amino silane that can be used in the silane-perfluorocarbon reaction includes, but not limited to, 3-aminopropyltrimethoxysilane.

Amounts of perfluorocarbon silane and amino silane used for surface modification can be of any ratio. For example, the ratio of amino silane to perfluorocarbon silane can range from about 1:1 to about 1:5000, or from about 1:1 to about 1:2500, from about 1:1 to about 1000, from about 1:1 to about 1:500, from about 1:1 to about 1:250, from about 1:1 to about 1:100, or from about 1:1 to about 1:50, from about 1:1 to about 1:25, or from about 1:1 to about 1:10. In one embodiment, 100% perfluorocarbon silane can be used in surface modification described herein.

In some embodiments, the surface of a solid substrate can be coated with a biocompatible polymer that does not interfere with binding of microbes or microbial matter to microbe-binding molecules. Without limitations, examples of biocompatible polymers that can be used herein include, polyethylene oxide (PEO), polyethylene glycol (PEG), collagen, fibronectin, keratin, polyaspartic acid, polylysine, alginate, chitosan, chitin, hyaluronic acid, pectin, polycaprolactone, polylactic acid, polyglycolic acid, polyhydroxyalkanoates, dextrans, polyanhydrides, PLA-PGA, polyanhydride, polyorthoester, polycaprolactone, polyfumarate, collagen, chitosan, alginate, hyaluronic acid and other biocompatible polymers.

In some embodiments the anchoring layer can be designed such that it contains reactive groups that can be used to bind the capturing molecule for functional display. Non-limiting examples of reactive groups for covalenting binding the capturing molecules include, NHS esters, aldehyde, Imidoesters, Pentaglurophenyl ester and hydroxymethyl phosphine, epoxides, hydrazides, alkoxyamines, alkynes, azides, maleimides, haloacetyles, pyridyldisulfides, thiosulfonates, vinylsulfones, and tosyl groups. Bifunctional linkers such as epichlorohydrin, glutaraldehyde, adipic dihydrazide, ethylenediamine can link to capturing molecules iva hydroxyl-, amino-, aldehyde and carboxylic acid groups; for other examples of reactive groups known in the field for linking molecules to surfaces see "Bioconjugate Techniques", G. Hermanson, Academic Press; 2 edition (May 2, 2008) the contents of which is incorporated by reference herein and combinations thereof.

Other anchoring molecules can be utilized, such as those described in PCT Patent Application No. PCT/US2013/021056, filed on January 10, 2013, the contents of which are incorporated by reference herein in its entirety.

### Binding Groups

Many different binding groups can be utilized. Some suitable binding molecules having desired binding groups include protein, peptides, nucleic acids, polysaccharides, saccharides, proteoglycans, heparin, heparin sulfate, poly(N-isopropylacrylamide), polyurethane, metals and metal oxides (e.g. ferric oxide, ferrous oxide, cupric oxide, aluminum, aluminum oxide, zinc oxide, zinc, magnesium, calcium, and the like), alginate, silk, glycosaminoglycans, keratin, silicates, phospholipids, polyethylene glycol diol, ethylene glycol, polypropylene gylcol, perfluoroglutamic acid, perfluoropolyether (Krytox), hydroxyl-terminated, amine-terminated, methyl-terminated, and/or hydrocarbon-terminated polydimethylsiloxane, polysulfone, polyethersulfone, polymethylmethacrylate, poly(lactic-co-glycolic acid), polyacrylimide, polybutadiene, water, formamide, gluteraldehyde, acetic acid, cellulose, keratin, chitosan, chitin, polylactic acid, aliphatic hydrocarbons, aromatic hydrocarbons, phenyl groups and aptamers. Such binding molecules may further provide other functionalities, such as conductivity, and the like.

In certain embodiments, when the anchoring layer contains two or more different functional groups, at least one of the functional groups can be provided with further chemical binding groups that can bind to certain desired target moieties. For example, as shown in **FIG. 3B****,** the functional groups denoted with square can be further provided with additional chemical groups that can bind to certain moieties through the reaction of an R₃ group. The lubricating layer can be deposited thereon (not shown in **FIG. 3B**). The actual thickness of the additional chemical groups may be a function of the method of application. The additional chemical groups in a typical thickness is assumed to be a monomolecular layer. Similarly, the additional chemical groups may have different lengths and extend past the thickness of the other anchoring molecules, as schematically illustrated in **FIG. 3B****.**

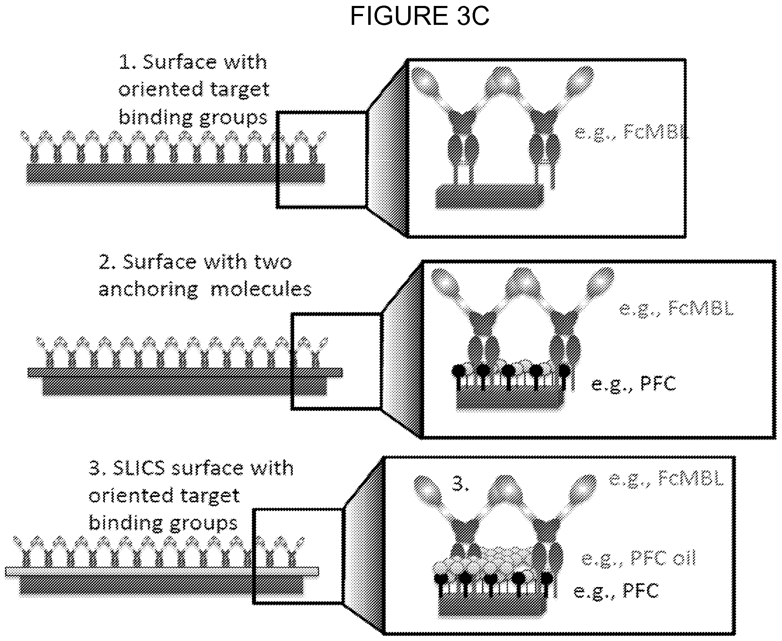

Other methods to deposit the binding molecules with the anchoring molecules are possible. For example, as shown in **FIG. 3C****,** binding molecules containing a first type of functional groups that can have an affinity with the underlying substrate and additional chemical binding groups that can bind to certain moieties can be first deposited (e.g., "Tethered FcMBL"). Then, anchoring molecules that contain, for example, perfluorinated groups can be provided to the surface (e.g., "Tethered PFC"). Thereupon, the lubricating layer may be deposited thereon ("PFC").

As schematically illustrated in **FIGS. 3B** and **3C****,** the anchoring molecules that allow selective binding of desired moieties can have a length that is longer than the other anchoring molecules that have affinity towards the lubricating liquid of the lubricating layer. As illustrated in **FIG. 3C****,** the length of the anchoring molecules that allow selective binding of desired moieties extends past the lubricating layer.

**FIG. 3C** shows the direct attachment of binding molecules having binding group 150 (e.g., FcMBL) onto a solid substrate surface. In some instances, direct attachment of the binding molecules onto the solid substrate surface can be carried out using chemical reactions. Some exemplary chemical reactive groups include those provided above in connection with the anchoring molecules.

In some instances, direct attachment of the binding molecules onto the solid substrate surface can be carried out using physical attachment. For example, the solid substrate can be plasma treated, followed by exposure (e.g., micro-contact printing) of the binding molecules onto the substrate can attach the binding molecules onto the solid substrate surface.

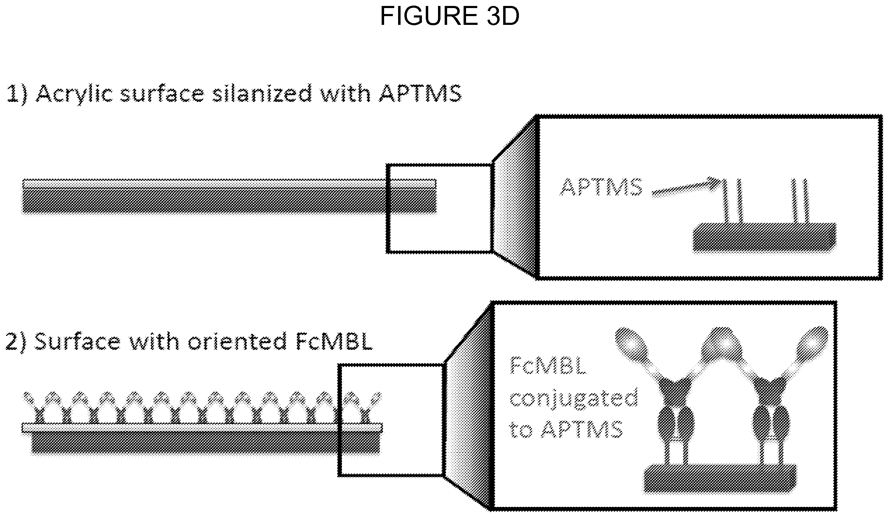

In certain embodiments, as shown in **FIG. 3D****,** the binding groups **150** can be attached onto the solid substrate via a two-step process, in which a first linker molecule (e.g., APTMS, biotin, APTES, etc.) is provided onto the binding regions **102.** Thereafter, the molecules containing the binding group **150** (e.g., FcMBL) can be deposited thereon.

These techniques described are applicable even when a patterned surface is formed. For example, as shown in **FIG. 3B****,** binding groups **150** are attached to anchoring molecules. **FIG. 3B** shows a zoomed in view at the boundary between the slippery regions **101** and the binding regions **102.** As shown, the anchoring molecules in the slippery regions **101** and the anchoring molecules in the binding regions **102** may be provided with different functional groups, so that the anchoring molecules in the binding regions **102** can be provided with further chemical groups that can bind to certain desired moieties. For example, as shown in **FIG. 3B****,** the functional groups denoted with square can be further provided with binding groups **150** through the reaction of an R₃ group. The actual thickness of the additional chemical groups bearing the binding groups **150** may be a function of the method of application. The additional chemical groups in a typical thickness is assumed to be a monomolecular layer. Similarly, the additional chemical groups may have different lengths and extend past the thickness of the other anchoring molecules, as schematically illustrated in **FIG. 3B****.**

In certain embodiments, and as schematically illustrated in **FIGS. 3B** and **3C****,** the binding molecules that allow selective binding of desired moieties can have a length that is longer than the other anchoring molecules that have affinity towards the lubricating liquid. The length of the binding groups 150 that allow selective binding of desired moieties, extends past the thickness of the lubricating liquid.

In some embodiments, the binding group comprises at least a portion of an immunoglobulin, *e.g.,* IgA, IgD, IgE, IgG and IgM including their subclasses (*e.g.,* IgG₁), or a modified molecule or recombinant thereof. Immunoglobulins, include IgG, IgA, IgM, IgD, IgE. An immunoglobulin portion (e.g., fragments) and immunoglobulin derivatives include but are not limited to single chain Fv (scFv), diabodies, Fv, and (Fab')₂, triabodies, Fc, Fab, CDR1, CDR2, CDR3, combinations of CDR's, variable regions of the light or heavy Ig chains, tetrabodies, bifunctional hybrid antibodies, framework regions, constant regions, and the like (*see,* Maynard et al., (2000) Ann. Rev. Biomed. Eng. 2:339-76; Hudson (1998) Curr. Opin. Biotechnol. 9:395-402). In one embodiment, an immunoglobulin molecule can encompass immunoglobulin ortholog genes, which are genes conserved among different biological species such as humans, dogs, cats, mice, and rats, that encode proteins (for example, homologs (including splice variants), mutants, and derivatives) having biologically equivalent functions as the human-derived protein. Immunoglobulin orthologs include any mammalian ortholog of IgG, IgA, IgM, IgD, IgE inclusive of the ortholog in humans and other primates, experimental mammals (such as mice, rats, hamsters and guinea pigs), mammals of commercial significance (such as horses, cows, camels, pigs and sheep), and also companion mammals (such as domestic animals, e.g., rabbits, ferrets, dogs, and cats), or a camel, llama, or shark.

For example, the Fc portion of an FcMBL molecule, or the Fc portion of any binding group of the instant invention, can be replaced with any of the immunoglobulin fragments described herein.

In some embodiments, the binding group comprises at least a portion of an adhesion molecule, or a modified molecule or recombinant thereof. Non-limiting examples of adhesion molecules include: cell adhesion molecules (e.g. cadherins, selectins, integrins, addressins, lymphocyte homing receptors (e.g. CD-34, GLYCAM-1)); Synaptic Cell Adhesion Molecules(SynCAMs); Neural Cell Adhesion Molecules (NCAMs); Intercellular Cell Adhesion Molecules (ICAM-1); Vascular Cell Adhesion Molecules (VCAM-1); Platelet-endothelial Cell Adhesion Molecules (PECAM-1). In one embodiment, an adhesion molecule can encompass ortholog genes discussed herein.

Other non-limiting examples of binding groups include a portion of L1, CHL1, MAG, Nectins and nectin-like molecules, CD2, CD48, SIGLEC family members (e.g. CD22, CD83), and CTX family members (e.g. CTX, JAMs, BT-IgSF, CAR, VSIG, ESAM)).

In some embodiments, the binding group comprises at least a portion of heparin. Heparin binds various proteins including growth factors (*e.g.,* FGF1, FGF2, FGF7), serine proteases (*e.g.,* Thrombin, Factor Xa) and serine protease inhibitors (such as Antithrombin). In some embodiments, the binding group comprises at least a portion of a glycosaminoglycan (GAG). In some embodiments, the binding group comprises at least one glycosaminoglycan (GAG). A GAG includes, but is not limited to a heparin/heparan sulfate GAG (HSGAG), a chondroitin/dermatan sulfate GAG (CSGAG), a keratan sulfate GAG, and hyaluronic acid. In some embodiments, the binding group comprises a portion of Hemopexin. Hemopexin binds Heme.

In other embodiments, the binding group can comprise at least a portion of a receptor molecule, or a modified molecule or recombinant thereof. Non-limiting examples of a receptor molecule include: an extracellular receptor molecule (e.g. nicotinic acetylcholine receptor, glycine receptor, GABA receptors,glutamate receptor, NMDA receptor, AMPA receptor, Kainate receptor, 5-HT3 receptor, P2X receptor); an intracellular receptor molecule (e.g. a cyclic nucleotide-gated ion channel, IP3 receptor, intracellular ATP receptor, ryanodine receptor); an immune receptor molecule (e.g. pattern recognition receptors, toll-like receptors, killer activated and killer inhibitor receptors, complement receptors, Fc receptors, B cell receptors and T cell receptors); a G protein coupled receptor molecule, a virus receptor molecule (e.g., CAR -Coxsackie Adenovirus Receptor); an iron scavenging receptor molecule (e.g., LRP/CD91, CD163). In one embodiment, a receptor molecule can encompass ortholog genes discussed herein. In other embodiments, the binding group comprises a hormone receptor. In some embodiments, the hormone receptor is a peptide hormone receptor or a steroid hormone receptor. The peptide hormone receptor can be a cell surface receptor or transmembrane receptor that binds to its cognate hormone ligand. The steroid hormone receptor is a soluble receptor that binds to its cognate hormone ligand. In one embodiment, the peptide hormone receptor comprises a thyroid-stimulating hormone receptor, a follicle-stimulating hormone receptor, a leutinizing hormone receptor, a glucagon receptor, or an insulin receptor. In another embodiment, the receptors comprises those for glucocorticoids, estrogens, androgens, thyroid hormone (T₃), calcitriol (vitamin D), and the retinoids (vitamin A). In some embodiments, the transmembrane receptor is a G-protein coupled receptor, which binds to Gs or Gi proteins.

In further embodiments, the binding group comprises at least a portion of a ligand that enriches for circulating tumor cells, for example antibodies to tumor cell markers. Ligands that enrich for circulating tumor cells include, but are not limited to, antibodies to EpCAM, antibodies to CD46, antibodies to CD24, and antibodies to CD133. In further embodiments, the binding group comprises at least a portion of a ligand that enriches for fetal cells in maternal circulation. Ligands that enrich for fetal cells include, but are not limited to, antibodies to CD71, and antibodies to glycophorin-A. In further embodiments, the binding group comprises at least a portion of a ligand that enriches for circulating leukocytes, such as antibodies to CD45, and antibodies to CD 15. In yet other embodiments, the binding group comprises at least a portion of non-immunogobulin binding proteins engineered for specific binding properties. For example, the binding proteins may contain ankyrin repeats, or the binding proteins can be anticalins. In one embodiment, anticalins can be used to screen libraries for binding to a target molecule (*e.g*., *see* Gebauer, M., & Skerra, A. (2009). Engineered protein scaffolds as next-generation antibody therapeutics. Current opinion in chemical biology, 13(3), 245-255; and Löfblom, J., Frejd, F. Y., & Ståhl, S. (2011). Non-immunoglobulin based protein scaffolds. Current Opinion in Biotechnology, 22(6), 843-848, each of which are incorporated by reference in their entireties)

For example, the Fc portion or any immunoglobulin fragment described herein can be coupled to any binding group embraced by the instant invention, which targets some specific ligand, cell, or combination thereof.

In certain embodiments, the binding groups can be derived from an engineered microbe-targeting molecule, as described in the corresponding U.S. Patent Application No. 61/508,957, entitled "Engineered Microbe-Targeting Molecules and Uses Thereof," filed herewith on even date, the contents on which are incorporated by reference herein in its entirety. Other related applications include U.S. Patent Application No. 61/508,957, filed on July 18, 2011; U.S. Patent Application No. 61/605,081, filed on February 29, 2012; U.S. Patent Application No. 61/605,052, filed on February 29, 2012; U.S. Patent Application No. 61/604,878, filed on February 29, 2012; and U.S. Patent Application No. 61/647,860, filed on May 16, 2012; the contents on which are incorporated by reference herein in their entireties.

### Microbe-binding molecules

Any molecule or material that can bind to a microbe can be employed as the microbe-binding molecule (or microbe-targeting molecules). Exemplary microbe-binding molecules (or microbe-targeting molecules) include, but are not limited to, opsonins, lectins, antibodies and antigen binding fragments thereof, proteins, peptides, nucleic acids, carbohydrates, lipids, and any combinations thereof. The microbe-binding molecule can comprise at least one (e.g., one, two, three, four, five, six, seven, eight, nine, ten, eleven, twelve, thirteen, fourteen, fifteen, sixteen, seventeen, eighteen, nineteen, twenty or more) microbe surface-binding domain ("microbe binding domain"). The term "microbe surface-binding domain" as used herein refers to any molecule or a fragment thereof that can specifically bind to the surface of a microbe or pathogen, e.g., any component present on a surface of a microbe or pathogen, and/or any microbial matter, e.g., any matter or component/fragment that is derived, originated or secreted from a microbe.

General methods of preparing any embodiments of the engineered microbe-binding molecules are known in the art (Ashkenazi, A. and S. M. Chamow (1997), "Immunoadhesins as research tools and therapeutic agents," Curr. Opin. Immunol. 9(2): 195-200, Chamow, S. M. and A. Ashkenazi (1996). "Immunoadhesins: principles and applications," Trends Biotechnol. 14(2):52-60). In one example, an engineered microbe-binding molecule can be made by cloning into an expression vector such as Fc-X vector as discussed in Lo *et al.* (1998) 11:495.

The engineered microbe-binding molecules can contain sequences from the same species or from different species. For example, an interspecies hybrid microbe-binding molecule can contain a linker, *e.g*., a peptide linker, from a murine species, and a human sequence from a carbohydrate recognition domain protein, provided that they do not provide unacceptable levels of deleterious effects. The engineered microbe-binding molecules described herein can also include those that are made entirely from murine-derived sequences or fully human.

Molecules or substances which can serve as microbe-binding molecules can include, for example, but are not limited to, peptides, polypeptides, proteins, peptidomimetics, antibodies, antibody fragments (*e.g*., antigen binding fragments of antibodies), carbohydrate-binding protein, *e.g.,* a lectin, glycoproteins, glycoprotein-binding molecules, amino acids, carbohydrates (including mono-, di-, tri- and poly-saccharides), lipids, steroids, hormones, lipid-binding molecules, cofactors, nucleosides, nucleotides, nucleic acids (*e.g.,* DNA or RNA, analogues and derivatives of nucleic acids, or aptamers), peptidoglycan, lipopolysaccharide, small molecules, and any combinations thereof. In some embodiments, the microbe-binding molecule can comprise a carbohydrate recognition domain or a fragment thereof. In some embodiments, a microbe-binding molecule can comprise a peptidomimetic that mimics any molecule or a fragment thereof that can specifically bind to the surface of a microbe or pathogen, and/or any microbial matter. For example, a microbe-binding domain can comprise a peptidomimetic that mimics any carbohydrate recognition domain or a fragment thereof, *e.g.,* carbohydrate recognition domain of MBL or a fragment thereof; or any carbohydrate recognition domain that is known in the art or a fragment thereof. In some embodiments, the microbe-binding domain comprises the full amino acid sequence of a carbohydrate-binding protein. The microbe-binding molecule can be covalently (e.g., cross-linked) or non-covalently linked to the surface of a capture element described herein.

In some embodiments, the microbe surface-binding domain comprises a carbohydrate recognition domain from a carbohydrate binding protein, a pattern recognition domain from a pattern recognition receptor, or a peptidoglycan binding domain from a peptidoglycan recognition protein. In some embodiments, the microbe surface-binding domain can comprise, in addition to the specified domain, a fragment or portion of the protein from which the domain is obtained. In some embodiments, the microbe surface-binding domain can comprise the full amino acid sequence of a carbohydrate binding protein, a pattern recognition receptor, or a peptidoglycan recognition protein.

In some embodiments, the microbe surface-binding domain can have an amino acid sequence of about 10 to about 300 amino acid residues, or about 50 to about 150 amino acid residues. In some embodiments, the microbe surface-binding domain can have an amino acid sequence of at least about 5, at least about 10, at least about 15, at least about 20, at least about 30, at least about 40, at least about 50, at least about 60, at least about 70, at least about 80, at least about 90, at least about 100 amino acid residues or more. For any known sequences of microbe surface-binding molecules, one of skill in the art can determine the optimum length of amino acid sequence for the microbe surface-binding domain.

In some embodiments, the microbe surface-binding domain can comprise an opsonin or a fragment thereof. The term "opsonin" as used herein refers to naturally-occurring and synthetic molecules which are capable of binding to or attaching to the surface of a microbe or a pathogen, of acting as binding enhancers for a process of phagocytosis. Examples of opsonins which can be used in the engineered molecules described herein include, but are not limited to, vitronectin, fibronectin, complement components such as Clq (including any of its component polypeptide chains A, B and C), complement fragments such as C3d, C3b and C4b, mannose-binding protein, conglutinin, surfactant proteins A and D, C-reactive protein (CRP), alpha2-macroglobulin, and immunoglobulins, for example, the Fc portion of an immunoglobulin.

In some embodiments, the microbe surface-binding domain comprises a carbohydrate recognition domain. In some embodiments, the microbe surface-binding domain can further comprise at least a portion of a carbohydrate-binding protein or a portion thereof. As used herein, the term "carbohydrate recognition domain" refers to a region, at least a portion of which, can bind to carbohydrates on a surface of a microbe (e.g., a pathogen). Examples of carbohydrate-binding proteins include, but are not limited to, lectin, collectin, ficolin, mannose-binding lectin (MBL), maltose-binding protein, arabinose-binding protein, and glucose-binding protein. Additional carbohydrate-binding proteins that can be included in the microbe surface-binding domain described herein can include, but is not limited to, lectins or agglutinins that are derived from a plant, e.g., Galanthus nivalis agglutinin (GNA) from the *Galanthus* (snowdrop) plant, and peanut lectin. In some embodiments, pentraxin family members, e.g., C-reactive protein, can also be used as a carbohydrate-binding protein. Pentraxin family members can generally bind capsulated microbes. The carbohydrate-binding proteins can be wild-type, recombinant or a fusion protein. The respective carbohydrate recognition domains for such carbohydrate-binding proteins are known in the art, and can be modified for various embodiments of the engineered microbe-binding molecules described herein. In some embodiments, peptidomimetics or any structural mimics mimicking a microbe surface-binding domain (*e.g.,* a carbohydrate recognition domain or a fragment thereof) and capable of binding to a microbe surface can also be used as a microbe surface-binding domain described herein.

In some embodiments, the microbe surface-binding domain comprises a lectin or a carbohydrate recognition or binding fragment or portion thereof. The term "lectin" as used herein refers to any molecules including proteins, natural or genetically modified (*e.g.,* recombinant), that interact specifically with saccharides (*e.g.,* carbohydrates). The term "lectin" as used herein can also refer to lectins derived from any species, including, but not limited to, plants, animals, insects and microorganisms, having a desired carbohydrate binding specificity. Examples of plant lectins include, but are not limited to, the Leguminosae lectin family, such as ConA, soybean agglutinin, peanut lectin, lentil lectin, and Galanthus nivalis agglutinin (GNA) from the *Galanthus* (snowdrop) plant. Other examples of plant lectins are the Gramineae and Solanaceae families of lectins. Examples of animal lectins include, but are not limited to, any known lectin of the major groups S-type lectins, C-type lectins, P-type lectins, and 1-type lectins, and galectins. In some embodiments, the carbohydrate recognition domain can be derived from a C-type lectin, or a fragment thereof. C-type lectin can include any carbohydrate-binding protein that requires calcium for binding. In some embodiments, the C-type lectin can include, but are not limited to, collectin, DC-SIGN, and fragments thereof. Without wishing to be bound by theory, DC-SIGN can generally bind various microbes by recognizing high-mannose-containing glycoproteins on their envelopes and/or function as a receptor for several viruses such as HIV and Hepatitis C.

Collectins are soluble pattern recognition receptors (PRRs) belonging to the superfamily of collagen containing C-type lectins. Exemplary collectins include, without limitations, mannan-binding lectin (MBL) or mannose-binding protein, surfactant protein A (SP-A), surfactant protein D (SP-D), collectin liver 1 (CL-L1), collectin placenta 1 (CL-P1), conglutinin, collectin of 43 kDa (CL-43), collectin of 46 kDa (CL-46), and a fragment thereof.

In some embodiments, the microbe-surface binding domain comprises the full amino acid sequence of a carbohydrate-binding protein.

In some embodiments, the microbe surface-binding molecule comprises a mannose-binding lectin (MBL) or a carbohydrate binding fragment or portion thereof. Mannose-binding lectin, also called mannose binding protein (MBP), is a calcium-dependent serum protein that can play a role in the innate immune response by binding to carbohydrates on the surface of a wide range of microbes or pathogens (viruses, bacteria, fungi, protozoa) where it can activate the complement system. MBL can also serve as a direct opsonin and mediate binding and uptake of microbes or pathogens by tagging the surface of a microbe or pathogen to facilitate recognition and ingestion by phagocytes. MBL and an engineered form of MBL (FcMBL and Akt-FcMBL) are described in PCT Application No. PCT/US2011/021603, filed January 19, 2011 and U.S. Provisional Application Nos. 61/508,957, filed July 18, 2011, and 61/605,081, filed February 29, 2012, the contents of both of which are incorporated herein by reference.

MBL is a member of the collectin family of proteins. A native MBL is a multimeric structure (*e.g.,* about 650 kDa) composed of subunits, each of which contains three identical polypeptide chains. Each MBL polypeptide chain (containing 248 amino acid residues in length with a signal sequence: SEQ ID NO. 1) comprises a N-terminal cysteine rich region, a collagen-like region, a neck region, and a carbohydrate recognition domain (CRD). The sequence of each region has been identified and is well known in the art SEQ ID NO. 2 shows a full-length amino acid sequence of MBL without a signal sequence.

The surface or carbohydrate recognition function of a native MBL is mediated by clusters of three C-type carbohydrate-recognition domains (CRDs) held together by coiled-coils of a-helices. The N-terminal portion collagen-like domain is composed of Gly-X-Y triplets. The short N-terminal domain contains several cysteine residues that form interchain disulfide bonds. Serum MBLs assemble into larger forms containing 2-4 trimeric subunits in rodents and as many as six subunits in humans. All three oligomeric forms of rat serum MBP, designated MBPA, can fix complement, although the larger oligomers have higher specific activity. Many species express a second form of MBP. In rats, the second form, MBP-C, is found in the liver. MBP-C does not form higher oligomers beyond the simple subunit that contains three polypeptides.

When a native MBL interacts with carbohydrates on the surface of microbes or pathogens, *e.g*., calcium-dependent binding to the carbohydrates mannose, N-acetylglucosamine, and/or fucose, it can form the pathogen recognition component of the lectin pathway of complement activation. The MBL binds to surface arrays containing repeated mannose or N-acetylglucosamine residues. It circulates as a complex with one or more MBP-associated serine proteases (MASPs) that autoactivate when the complex binds to an appropriate surface. The MBL and associated MASP proteins can activate C2/C4 convertase leading to the deposition of C4 on the pathogen surface and opsonization for phagocytosis. The native MBL can also activate coagulation function through MASP proteins.

While native MBL can detect microbes or pathogens and act as opsonins for tagging the microbes for phagocytosis, native MBLs may not be desirable for use in treatment of microbe-induced inflammatory diseases or infections, *e.g*., sepsis, because native MBLs can activate complement system and induce an inflammatory response. Provided herein is an engineered MBL molecule that binds to microbes or pathogens, comprising at least one carbohydrate recognition domain or a fragment thereof, *e.g*., derived from MBL. In some embodiments, the engineered MBL molecule can comprises at least two, at least three or at least four carbohydrate recognition domains or a fragment thereof. In some embodiments, the engineered MBL molecules do not activate complement system or coagulation side effects that are present in a native MBL. Such embodiments can be used as dominant-negative inhibitors of downstream responses *in vivo* or as microbe-binding proteins that do not induce coagulation or complement fixation *in vitro.* For example, the engineered MBL molecules that do not have complement fixation and/or coagulation domains can act as a dominant negative protein in terms of activating cytokine and/or inflammatory cascades, and thus reduce system inflammatory syndrome and/or sepsis symptons.

In one embodiment, a dimeric engineered MBL molecule comprises at least two carbohydrate recognition domains (*e.g.,* MBL CRD) connected, directly or indirectly, to a linker, *e.g*., a Fc region. The N-terminal of the Fc region can further comprise an oligopeptide, *e.g.,* comprising an amino acid sequence AKT. In some embodiments, the carbohydrate recognition domains can further comprise neck regions such as MBL neck to provide flexibility of the CRD interacting with microbes.

Without wishing to be bound by a theory, microbe binding molecules comprising lectins or modified versions thereof can act as broad-spectrum microbe binding molecules (e.g., pathogen binding molecules). Accordingly, capture of microbes utilizing lectins (e.g., MBL and genetically engineered version of MBL (FcMBL and Akt-FcMBL)) as broad-spectrum microbe binding molecules (e.g., pathogen binding molecules) can be carried out without identifying the microbe (e.g., pathogen).

In some embodiments, at least two microbe surface-binding domains (*e.g*., carbohydrate recognition domains), including at least three, at least four, at least five, at least six, at least seven, at least eight, at least nine, at least ten or more microbe surface-binding domains, can be linked together to form a multimeric microbe surface-binding domain or carbohydrate recognition domain. In such embodiments, the distances between microbe surface-binding domains (*e.g.,* carbohydrate recognition domains) can be engineered to match with the distance between the binding sites on the target microbe surface.

A multimeric microbe surface-binding domain can have each of the individual microbe surface-binding domains be identical. Alternatively, a multimeric microbe surface-binding domain can have at least one, at least two, or at least three microbe surface-binding domains different from the rest In such embodiments, microbe surface-binding domains that share a common binding specificity for molecule on a microbe surface can be used. By way of example only, the fibrinogen-like domain of several lectins has a similar function to the CRD of C-type lectins including MBL, and function as pattern-recognition receptors to discriminate microbes or pathogens from self. One of such lectins comprising the fibrinogen-like domain is serum ficolins.

Serum ficolins have a common binding specificity for GlcNAc (N-acetylglucosamine), elastin or GalNAc (N-acetyl-galactosamine). The fibrinogen-like domain is responsible for the carbohydrate binding. In human serum, two types of ficolin, known as L-ficolin (also called P35, ficolin L, ficolin 2 or hucolin) and H-ficolin (also called Hakata antigen, ficolin 3 or thermolabile b2-macroglycoprotein), have been identified, and both of them have lectin activity. L-ficolin recognises GlcNAc and H-ficolin recognises GalNAc. Another ficolin known as M-ficolin (also called P35-related protein, ficolin 1 or ficolin A) is not considered to be a serum protein and is found in leucocytes and in the lungs. L-ficolin and H-ficolin activate the lectin-complement pathway in association with MASPs. M-Ficolin, L-ficolin and H-ficolin have calcium-independent lectin activity. Accordingly, in some embodiments, a microbe-binding molecule can comprise MBL and L-ficolin carbohydrate recognition domains, MBL and H-ficolin carbohydrate recognition domains, or a combination thereof.

Any art-recognized recombinant carbohydrate-binding proteins or carbohydrate recognition domains can also be used in the microbe-binding molecules. For example, recombinant manose-binding lectins, e.g., but not limited to, the ones disclosed in the U.S. Patent Nos. 5,270,199; 6,846,649; and U.S. Patent Application No. US 2004/0,229,212, content of both of which is incorporated herein by reference, can be used in constructing a microbe-binding molecule.

The microbe binding molecule can further comprise at least one (e.g., one, two, three, four, five, six, seven, eight, nine, ten, eleven, twelve, thirteen, fourteen, fifteen, sixteen, seventeen, eighteen, nineteen, twenty or more) substrate surface binding domain ("substrate binding domain") adapted for orienting the microbe binding domain away from the substrate surface. As used herein, the term "substrate-binding domain" refers to any molecule that facilitates the conjugation of the engineered molecules described herein to a substrate or a functionalized substrate. The microbe binding domain and the substrate binding domains can be linked by a linker. Similarly, the substrate binding domain and the substrate surface can be linked by a linker.

The substrate-binding domain can comprise at least one amino group that can non-covalently or covalently couple with functional groups on the surface of the substrate. For example, the primary amines of the amino acid residues (e.g., lysine or cysteine residues) at the N-terminus or in close proximity to the N-terminus of the microbe surface-binding domains can be used to couple with functional groups on the substrate surface.

In some embodiments, the substrate-binding domain can comprise at least one, at least two, at least three or more oligopeptides. The length of the oligonucleotide can vary from about 2 amino acid residues to about 10 amino acid residues, or about 2 amino acid residues to about 5 amino acid residues. Determination of an appropriate amino acid sequence of the oligonucleotide for binding with different substrates is well within one of skill in the art. For example, an oligopeptide comprising an amino acid sequence of AKT, which provides a single biotinylation site for subsequent binding to streptavidin-coated substrate. Such single biotinylation site can also enable the microbe surface binding domain of a microbe binding molecule to orient away from the substrate, and thus become more accessible to microbes or pathogens. See, for example, Witus et al. (2010) JACS 132: 16812.

In some embodiments, the substrate-binding domain can comprise at least one oligonucleotide. The sequence and length of the oligonucleotides can be configured according to the types of the substrate, binding density, and/or desired binding strength. For example, if the substrate is a nucleic acid scaffold, e.g., a DNA scaffold, the oligonucleotide sequence of the substrate-binding domain can be designed such that it is complementary to a sub-sequence of the nucleic acid scaffold to where the substrate-binding domain can hybridize.

In some embodiments, the oligonucleotides can include aptamers. As used herein, the term "aptamer" means a single-stranded, partially single-stranded, partially double-stranded or double-stranded nucleotide sequence capable of specifically recognizing a selected non-oligonucleotide molecule or group of molecules by a mechanism other than Watson-Crick base pairing or triplex formation. Aptamers can include, without limitation, defined sequence segments and sequences comprising nucleotides, ribonucleotides, deoxyribonucleotides, nucleotide analogs, modified nucleotides and nucleotides comprising backbone modifications, branchpoints and nonnucleotide residues, groups or bridges. Methods for selecting aptamers for binding to a molecule are widely known in the art and easily accessible to one of ordinary skill in the art. The oligonucleotides including aptamers can be of any length, e.g., from about 1 nucleotide to about 100 nucleotides, from about 5 nucleotides to about 50 nucleotides, or from about 10 nucleotides to about 25 nucleotides. Generally, a longer oligonucleotide for hybridization to a nucleic acid scaffold can generate a stronger binding strength between the engineered microbe surface-binding domain and substrate.

The microbe-binding molecules can contain sequences from the same species or from different species. For example, an interspecies hybrid microbe-binding molecule can contain a linker, e.g., a peptide linker, from a murine species, and a human sequence from a carbohydrate recognition domain protein, provided that they do not provide unacceptable levels of deleterious effects. The engineered microbe-binding molecules described herein can also include those that are made entirely from murine-derived sequences or fully human.

General methods of preparing such microbe-binding molecules are well known in the art (Ashkenazi, A. and S. M. Chamow (1997), "Immunoadhesins as research tools and therapeutic agents," Curr. Opin. Immunol. 9(2): 195-200, Chamow, S. M. and A. Ashkenazi (1996). "Immunoadhesins: principles and applications," Trends Biotechnol. 14(2):52-60). In one example, an engineered microbe-binding molecule can be made by cloning into an expression vector such as Fc-X vector as discussed in Lo et al. (1998) 11:495 and PCT application no. PCT/US2011/021603, filed January 19,2011, content of both of which is incorporated herein by reference.

In one embodiment, the microbe-binding molecule comprises an MBL, a carbohydrate recognition domain of an MBL, or a genetically engineered version of MBL (FcMBL) as described in International Application No. PCT/US2011/021603, filed January 19, 2011, and NO. PCT/US2012/047201, filed July 18,2012, content of which is incorporated herein by reference. As noted above, when the microbe surface-binding domain is a carbohydrate recognition domain of an MBL, the microbe-binding molecule further comprises an antimicrobial peptide or a functional fragment thereof. Amino acid sequences for MBL and engineered MBL are:
(i) *MBL full length (SEQ ID NO. 1):* MSLFPSLPLL LLSMVAASYS ETVTCEDAQK TCPAVIACSS PGINGFPGKD GRDGTKGEKG EPGQGLRGLQ GPPGKLGPPG NPGPSGSPGP KGQKGDPGKS PDGDSSLAAS ERKALQTEMA RIKKWLTFSL GKQVGNKFFL TNGEIMTFEK VKALCVKFQA SVATPRNAAE NGAIQNLIKE EAFLGITDEK TEGQFVDLTG NRLTYTNWNE GEPNNAGSDE DCVLLLKNGQ WNDVPCSTSH LAVCEFPI
(ii) *MBL without the signal sequence (SEQ ID NO. 2):* ETVTCEDAQK TCPAVIACSS PGINGFPGKD GRDGTKGEKG EPGQGLRGLQ GPPGKLGPPG NPGPSGSPGP KGQKGDPGKS PDGDSSLAAS ERKALQTEMA RIKKWLTFSL GKQVGNKFFL TNGEIMTFEK VKALCVKFQA SVATPRNAAE NGAIQNLIKE EAFLGITDEK TEGQFVDLTG NRLTYTNWNE GEPNNAGSDE DCVLLLKNGQ WNDVPCSTSH LAVCEFPI
(iii) *Truncated MBL (SEQ ID NO. 3):* AASERKALQT EMARIKKWLT FSLGKQVGNK FFLTNGEIMT FEKVKALCVK FQASVATPRN AAENGAIQNL IKEEAFLGIT DEKTEGQFVD LTGNRLTYTN WNEGEPNNAG SDEDCVLLLK NGQWNDVPCS TSHLAVCEFP I
(iv) *Carbohydrate recognition domain (CRD) of MBL (also referred to as the "head"; SEQ ID NO. 4):* VGNKFFLTNG EIMTFEKVKA LCVKFQASVA TPRNAAENGA IQNLIKEEAF LGITDEKTEG QFVDLTGNRL TYTNWNEGEP NNAGSDEDCV LLLKNGQWND VPCSTSHLAV CEFPI
(v) ***Neck*** + *Carbohydrate recognition domain of MBL (SEQ ID NO. 5):* **PDGDSSLAAS ERKALQTEMA RIKKWLTFSL** GKQVGNKFFL TNGEIMTFEK VKALCVKFQA SVATPRNAAE NGAIQNLIKE EAFLGITDEK TEGQFVDLTG NRLTYTNWNE GEPNNAGSDE DCVLLLKNGQ WNDVPCSTSH LAVCEFPI
(vi) *FcMBL.81 (SEQ ID NO. 6):* EPKSSDKTHT CPPCPAPELL GGPSVFLFPP KPKDTLMISR TPEVTCVWD VSHEDPEVKFNWYVDGVEVH NAKTKPREEQ YNSTYRWSV LTVLHQDWLN GKEYKCKVSN KALPAPIEKT ISKAKGQPRE PQVYTLPPSR DELTKNQVSL TCLVKGFYPS DIAVEWESNG QPENNYKTTPPVLDSDGSFF LYSKLTVDKS RWQQGNVFSC SVMHEALHNH YTQKSLSLSP GAPDGDSSLAASERKALQTE MARIKKWLTF SLGKQVGNKF FLTNGEIMTF EKVKALCVKF QASVATPRNA AENGAIQNLI KEEAFLGITD EKTEGQFVDL TGNRLTYTNW NEGEPNNAGS DEDCVLLLKN GQWNDVPCST SHLAVCEFPI
(vii) *Akt-FcMBL (SEQ ID NO. 7*): AKTEPKSSDKTHT CPPCPAPELL GGPSVFLFPP KPKDTLMISR TPEVTCVWD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR DELTKNQVSL TCLVKGFYPS DIAVEWESNG QPENNYKTTP PVLDSDGSFF LYSKLTVDKS RWQQGNVFSC SVMHEALHNH YTQKSLSLSP GAPDGDSSLA ASERKALQTE MARIKKWLTF SLGKQVGNKF FLTNGEIMTF EKVKALCVKF QASVATPRNA AENGAIQNLI KEEAFLGITD EKTEGQFVDL TGNRLTYTNW NEGEPNNAGS DEDCVLLLKN GQWNDVPCST SHLA VCEFPI
(viii) FriVfBL. *111 (SEQ ID NO. 8):* EPKSSDKTHT CPPCPAPELL GGPSVFLFPP KPKDTLMISR TPEVTCVWD VSHEDPEVKF NWYVDGVEVH NAKTKPREEQ YNSTYRVVSV LTVLHQDWLN GKEYKCKVSN KALPAPIEKT ISKAKGQPRE PQVYTLPPSR DELTKNQVSL TCLVKGFYPS DIAVEWESNG QPENNYKTTP PVLDSDGSFF LYSKLTVDKS RWQQGNVFSC SVMHEALHNH YTQKSLSLSP GATSKQVGNKF FLTNGEIMTF EKVKALCVKF QASVATPRNA AENGAIQNLI KEEAFLGITD EKTEGQFVDL TONRLTYTNW NEGEPNNAGS DEDCVLLLKN GQWNDVPCST SHLAVCEFPI

In some embodiments, the microbe-binding molecule comprises an amino acid sequence selected from SEQ ID NO. 1 - SEQ ID NO. 8.

Without wishing to be bound by a theory, microbe-binding molecules comprising lectins or modified versions thereof can act as broad-spectrum pathogen binding molecules. Accordingly, microbes and/or microbial matter present in a test sample can be captured using lectin-based microbe-binding molecules without identifying the microbe.

The full-length amino acid sequence of carbohydrate recognition domain (CRD) of MBL is shown in SEQ ID NO. 4. The carbohydrate recognition domain of an engineered MBL described herein can have an amino acid sequence of about 10 to about 300 amino acid residues, or about 50 to about 160 amino acid residues. In some embodiments, the microbe surface-binding domain can have an amino acid sequence of at least about 5, at least about 10, at least about 15, at least about 20, at least about 30, at least about 40, at least about 50, at least about 60, at least about 70, at least about 80, at least about 90, at least about 100, at least about 150 amino acid residues or more. Accordingly, in some embodiments, the carbohydrate recognition domain of the engineered MBL molecule can comprise SEQ ID NO. 4. In some embodiments, the carbohydrate recognition domain of the engineered MBL molecule can comprise a fragment of SEQ ID NO. 4. Exemplary amino acid sequences of such fragments include, but are not limited to, ND (SEQ ID NO. 10), EZN (SEQ ID NO. 11: where Z is any amino acid, e.g., P), NEGEPNNAGS (SEQ ID NO. 12) or a fragment thereof comprising EPN, GSDEDCVLL (SEQ ID NO. 13) or a fragment thereof comprising E, and LLLKNGQWNDVPCST (SEQ ID NO. 14) or a fragment thereof comprising ND. Modifications to such CRD fragments, *e.g*., by conservative substitution, are also within the scope described herein. In some embodiments, the MBL or a fragment thereof used in the microbe surface-binding domain of the engineered microbe-binding molecules described herein can be a wild-type molecule or a recombinant molecule.

The exemplary sequences provided herein for the carbohydrate recognition domain of the engineered microbe-binding molecules are not construed to be limiting. For example, while the exemplary sequences provided herein are derived from a human species, amino acid sequences of the same carbohydrate recognition domain in other species such as mice, rats, porcine, bovine, feline, and canine are known in the art and within the scope described herein.

In some embodiments, the nucleic acid encodes a carbohydrate recognition domain having greater than 50% homology, including greater than 60%, greater than 70%, greater than 80%, greater than 90% homology or higher, to a fragment of at least 50, at least 60, at least 70, at least 80, at least 90, at least 100, at least 150 contiguous amino acids or more, of any known carbohydrate-binding molecules (*e.g*., mannose-binding lectins).

The term "carbohydrate recognition domain" as used herein refers to a region, at least a portion of which, can bind to carbohydrates on a surface of microbes or pathogens. For example, the carbohydrate recognition domain, in some embodiments, can encompass MBL CRD. However, in some embodiments, the carbohydrate recognition domain can be also construed to encompass a neck region in addition to MBL CRD. In some embodiments, the carbohydrate recognition domain can comprise at least about 50% of its domain, including at least about 60%, at least about 70%, at least about 80%, at least about 90% or higher, capable of binding to carbohydrates on a microbe surface. In some embodiments, 100% of the carbohydrate recognition domain can be used to bind to microbes or pathogens. In other embodiments, the carbohydrate recognition domain can comprise additional regions that are not capable of carbohydrate binding, but can have other characteristics or perform other functions, e.g., to provide flexibility to the carbohydrate recognition domain when interacting with microbes or pathogens.

Accordingly, in some embodiments, the carbohydrate recognition domain can further comprise a neck region of the MBL with an amino acid sequence: PDGDSSLAAS ERKALQTEMA RIKKWLTFSL GKQ (SEQ ID NO. 15), or a fragment thereof. Without wishing to be bound by theory, the neck region can provide flexibility and proper orientation of the CRD to bind to a microbe surface. In some embodiments, the carbohydrate recognition domain can comprises a full-length CRD of MBL (SEQ ID NO. 4; termed as "CRD head") and the neck region thereof. The amino acid sequence encoding a full-length CRD of MBL and the neck region thereof is shown in SEQ ID NO. 5. The crystal structure of a native MBL "neck and CRD head" has been previously shown in Chang et al. (1994) J Mol Biol. 241:125-7. A skill artisan can readily modify the identified CRD and fragments thereof to modulate its orientation and binding performance to carbohydrates on a microbe surface, *e.g*., by theoretical modeling and/or *in vitro* carbohydrate-binding experiments. In addition, based on the crystal structure of the native MBL "neck and CRD head", peptidomimetics that can effectively mimic at least a fragment of the CRD head and optionally the neck region can be also used as a carbohydrate recognition domain of the engineered microbe-binding molecule or MBL molecule described herein. One of skill in the art can readily determine such peptidomimetic structure without undue experimentations, using any methods known in the art and the known crystal structure.

In some embodiments, the carbohydrate recognition domain of the microbe-binding molecule can further comprise a portion of a carbohydrate-binding protein. However, in some circumstances, complement or coagulation activation induced by a carbohydrate-binding protein or a fragment thereof can be undesirable depending on various applications, *e.g., in vivo* administration for treatment of sepsis. In such embodiments, the portion of the carbohydrate-binding protein can exclude at least one of complement and coagulation activation regions. By way of example, when the carbohydrate-binding protein is mannose-binding lectin or a fragment thereof, the mannose-binding lectin or a fragment thereof can exclude at least one of the complement and coagulation activation regions located on the collagen-like region. In such embodiments, the mannose-binding lectin or a fragment thereof can exclude at least about one amino acid residue, including at least about two amino acid residues, at least about three amino acid residues, at least about four amino acid residues, at least about five amino acid residues, at least about six amino acid residues, at least about seven amino acid residues, at least about eight amino acid residues, at least about nine amino acid residues, at least about ten amino acid residues or more, around amino acid residue K55 or L56 of SEQ ID NO. 2. Exemplary amino sequences comprising K55 or L56 of SEQ ID NO. 2 that can be excluded from the engineered MBL molecule include, but are not limited to, EPGQGLRGLQGPPGKLGPPGNPGPSGS (SEQ ID NO. 16), GKLG (SEQ ID NO. 17), GPPGKLGPPGN (SEQ ID NO. 18), RGLQGPPGKL (SEQ ID NO. 19), GKLGPPGNPGPSGS (SEQ ID NO. 20), GLRGLQGPPGKLGPPGNPGP (SEQ ID NO. 21), or any fragments thereof.

MBL is known to bind strongly to mannose and N-acetylglucosamine sugars on fungi, gram-positive, and gram-negative bacteria. For example, MBL binds strongly to *Candida* spp., *Aspergillusfumigatus, Staphylococcus aureus,* and β hemolytic group A streptococci. MBL has intermediate affinity to *Escherichia coli, Klebsiella* spp., and *Haemophilus* influenzae type b. MBL binds weakly to β hemolytic group B *streptococci, Streptococcus pneumoniae,* and *Staphylococcus epidermidis.* Neth et al., 68 Infect. & Immun. 688 (2000). The capsular polysaccharide of *Neisseria meningitides* serogroup B, H .influenzae type b and *Cryptococcus neoformans* are thought to decrease MBL binding, as does bacterial endotoxin. Id.; Van Emmerik et al., 97 Clin. Exp. Immunol. 411 (1994); Schelenz et al., 63 Infect. Immun. 3360 (1995).

***Antimicrobial peptides:*** In some embodiments, the engineered microbe-binding molecule can further comprise an antimicrobial peptide or a functional fragment thereof. The antimicrobial peptide can be located at the N-terminal or C-terminal of the carbohydrate domain of the microbe surface-binding domain. Further, the antimicrobial peptide can be directly linked or via a linker (e.g., a peptide of 1, 2, 3, 4, 5, 6, 7, 8, 9, 10 or more amino acids) to the microbe surface-binding domain. In one embodiment, the antimicrobial peptide is linked to the C-terminal of the microbe surface-binding domain.

Antimicrobial peptides (also called host defense peptides) are an evolutionarily conserved component of the innate immune response and are found among all classes of life. Fundamental differences exist between prokaryotic and eukaryotic cells that may represent targets for antimicrobial peptides. These peptides are potent, broad spectrum antibiotics which demonstrate potential as novel therapeutic agents. Antimicrobial peptides have been demonstrated to kill Gram negative and Gram positive bacteria (including strains that are resistant to conventional antibiotics), mycobacteria (including Mycobacterium tuberculosis), enveloped viruses, fungi and even transformed or cancerous cells. Unlike the majority of conventional antibiotics it appears as though antimicrobial peptides may also have the ability to enhance immunity by functioning as immunomodulators.

Antimicrobial peptides are a unique and diverse group of molecules, which are divided into subgroups on the basis of their amino acid composition and structure. Antimicrobial peptides are generally between 12 and 50 amino acids. These peptides include two or more positively charged residues provided by arginine, lysine or, in acidic environments, histidine, and a large proportion (generally >50%) of hydrophobic residues. The secondary structures of these molecules follow 4 themes, including i) α-helical, ii) β-stranded due to the presence of 2 or more disulfide bonds, iii) β-hairpin or loop due to the presence of a single disulfide bond and/or cyclization of the peptide chain, and iv) extended. Many of these peptides are unstructured in free solution, and fold into their final configuration upon partitioning into biological membranes. It contains hydrophilic amino acid residues aligned along one side and hydrophobic amino acid residues aligned along the opposite side of a helical molecule. This amphipathicity of the antimicrobial peptides allows to partition into the membrane lipid bilayer. The ability to associate with membranes is a definitive feature of antimicrobial peptides although membrane permeabilization is not necessary. These peptides have a variety of antimicrobial activities ranging from membrane permeabilization to action on a range of cytoplasmic targets.

The modes of action by which antimicrobial peptides kill bacteria is varied and includes disrupting membranes, interfering with metabolism, and targeting cytoplasmic components. The initial contact between the peptide and the target organism is electrostatic, as most bacterial surfaces are anionic, or hydrophobic, such as in the antimicrobial peptide Piscidin. Their amino acid composition, amphipathicity, cationic charge and size allow them to attach to and insert into membrane bilayers to form pores by 'barrel-stave', 'carpet' or 'toroidal-pore' mechanisms. Alternately, they can penetrate into the cell to bind intracellular molecules which are crucial to cell living. Intracellular binding models includes inhibition of cell wall synthesis, alteration of the cytoplasmic membrane, activation of autolysin, inhibition of DNA, RNA, and protein synthesis, and inhibition of certain enzymes. However, in many cases, the exact mechanism of killing is not known. One emerging technique for the study of such mechanisms is dual polarization interferometry. In contrast to many conventional antibiotics these peptides appear to be bactericidal (bacteria killer) instead of bacteriostatic (bacteria growth inhibitor). In general the antimicrobial activity of these peptides is determined by measuring the minimal inhibitory concentration (MIC), which is the lowest concentration of drug that inhibits bacterial growth.

In addition to killing bacteria directly, antimicrobial peptides have been demonstrated to have a number of immunomodulatory functions that can be involved in the clearance of infection, including the ability to alter host gene expression, act as chemokines and/or induce chemokine production, inhibiting lipopolysaccharide induced pro-inflammatory cytokine production, promoting wound healing, and modulating the responses of dendritic cells and cells of the adaptive immune response. Animal models indicate that host defense peptides are crucial for both prevention and clearance of infection.

Antimicrobial peptides are produced by all species, including peptides from bacteria, from fungi, Hydra, insects, (mastoparan, poneratoxin, cecropin, moricin, melittin and so on), frogs (magainin, dermaseptin and others), and mammals (for example, cathelicidins, defensins and protegrins).

Antimicrobial peptides are excellent candidates for development as novel therapeutic agents and complements to conventional antibiotic therapy because in contrast to conventional antibiotics they do not appear to induce antibiotic resistance while they generally have a broad range of activity, are bactericidal as opposed to bacteriostatic and require a short contact time to induce killing. A number of naturally occurring peptides and their derivatives have been developed as novel anti-infective therapies for conditions as diverse as oral mucositis, lung infections associated with cystic fibrosis (CF), cancer, and topical skin infections. Pexiganan has been shown to be useful to treat infection related diabetic foot ulcer.

In the competition of bacterial cells and host cells with the antimicrobial peptides, antimicrobial peptides preferentially interact with the bacterial cell to the mammalian cells, which enables them to kill microorganisms without being significantly toxic to mammalian cells. Selectivity is a very important feature of the antimicrobial peptides and it can guarantee their function as antibiotics in host defense systems.

The cell membranes of bacteria are rich in acidic phospholipids, such as phosphatidylglycerol and cardiolipin. These phospholipid headgroups are heavily negatively charged. Therefore, the outmost leaflets of the bilayer which is exposed to the outside of the bacterial membranes are more attractive to the attack of the positively charged antimicrobial peptides. So the interaction between the positive charges of antimicrobial peptides and the negatively charged bacterial membranes is mainly the electrostatic interactions, which is the major driving force for cellular association. Besides, since antimicrobial peptides form structures with a positively charged face as well as a hydrophobic face, there are also some hydrophobic interactions between the hydrophobic regions of the antimicrobial peptides and the zwitterionic phospholipids (electrically neutral) surface of the bacterial membranes, which act only as a minor effect in this case.

In contrast, the outer part of the membranes of the plants and mammals is mainly composed of lipid without any net charges since most of the lipids with negatively charged headgroups are principally sequestered into the inner leaflet of the plasma membranes. Thus in the case of mammals cells, the outer surfaces of the membranes are usually made of zwitterionic phosphatidylcholine and sphingomyelin, even though a small portion of the membranes outer surfaces contain some negatively charged gangliosides. So the hydrophobic interaction between the hydrophobic face of amphipathic antimicrobial peptides and the zwitterionic phospholipids on the cell surface of mammalian cell membranes plays a major role in the formation of peptide-cell binding. However, the hydrophobic interaction is relatively weak when compared to the electrostatic interaction; thus, the antimicrobial peptides will preferentially interact with the bacterial membranes.

Exemplary types of antimicrobial peptides include, but are not limited to, anionic peptides (e.g., maximin H5 from amphibians and dermcidin from humans), generally rich in glutamic and aspartic acids; linear cationic a-helical peptides (e.g., cecropins, andropin, moricin, ceratotoxin and melittin from insects, magainin, dermaseptin, bombinin, brevinin-1,esculentins and buforin II from amphibians, CAP18 from rabbits, LL37 from humans), generally lack cysteine; catioinic peptide enriched for specific amino acid (e.g., abaecin, apidaecins from honeybees, prophenin from pigs, indolicidin from cattle), generally rich in proline, arginine, phenylalanine, glycine, or tryptophan; and anionic and cationic peptides that generally contain 1~3 disulfide bonds (e.g. brevinins (1bond), protegrin from pig, and tachyplesins from horseshoe crabs (2 bonds), defensins from humans (3 bonds), drosomycin in fruit flies (more than 3 bonds)

In some embodiments, the antimicrobial peptide comprises the amino acid sequence GSAWWSYWWTQWASELGSPGSP (SEQ ID NO: 54).

In some embodiments, when the microbe surface-binding domain of the engineered microbe-binding molecule is a carbohydrate recognition domain from a carbohydrate binding protein, and the engineered microbe-binding molecule further comprises an antimicrobial peptide. For example, when the microbe surface-binding domain of the engineered microbe-binding molecule is a carbohydrate recognition domain from the mannose-binding lection (MBL) and the engineered microbe-binding molecule further comprises an antimicrobial peptide.

***CD209:*** In some embodiments, the microbe-binding domain comprises the carbohydrate recognition domain of CD209 (Cluster of Differentiation 209) or a functional fragment thereof. CD209 is a protein which in humans is encoded by the CD209 gene. CD209 is also known as DC-SIGN (Dendritic Cell-Specific Intercellular adhesion molecule-3-Grabbing Non-integrin). DC-SIGN is a C-type lectin receptor present on both macrophages and dendritic cells. CD209 on macrophages recognises and binds to mannose type carbohydrates, a class of Pathogen associated molecular patterns PAMPs commonly found on viruses, bacteria and fungi. This binding interaction activates phagocytosis. On myeloid and pre-plasmacytoid dendritic cells CD209 mediates dendritic cell rolling interactions with blood endothelium and activation of CD4+ T cells, as well as recognition of pathogen haptens. CD209 is a C-type lectin and has a high affinity for the ICAM3 molecule. It binds various microorganisms by recognizing high-mannose-containing glycoproteins on their envelopes and especially functions as receptor for several viruses such as HIV and Hepatitis C. Binding to DC-SIGN can promote HIV and Hepatitis C virus to infect T-cell from dendritic cells. Thus binding to DC-SIGN is an essential process for HIV infection. Besides functioning as an adhesion molecule, recent study has also shown that CD209 can initiate innate immunity by modulating toll-like receptors, though the detailed mechanism is not yet known. DC-SIGN together with other C-type lectins is involved in recognition of tumors by dendritic cells. CD209 is also a potential engineering target for dendritic cell based cancer vaccine. Exemplary binding targts of CD209 include mannose and other sugars.

In some embodiments, the mcirob-binding domain comprises the carbohydrate recognition domain of CD209 and comprises the amino acid sequence of SEQ ID NO: 24.

***CD209L:*** In some embodiments, the microbe-binding domain comprises the carbohydrate recognition domain of CD209L or a functional fragment thereof. CD209Lis also called L-SIGN (liver/lymph node-specific intracellular adhesion molecules-3 grabbing non-integrin) and is a type II integral membrane protein that is 77% identical to CD209 antigen, an HIV gp120-binding protein. This protein, like CD209, efficiently binds both intercellular adhesion molecule 3 (ICAM3) and HIV-1 gp120, and enhances HIV-1 infection of T cells. The gene for L-SIGN is mapped to 19p13.3, in a cluster with the CD209 and CD23/FCER2 genes. Multiple alternatively spliced transcript variants have been found for this gene, but the biological validity of some variants has not been determined. Exemplary binding targts of CD209L include mannose and other sugars.

In some embodiments, the mcirob-binding domain comprises the carbohydrate recognition domain of L-SIGN and comprises the amino acid sequence of SEQ ID NO: 25.

***Pattern Recognition Receptors (PRRs):*** In some embodiments, the microbe-binding domain comprises a pattern recognition receptor or a functional fragment thereof. Pattern recognition receptors (PRRs) are a primitive part of the immune system. They are proteins expressed by cells of the innate immune system to identify pathogen-associated molecular patterns (PAMPs), which are associated with microbial pathogens or cellular stress, as well as damage-associated molecular patterns (DAMPs), which are associated with cell components released during cell damage. They are also called pathogen recognition receptors or primitive pattern recognition receptors because they evolved before other parts of the immune system, particularly before adaptive immunity. The microbe-specific molecules that are recognized by a given PRR are called pathogen-associated molecular patterns (PAMPs) and include bacterial carbohydrates (such as lipopolysaccharide or LPS, mannose), nucleic acids (such as bacterial or viral DNA or RNA), bacterial peptides (flagellin, ax21), peptidoglycans and lipoteichoic acids (from Gram positive bacteria), N-formylmethionine, lipoproteins and fungal glucans. Endogenous stress signals are called danger-associated molecular patterns (DAMPs) and include uric acid. Exemplary binding targets for PGRPs include peptidoglycan (PGN).

PRRs are classified according to their ligand specificity, function, localization and/or evolutionary relationships. On the basis of function, PRRs may be divided into endocytic PRRs or signaling PRRs. Signaling PRRs include the large families of membrane-bound Toll-like receptors and cytoplasmic NOD-like receptors. Endocytic PRRs promote the attachment, engulfment and destruction of microorganisms by phagocytes, without relaying an intracellular signal. These PRRs recognize carbohydrates and include mannose receptors of macrophages, glucan receptors present on all phagocytes and scavenger receptors that recognize charged ligands, are found on all phagocytes and mediate removal of apoptotic cells.

In some embodiments, the PRR is a CD14. CD14 acts as a co-receptor (along with the Toll-like receptor TLR 4 and MD-2) for the detection of bacterial lipopolysaccharide (LPS). CD14 can bind LPS only in the presence of lipopolysaccharide-binding protein (LBP). Although LPS is considered its main ligand, CD14 also recognizes other pathogen-associated molecular patterns. Exemplary binding targets for CD14 include, but are not limited to, lipopolysaccharide (LPS), peptidoglycan (PGN), and lipoteichoic acid (LTA).

In some embodiments, the microbe-binding domain is a PRR and has the amino acid of SEQ ID NO: 26.

***Peptidoglycan recognition proteins:*** Peptidoglycan recognition proteins (PGRPs) are pattern recognition molecules that are conserved from insects to mammals and recognize bacteria and their unique cell wall component, peptidoglycan (PGN). PGRPs have at least one carboxy-terminal PGRP domain (approximately 165 amino acids long), which is homologous to bacteriophage and bacterial type 2 amidases. Insects have up to 19 PGRPs, classified into short (S) and long (L) forms. The short forms are present in the hemolymph, cuticle, and fat-body cells, and sometimes in epidermal cells in the gut and hemocytes, whereas the long forms are mainly expressed in hemocytes.

Drosophila, mosquito, and mammals have families of 13, 7, and 4 PGRP genes, respectively, and some of these genes are alternatively spliced. PGRPs are differentially expressed in various cells and tissues, their expression is often upregulated by bacteria, and they mediate host responses to bacterial infections. Insect PGRPs have four known effector functions that are unique for insects: activation of prophenoloxidase cascade, activation of Toll receptor, activation of Imd pathway, and induction of phagocytosis. One function, amidase activity, is shared by some insect and mammalian PGRPs, whereas antibacterial activity of some mammalian PGRPs is unique for mammals. The expression of insect PGRPs is often upregulated by exposure to bacteria.

Mammals have a family of four PGRPs, which were initially named PGRP-S, PGRP-L, and PGRP-Iα and PGRP-Iβ (for 'short', 'long', or 'intermediate' transcripts, respectively), by analogy to insect PGRPs. Subsequently, the Human Genome Organization Gene Nomenclature Committee changed their symbols to PGLYRP-1, PGLYRP-2, PGLYRP-3, and PGLYRP-4, respectively. This terminology is also used for mouse PGRPs, and is beginning to be adopted for all vertebrate PGRPs. One mammalian PGRP, PGLYRP-2, is an *N*-acetylmuramoyl-L-alanine amidase that hydrolyzes bacterial peptidoglycan and reduces its proinflammatory activity; PGLYRP-2 is secreted from the liver into the blood and is also induced by bacteria in epithelial cells. The three remaining mammalian PGRPs are bactericidal proteins that are secreted as disulfide-linked homo- and hetero-dimers. PGLYRP-1 is expressed primarily in polymorphonuclear leukocyte granules and PGLYRP-3 and PGLYRP-4 are expressed in the skin, eyes, salivary glands, throat, tongue, esophagus, stomach, and intestine. These three proteins kill bacteria by interacting with cell wall peptidoglycan, rather than permeabilizing bacterial membranes as other antibacterial peptides do. Direct bactericidal activity of these PGRPs either evolved in the vertebrate (or mammalian) lineage or is yet to be discovered in insects. The mammalian PGLYRP-1, PGLYRP-2, PGLYRP-3, and PGLYRP-4 are also referred respectively as PGRP-1, PGRP-2, PGRP-3 and PGRP-4 herein.

In some embodiments, the microbe-binding domain comprises a PGRP or a fragment thereof. In some embodiments, the microbe-binding domain comprises a PGRP or a fragment thereof from human, mouse, bovine, or beetle. In some embodiments, the microbe-binding domain comprises a PGRP or a fragment therefore comprising the amino acid sequence selected from the group consisting of SEQ ID NO: 27, SEQ ID NO: 29, SEQ ID NO: 31, SEQ ID NO: 32, SEQ ID NO: 34, and SEQ ID NO: 35.

***From other species:*** In some embodiments, the microbe-binding domain comprises a carbohydrate recognition domain or a fragment thereof from shrimps. For example, the microbe-binding domain can comprise the carbohydrate recognition domain or a fragment thereof of Mj Lectin C or Mj Lectin B of shrimp. Exemplary binding targets for MjLectin C include the microbe cell wall. In some embodiments, the microbe-binding domain comprises the amino acid sequence SEQ ID NO: 23 or SEQ ID NO: 36.

In some embodiments, the microbe-binding domain comprises a carbohydrate recognition domain or a fragment thereof from wheat germ agglutinin or WGA. WGA is a lectin that protects wheat (Triticum vulgaris) from insects, yeast and bacteria. An agglutinin protein, it binds to N-acetyl-D-glucosamine and Sialic acid. N-acetyl-D-glucosamine in the natural environment of wheat is found in the chitin of insects, and the cell membrane of yeast & bacteria. WGA is found abundantly-but not exclusively-in the wheat kernel, where it got the 'germ' name from. In mammals the N-acetyl-D-glucosamine that WGA binds to is found in cartilage and cornea among other places. In those animals sialic acid is found in mucous membranes, e.g. the lining of the inner nose, and digestive tract. In solution, WGA exists mostly as a heterodimer of 38,000 Daltons. It is cationic at physiological pH. In some embodiments, the microbe-binding domain comprises a carbohydrate recognition domain or a fragment thereof from WGA and comprises the amino acid sequence of SEQ ID NO: 37.

In the tobacco hookworm, *Manduca sexta,* Peptidoglycan recognition proteins have been shown to function as stimulatory PRRs to enhance immune responses. Accordingly, in some embodiments, the microbe-binding domain comprises a PRR domain from *Manduca sexta.* In some embodiments, the microbe-binding domain comprises the amino acid sequence of SEQ ID NO: 30.

Without wishing to be bound by a theory, microbe-binding molecules described herein or modified versions thereof can act as broad-spectrum pathogen binding molecules. Accordingly, microbes and/or microbial matter present in a test sample can be captured using microbe-binding molecules described herein without identifying the microbe.

In some embodiments, the microbe surface-binding domain comprises an amino acid sequence selected from the sequences shown in **Table 2** and combination thereof.

**Table 2: Some exemplary microbe surface-binding domain amino acid sequences**

| | **SEQ ID NO:** | **Sequence** |
|---|---|---|
| MBL-antimicrobial-peptide | 22 | PDGDSSLAASERKALQTEMARIKKWLTFSLGKQVGNKFFLTNGE IMTFEKVKALCVKFQASVATPRNAAENGAIQNLIKEEAFLGITDE KTEGQFVDLTGNRLTYTNWNEGEPNNAGSDEDCVLLLKNGQW NDVPCSTSHLAVCEFPIGSAWWSYWWTQWASELGSPGSP |
| MjLectinC (Shrimp, *Marsupenaeus japonicus*) | 23 | ATCATFCTAQVNPCPNGYIVFWMDSVTPVCLKFAMYGKGTWT NLRMMCQAEGADLAKLDGNLHYQVIQYINNQRPDLQDEAFWIG GTDAASEGYWVWAMDGTQMDMSNPPWYPGQPNRGTIANYAC LYTPDFMFHSCDNDRKIYAICQI |
| CD209 | 24 | ERLCHPCPWEWTFFQGNCYFMSNSQRNWHDSITACKEVGAQLV VIKSAEEQNFLQLQSSRSNRFTWMGLSDLNQEGTWQWVDGSPL LPSFKQYWNRGEPNNVGEEDCAEFSGNGWNDDKCNLAKFWIC KKSAASCSRDE |
| CD209L | 25 | ERLCRHCPKDWTFFQGNCYFMSNSQRNWHDSVTACQEVRAQL VVIKTAEEQNFLQLQTSRSNRFSWMGLSDLNQEGTWQWVDGSP LSPSFQRYWNSGEPNNSGNEDCAEFSGSGWNDNRCDVDNYWIC KKPAACFRDE |
| CD14 | 26 | TTPEPCELDDEDFRCVCNFSEPQPDWSEAFQCVSAVEVEIHAGGL NLEPFLKRVDADADPRQYADTVKALRVRRLTVGAAQVPAQLLV GALRVLAYSRLKELTLEDLKITGTMPPLPLEATGLALSSLRLRNV SWATGRSWLAELQQWLKPGLKVLSIAQAHSPAFSCEQVRAFPA LTSLDLSDNPGLGERGLMAALCPHKFPAIQNLALRNTGMETPTG VCAALAAAGVQPHSLDLSHNSLRATVNPSAPRCMWSSALNSLN LSFAGLEQVPKGLPAKLRVLDLSCNRLNRAPQPDELPEVDNLTL DGNPFLVPGTALPHEGSMNSGVVPACARSTLSVGVSGTLVLLQG ARGFA |
| PGRP-1 (mouse) | 27 | CSFIVPRSEWRALPSECSSRLGHPVRYVVISHTAGSFCNSPDSCEQ QARNVQHYHKNELGWCDVAYNFLIGEDGHVYEGRGWNIKGDH TGPIWNPMSIGITFMGNFMDRVPAKRALRAALNLLECGVSRGFL RSNYEVKGHRDVQSTLSPGDQLYQVIQSWEHYRE |
| PGRP-2 (Beetle) | 28 | PSPGCPTIVSKNRWGGQQASQVQYTVKPLKYVIIHHTSTPTCTNE DDCSRRLVNIQDYHMNRLDFDDIGYNFMIGGDGQIYEGAGWHK EGAHARGWNSKSLGIGFIGDFQTNLPSSKQLDAGKKFLECAVEK GEIEDTYKLIGARTVRPTDSPGTLLFREIQTWRGFTRNP |
| PGRP-4 | 29 | DSSWNKTQAKQVSEGLQYLFENISQLTEKGLPTDVSTTVSRKAW |
| (human) | | GAEAVGCSIQLTTPVNVLVIHHVPGLECHDQTVCSQRLRELQAH HVHNNSGCDVAYNFLVGDDGRVYEGVGWNIQGVHTQGYNNIS LGFAFFGTKKGHSPSPAALSAMENLITYAVQKGHLSSSYVQPLL GKGENCLAPRQKTSLKKACPGVVPRSVWGARETHCPRMTLPAK YGIIIHTAGRTCNISDECRLLVRDIQSFYIDRLKSCDIGYNFLVGQ DGAIYEGVGWNVQGSSTPGYDDIALGITFMGTFTGIPPNAAALE AAQDLIQCAMVKGYLTPNYLLVGHSDVARTLSPGQALYNIISTW PHFKH |
| GBP-1 (Tobacco Hookworm) | 30 | PSPCLEVPDAKLEAIYPKGLRVSIPDDGYTLFAFHGKLNEEMEGL EAGHWSRDITKAKNGRWIFRDRNAKLKIGDKIYFWTYILKDGLG YRQDNGEWTVTGYVNEDGEPLDANFEPRSTASTAAPPQAGAGQ APGPSYPCELSVSEVSVPGFVCKGQMLFEDNFNKPLADGRIWTP EIMFPGEPDYPFNVYMKETDNLHVGNGNLVIKPMPLVTAFGEDA IWKTLDLSDRCTGLLGTAQCKRDPSDAIIVPPIVTAKINTKKTFAF KYGRVEISAKMPRGDWLVPLIQLEPVNKNYGIRNYVSGLLRVAC VKGNTEYIKTLVGGPIMSEAEPYRTANLKEFISNEPWTNEFHNYT LEWSPDAITMAVDGIVYGRVTAPAGGFYKEANEQNVEAAARWI QGSNIAPFDDMFYISLGMDVGGVHEFPDEAINKPWKNTATKAM VNFWNARSQWNPTWLESEKALLVDYVRVYAL |
| PGRP-1 (human) | 31 | QETEDPACCSPIVPRNEWKALASECAQHLSLPLRYVVVSHTAGS SCNTPASCQQQARNVQHYHMKTLGWCDVGYNFLIGEDGLVYE GRGWNFTGAHSGHLWNPMSIGISFMGNYMDRVPTPQAIRAAQG LLACGVAQGALRSNYVLKGHRDVQRTLSPGNQLYHLIQNWPHY RSP |
| PGRP-3short (human) | 32 | CPNIIKRSAWEARETHCPKMNLPAKYVIIIHTAGTSCTVSTDCQT VVRNIQSFHMDTRNFCDIGYHFLVGQDGGVYEGVGWHIQGSHT YGFNDIALGIAFIGYFVEKPPNAAALEAAQDLIQCAVVEGYLTPN YLLMGHSDVVNILSPGQALYNIISTWPHFKH |
| PGRP (cow) | 33 | QDCGSIVSRGKWGALASKCSQRLRQPVRYVVVSHTAGSVCNTP ASCQRQAQNVQYYHVRERGWCDVGYNFLIGEDGLVYEGRGWN TLGAHSGPTWNPIAIGISFMGNYMHRVPPASALRAAQSLLACGA ARGYLTPNYEVKGHRDVQQTLSPGDELYKIIQQWPHYRRV |
| PGRP-2 (human) | 34 | CPAIHPRCRWGAAPYRGRPKLLQLPLGFLYVHHTYVPAPPCTDF TRCAANMRSMQRYHQDTQGWGDIGYSFVVGSDGYVVYEGRGW HWVGAHTLGHNSRGFGVAIVGNYTAALPTEAALRTVRDTLPSC AVRAGLLRPDYALLGHRQLVRTDCPGDALFDLLRTWPHF |
| PGRP-3 (human) | 35 | PTIVSRKEWGARPLACRALLTLPVAYIITDQLPGMQCQQQSVCS QMLRGLQSHSVYTIGWCDVAYNFLVGDDGRVYEGVGWNIQGL HTQGYNNISLGIAFFGNKIGSSPSPAALSAAEGLISYAIQKGHLSP RYIQPLLLKEETCLDPQHPVMPRKVCPNIIKRSAWEARETHCPK MNLPAKYVIIIHTAGTSCTVSTDCQTVVRNIQSFHMDTRNFCDIG YHFLVGQDGGVYEGVGWHIQGSHTYGFNDIALGIAFIGYFVEKP PNAAALEAAQDLIQCAVVEGYLTPNYLLMGHSDVVNILSPGQAL YNIISTWPHFKH |
| MjLectinB (shrimp) | 36 | AWGGATATGPRKEAGDHVRNDVCPHPFVDINGRCLFVDNFAHL NWDAARTFCQGFQGDLVTLDEANLLGYIVDFIHQEGLTERSYWI GGSDRTSEGTWVWTDGSSVRMGTPTWGVDGETQQPTGGTSEN CIGLHKDNFFFFNDFSCNNEMSLICEFNM |
| WGA | 37 | RCGEQGSNMECPNNLCCSQYGYCGMGGDYCGKGCQNGACWT SKRCGSQAGGATCPNNHCCSQYGHCGFGAEYCGAGCQGGPCR ADIKCGSQSGGKLCPNNLCCSQWGFCGLGSEFCGGGCQSGACST DKPCGKDAGGRVCTNNYCCSKWGSCGIGPGYCGAGCQSGGCD AVFAGAITANSTLLAE |

In some embodiments, the microbe-binding molecule comprises the amino acid sequence selected from the group consisting of the sequences shown in **Table 3** and any combination thereof.

**Table 3: Some exemplary engineered microbe-bindine molecule amino acid sequences**

| | **SEQ ID NO:** | **Sequence** |
|---|---|---|
| FcMBL-peptide | 38 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAPDGDSSLAASERKALQTEMARIKKWLTF SLGKQVGNKFFLTNGEIMTFEKVKALCVKFQASVATPRNAAEN GAIQNLIKEEAFLGITDEKTEGQFVDLTGNRLTYTNWNEGEPNN AGSDEDCVLLLKNGQWNDVPCSTSHLAVCEFPIGSAWWSYWW TQWASELGSPGSP |
| FcMjLectinC (Shrimp, *Marsupenaeus japonicus*) | 39 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAATCATFCTAQVNPCPNGYIVFWMDSVTP VCLKFAMYGKGTWTNLRMMCQAEGADLAKLDGNLHYQVIQYI NNQRPDLQDEAFWIGGTDAASEGYWVWAMDGTQMDMSNPPW YPGQPNRGTIANYACLYTPDFMFHSCDNDRKIYAICQI |
| FcCD209 | 40 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAERLCHPCPWEWTFFQGNCYFMSNSQRN WHDSITACKEVGAQLVVIKSAEEQNFLQLQSSRSNRFTWMGLSD LNQEGTWQWVDGSPLLPSFKQYWNRGEPNNVGEEDCAEFSGN GWNDDKCNLAKFWICKKSAASCSRDE |
| FcCD209L | 41 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAERLCRHCPKDWTFFQGNCYFMSNSQRN WHDSVTACQEVRAQLVVIKTAEEQNFLQLQTSRSNRFSWMGLS DLNQEGTWQWVDGSPLSPSFQRYWNSGEPNNSGNEDCAEFSGS GWNDNRCDVDNYWICKKPAACFRDE |
| FcCD14 | 42 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGATTPEPCELDDEDFRCVCNFSEPQPDWSEA FQCVSAVEVEIHAGGLNLEPFLKRVDADADPRQYADTVKALRV |
| | | RRLTVGAAQVPAQLLVGALRVLAYSRLKELTLEDLKITGTMPPL PLEATGLALSSLRLRNVSWATGRSWLAELQQWLKPGLKVLSIAQ AHSPAFSCEQVRAFPALTSLDLSDNPGLGERGLMAALCPHKFPAI QNLALRNTGMETPTGVCAALAAAGVQPHSLDLSHNSLRATVNP SAPRCMWSSALNSLNLSFAGLEQVPKGLPAKLRVLDLSCNRLNR APQPDELPEVDNLTLDGNPFLVPGTALPHEGSMNSGVVPACARS TLSVGVSGTLVLLQGARGFA |
| FcPGRP-1 (mouse) | 43 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGACSFIVPRSEWRALPSECSSRLGHPVRYVV ISHTAGSFCNSPDSCEQQARNVQHYHKNELGWCDVAYNFLIGED GHVYEGRGWNIKGDHTGPIWNPMSIGITFMGNFMDRVPAKRAL RAALNLLECGVSRGFLRSNYEVKGHRDVQSTLSPGDQLYQVIQS WEHYRE |
| FcPGRP-2 (Beetle) | 44 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAPSPGCPTIVSKNRWGGQQASQVQYTVKP LKYVIIHHTSTPTCTNEDDCSRRLVNIQDYHMNRLDFDDIGYNF MIGGDGQIYEGAGWHKEGAHARGWNSKSLGIGFIGDFQTNLPSS KQLDAGKKFLECAVEKGEIEDTYKLIGARTVRPTDSPGTLLFREI QTWRGFTRNP |
| FcPGRP-4 (human) | 45 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGDSSWNKTQAKQVSEGLQYLFENISQLTEK GLPTDVSTTVSRKAWGAEAVGCSIQLTTPVNVLVIHHVPGLECH DQTVCSQRLRELQAHHVHNNSGCDVAYNFLVGDDGRVYEGVG WNIQGVHTQGYNNISLGFAFFGTKKGHSPSPAALSAMENLITYA VQKGHLSSSYVQPLLGKGENCLAPRQKTSLKKACPGVVPRSVW GARETHCPRMTLPAKYGIIHTAGRTCNISDECRLLVRDIQSFYID RLKSCDIGYNFLVGQDGAIYEGVGWNVQGSSTPGYDDIALGITF MGTFTGIPPNAAALEAAQDLIQCAMVKGYLTPNYLLVGHSDVA RTLSPGQALYNIISTWPHFKH |
| FcGBP-1 (Tobacco Hookworm) | 46 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAPSPCLEVPDAKLEAIYPKGLRVSIPDDGY TLFAFHGKLNEEMEGLEAGHWSRDITKAKNGRWIFRDRNAKLK IGDKIYFWTYILKDGLGYRQDNGEWTVTGYVNEDGEPLDANFE PRSTASTAAPPQAGAGQAPGPSYPCELSVSEVSVPGFVCKGQML FEDNFNKPLADGRIWTPEIMFPGEPDYPFNVYMKETDNLHVGNG NLVIKPMPLVTAFGEDAIWKTLDLSDRCTGLLGTAQCKRDPSDA IIVPPIVTAKINTKKTFAFKYGRVEISAKMPRGDWLVPLIQLEPVN KNYGIRNYVSGLLRVACVKGNTEYIKTLVGGPIMSEAEPYRTAN |
| | | LKEFISNEPWTNEFHNYTLEWSPDAITMAVDGIVYGRVTAPAGG FYKEANEQNVEAAARWIQGSNIAPFDDMFYISLGMDVGGVHEF PDEAINKPWKNTATKAMVNFWNARSQWNPTWLESEKALLVDY VRVYAL |
| FcPGRP-1 (human) | 47 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAQETEDPACCSPIVPRNEWKALASECAQH LSLPLRYVVVSHTAGSSCNTPASCQQQARNVQHYHMKTLGWC DVGYNFLIGEDGLVYEGRGWNFTGAHSGHLWNPMSIGISFMGN YMDRVPTPQAIRAAQGLLACGVAQGALRSNYVLKGHRDVQRT LSPGNQLYHLIQNWPHYRSP |
| FcPGRP-3short (human) | 48 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGACPNIIKRSAWEARETHCPKMNLPAKYVII IHTAGTSCTVSTDCQTVVRNIQSFHMDTRNFCDIGYHFLVGQDG GVYEGVGWHIQGSHTYGFNDIALGIAFIGYFVEKPPNAAALEAA QDLIQCAVVEGYLTPNYLLMGHSDVVNILSPGQALYNIISTWPHF KH |
| FcPGRP (cow) | 49 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAQDCGSIVSRGKWGALASKCSQRLRQPVR YVVVSHTAGSVCNTPASCQRQAQNVQYYHVRERGWCDVGYNF LIGEDGLVYEGRGWNTLGAHSGPTWNPIAIGISFMGNYMHRVPP ASALRAAQSLLACGAARGYLTPNYEVKGHRDVQQTLSPGDELY KIIQQWPHYRRV |
| FcPGRP-2 (human) | 50 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGACPAIHPRCRWGAAPYRGRPKLLQLPLGF LYVHHTYVPAPPCTDFTRCAANMRSMQRYHQDTQGWGDIGYS FVVGSDGYVYEGRGWHWVGAHTLGHNSRGFGVAIVGNYTAAL PTEAALRTVRDTLPSCAVRAGLLRPDYALLGHRQLVRTDCPGDA LFDLLRTWPHF |
| FcPGRP-3 (human) | 51 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAPTIVSRKEWGARPLACRALLTLPVAYIIT DQLPGMQCQQQSVCSQMLRGLQSHSVYTIGWCDVAYNFLVGD DGRVYEGVGWNIQGLHTQGYNNISLGIAFFGNKIGSSPSPAALSA AEGLISYAIQKGHLSPRYIQPLLLKEETCLDPQHPVMPRKVCPNII KRSAWEARETHCPKMNLPAKYVIIIHTAGTSCTVSTDCQTVVRNI |
| | | QSFHMDTRNFCDIGYHFLVGQDGGVYEGVGWHIQGSHTYGFND IALGIAFIGYFVEKPPNAAALEAAQDLIQCAVVEGYLTPNYLLMG HSDVVNILSPGQALYNIISTWPHFKH |
| FcMjLectinB (shrimp) | 52 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGAAWGGATATGPRKEAGDHVRNDVCPHPF VDINGRCLFVDNFAHLNWDAARTFCQGFQGDLVTLDEANLLGY IVDFIHQEGLTERSYWIGGSDRTSEGTWVWTDGSSVRMGTPTW GVDGETQQPTGGTSENCIGLHKDNFFFFNDFSCNNEMSLICEFN M |
| FcWGA | 53 | AKTEPKSSDKTHTCPPCPAPELLGGPSVFLFPPKPKDTLMISRTPE VTCVVVDVSHEDPEVKFNWYVDGVEVHNAKTKPREEQYDSTY RVVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHEA LHNHYTQKSLSLSPGARCGEQGSNMECPNNLCCSQYGYCGMGG DYCGKGCQNGACWTSKRCGSQAGGATCPNNHCCSQYGHCGFG AEYCGAGCQGGPCRADIKCGSQSGGKLCPNNLCCSQWGFCGLG SEFCGGGCQSGACSTDKPCGKDAGGRVCTNNYCCSKWGSCGIG PGYCGAGCQSGGCDAVFAGAITANSTLLAE |

In some embodiments, the microbe-binding molecule comprises at least a portion of an immunoglobulin, *e.g*., IgA, IgD, IgE, IgG and IgM including their subclasses (*e.g.,* IgG₁), or a modified molecule or recombinant thereof. Immunoglobulins, include IgG, IgA, IgM, IgD, IgE. An immunoglobulin portion (e.g., fragments) and immunoglobulin derivatives include but are not limited to single chain Fv (scFv), diabodies, Fv, and (Fab')₂, triabodies, Fc, Fab, CDR1, CDR2, CDR3, combinations of CDR's, variable regions of the light or heavy Ig chains, tetrabodies, bifunctional hybrid antibodies, framework regions, constant regions, and the like (*see,* Maynard et al., (2000) Ann. Rev. Biomed. Eng. 2:339-76; Hudson (1998) Curr. Opin. Biotechnol. 9:395-402). In one embodiment, an immunoglobulin molecule can encompass immunoglobulin ortholog genes, which are genes conserved among different biological species such as humans, dogs, cats, mice, and rats, that encode proteins (for example, homologs (including splice variants), mutants, and derivatives) having biologically equivalent functions as the human-derived protein. Immunoglobulin orthologs include any mammalian ortholog of IgG, IgA, IgM, IgD, IgE inclusive of the ortholog in humans and other primates, experimental mammals (such as mice, rats, hamsters and guinea pigs), mammals of commercial significance (such as horses, cows, camels, pigs and sheep), and also companion mammals (such as domestic animals, e.g., rabbits, ferrets, dogs, and cats), or a camel, llama, or shark.

For example, the Fc portion of an FcMBL molecule, or the Fc portion of any microbe-binding molecule of the instant invention, can be replaced with any of the immunoglobulin fragments described herein.

In some embodiments, the microbe-binding molecule comprises at least a portion of an adhesion molecule, or a modified molecule or recombinant thereof. Non-limiting examples of adhesion molecules include: cell adhesion molecules (e.g. cadherins, selectins, integrins, addressins, lymphocyte homing receptors (e.g. CD-34, GLYCAM-1)); Synaptic Cell Adhesion Molecules(SynCAMs); Neural Cell Adhesion Molecules (NCAMs); Intercellular Cell Adhesion Molecules (ICAM-1); Vascular Cell Adhesion Molecules (VCAM-1); Platelet-endothelial Cell Adhesion Molecules (PECAM-1). In one embodiment, an adhesion molecule can encompass ortholog genes discussed herein.

Other non-limiting examples of microbe-binding molecules include L1, CHL1, MAG, Nectins and nectin-like molecules, CD2, CD48, SIGLEC family members (e.g. CD22, CD83), and CTX family members (e.g. CTX, JAMs, BT-IgSF, CAR, VSIG, ESAM)).

In some embodiments, the microbe-binding molecule comprises at least a portion of heparin. Heparin binds various proteins including growth factors (*e.g*., FGF1, FGF2, FGF7), serine proteases (*e.g*., Thrombin, Factor Xa) and serine protease inhibitors (such as Antithrombin). In some embodiments, the microbe-binding molecule comprises at least a portion of a glycosaminoglycan (GAG). In some embodiments, the microbe-binding molecule comprises at least one glycosaminoglycan (GAG). A GAG includes, but is not limited to a heparin/heparan sulfate GAG (HSGAG), a chondroitin/dermatan sulfate GAG (CSGAG), a keratan sulfate GAG, and hyaluronic acid. In some embodiments, the microbe-binding molecule comprises at least a portion of Hemopexin. Hemopexin beinds Heme.

In other embodiments, the microbe-binding molecule can comprise at least a portion of a receptor molecule, or a modified molecule or recombinant thereof. Non-limiting examples of a receptor molecule include: an extracellular receptor molecule (e.g. nicotinic acetylcholine receptor, glycine receptor, GABA receptors,glutamate receptor, NMDA receptor, AMPA receptor, Kainate receptor, 5-HT3 receptor, P2X receptor); an intracellular receptor molecule (e.g. a cyclic nucleotide-gated ion channel, IP3 receptor, intracellular ATP receptor, ryanodine receptor); an immune receptor molecule (e.g. pattern recognition receptors, toll-like receptors, killer activated and killer inhibitor receptors, complement receptors, Fc receptors, B cell receptors and T cell receptors); a G protein coupled receptor molecule, a virus receptor molecule (e.g., CAR -Coxsackie Adenovirus Receptor); an iron scavenging receptor molecule (e.g., LRP/CD91, CD163). In one embodiment, a receptor molecule can encompass ortholog genes discussed herein. In other embodiments, the microbe-binding molecule comprises a hormone receptor. In some embodiments, the hormone receptor is a peptide hormone receptor or a steroid hormone receptor. The peptide hormone receptor can be a cell surface receptor or transmembrane receptor that binds to its cognate hormone ligand. The steroid hormone receptor is a soluble receptor that binds to its cognate hormone ligand. In one embodiment, the peptide hormone receptor comprises a thyroid-stimulating hormone receptor, a follicle-stimulating hormone receptor, a leutinizing hormone receptor, a glucagon receptor, or an insulin receptor. In another embodiment, the receptors comprises those for glucocorticoids, estrogens, androgens, thyroid hormone (T₃), calcitriol (vitamin D), and the retinoids (vitamin A). In some embodiments, the transmembrane receptor is a G-protein coupled receptor, which binds to Gs or Gi proteins.

In further embodiments, the microbe-binding molecule comprises at least a portion of a ligand that enriches for circulating tumor cells, for example antibodies to tumor cell markers. Ligands that enrich for circulating tumor cells include, but are not limited to, antibodies to EpCAM, antibodies to CD46, antibodies to CD24, and antibodies to CD133. In further embodiments, the microbe-binding molecule comprises a ligand that enriches for fetal cells in maternal circulation. Ligands that enrich for fetal cells include, but are not limited to, antibodies to CD71, and antibodies to glycophorin-A. In further embodiments, the microbe-binding molecule comprises at least a portion of a ligand that enriches for circulating leukocytes, such as antibodies to CD45, and antibodies to CD15. In yet other embodiments, the microbe-binding molecule comprises at least a portion of a non-immunogobulin binding protein engineered for specific binding properties. For example, the binding proteins may contain ankyrin repeats, or the binding proteins can be anticalins. In one embodiment, anticalins can be used to screen libraries for binding to a target molecule (*e.g., see* Gebauer, M., & Skerra, A. (2009). Engineered protein scaffolds as next-generation antibody therapeutics. Current opinion in chemical biology, 13(3), 245-255; and Löfblom, J., Frejd, F. Y., & Ståhl, S. (2011). Non-immunoglobulin based protein scaffolds. Current Opinion in Biotechnology, 22(6), 843-848, each of which are incorporated by reference in their entireties)

For example, the Fc portion or any immunoglobulin fragment described herein can be coupled to any microbe-binding molecule embraced by the instant invention which targets some specific ligand, cell, or combination thereof.

### Linkers

As used herein, the term "linker" generally refers to a molecular entity that can directly or indirectly connect at two parts of a composition, *e*.*g*., at least one microbe surface-binding domain and at least one substrate-binding domain, or at least one enzyme and at least one microbe-binding molecule. In some embodiments, the linker can directly or indirectly connect to one or more microbe surface-binding domains. In some embodiments, the linker can directly or indirectly connect to one or more microbe surface-binding domains. Without limitations, in some embodiments, the linker can also provide binding sites to one or more microbes, microbial matter, and/or other target molecules. In such embodiments, the microbe-binding sites on the linker can bind to the same types and/or species of microbes as the microbes bind to a microbe-surface-binding domain. Alternatively or additionally, the microbe-binding sites on the linker can capture different types and/or species of microbes than the ones that bind to a microbe surface-binding domain described herein.

Linker can be attached to the N- or C-terminal of the microbe surface-binding domain. Further, the linker can be linked directly or via another linker (e.g., a peptide of one, two, three, four, five, six, seven, eight, nine, ten or more amino acids) to the microbe surface-binding domain. In one embodiment, the linker is attached to the N-terminal of the microbe surface-binding domain.

Linkers can be configured according to a specific need, *e*.*g*., based on at least one of the following characteristics. By way of example only, in some embodiments, linkers can be configured to have a sufficient length and flexibility such that it can allow for a microbe surface-binding domain to orient accordingly with respect to at least one carbohydrate on a microbe surface. In some embodiments, linkers can be configured to allow multimerization of at least two engineered microbe-binding molecules (*e.g*., to from a di-, tri-, tetra-, penta-, or higher multimeric complex) while retaining biological activity (*e.g*., microbe-binding activity). In some embodiments, linkers can be configured to facilitate expression and purification of the engineered microbe-binding molecule described herein. In some embodiments, linkers can be configured to provide at least one recognition site for proteases or nucleases. In addition, linkers are preferably non-reactive with the functional components of the engineered molecule described herein (*e.g*., minimal hydrophobic or charged character to react with the functional protein domains such as a microbe surface-binding domain or a substrate-binding domain).

In some embodiments, a linker can be configured to have any length in a form of a peptide, peptidomimetic, an aptamer, a protein, a nucleic acid (*e*.*g*., DNA or RNA), polyethylene glycol diol, ethylene glycol, polypropylene gylcol, perfluoroglutamic acid, perfluoropolyether (Krytox), hydroxyl-terminated, amine-terminated, methyl-terminated, or hydrocarbon-terminated polydimethylsiloxane, polysulfone, polyethersulfone, polymethylmethacrylate, polyacrylimide, polybutadiene, water, formamide, gluteraldehyde, acetic acid, cellulose, keratin, chitosan, chitin, polylactic acid, polysaccharides, saccharides, proteoglycans, heparin, heparin sulfate, poly(N-isopropylacrylamide), poly(lactic-co-glycolic acid), polyurethane, metals and metal oxides (e.g. ferric oxide, ferrous oxide, cupric oxide, aluminum, aluminum oxide, zinc oxide, zinc, magnesium, calcium, and the like), alginate, silk, glycosaminoglycans, keratin, silicates, phospholipids, aliphatic hydrocarbons, aromatic hydrocarbons, phenyl groups or any combinations thereof. In some embodiments, the nucleic acid linker can vary from about 1 to about 1000 nucleic acids long, from about 10 to about 500 nucleic acids long, from about 30 to about 300 nucleic acids long, or from about 50 to about 150 nucleic acids long. In some embodiments, the peptidyl linker can vary from about 1 to about 1000 amino acids long, from about 10 to about 500 amino acids long, from about 30 to about 300 amino acids long, or from about 50 to about 150 amino acids long. Longer or shorter linker sequences can be also used for the engineered microbe-binding molecules described herein. In one embodiment, the peptidyl linker has an amino acid sequence of about 200 to 300 amino acids in length.

In some embodiments, a peptide or nucleic acid linker can be configured to have a sequence comprising at least one of the amino acids selected from the group consisting of glycine (Gly), serine (Ser), asparagine (Asn), threonine (Thr), methionine (Met) or alanine (Ala), or at least one of codon sequences encoding the aforementioned amino acids (*i.e.,* Gly, Ser, Asn, Thr, Met or Ala). Such amino acids and corresponding nucleic acid sequences are generally used to provide flexibility of a linker. However, in some embodiments, other uncharged polar amino acids *(e.g.,* Gln, Cys or Tyr), nonpolar amino acids (*e.g.,* Val, Leu, Ile, Pro, Phe, and Trp), or nucleic acid sequences encoding the amino acids thereof can also be included in a linker sequence. In alternative embodiments, polar amino acids or nucleic acid sequence thereof can be added to modulate the flexibility of a linker. One of skill in the art can control flexibility of a linker by varying the types and numbers of residues in the linker. See, *e.g.,* Perham, 30 Biochem. 8501 (1991); Wriggers et al., 80 Biopolymers 736 (2005).

In alternative embodiments, a linker can be a chemical linker of any length. In some embodiments, chemical linkers can comprise a direct bond or an atom such as oxygen or sulfur, a unit such as NH, C(O), C(O)NH, SO, SO₂, SO₂NH, or a chain of atoms, such as substituted or unsubstituted C₁-C₆ alkyl, substituted or unsubstituted C₂-C₆ alkenyl, substituted or unsubstituted C₂-C₆ alkynyl, substituted or unsubstituted C₆-C₁₂ aryl, substituted or unsubstituted C₅-C₁₂ heteroaryl, substituted or unsubstituted C₅-C₁₂ heterocyclyl, substituted or unsubstituted C₃-C₁₂ cycloalkyl, where one or more methylenes can be interrupted or terminated by O, S, S(O), SO₂, NH, or C(O). In some embodiments, the chemical linker can be a polymer chain (branched or linear).

In some embodiments where the linker is a peptide, such peptidyl linker can comprise at least a portion of an immunoglobulin, *e*.*g*., IgA, IgD, IgE, IgG and IgM including their subclasses (e.g., IgG₁), or a modified molecule or recombinant thereof. Immunoglobulins, include IgG, IgA, IgM, IgD, IgE. An immunoglobulin portion (e.g., fragments) and immunoglobulin derivatives include but are not limited to single chain Fv (scFv), diabodies, Fv, and (Fab')₂, triabodies, Fc, Fab, CDR1, CDR2, CDR3, combinations of CDR's, variable regions of the light or heavy Ig chains, tetrabodies, bifunctional hybrid antibodies, framework regions, constant regions, and the like (*see,* Maynard et al., (2000) Ann. Rev. Biomed. Eng. 2:339-76; Hudson (1998) Curr. Opin. Biotechnol. 9:395-402). In one embodiment, an immunoglobulin molecule can encompass immunoglobulin ortholog genes, which are genes conserved among different biological species such as humans, dogs, cats, mice, and rats, that encode proteins (for example, homologs (including splice variants), mutants, and derivatives) having biologically equivalent functions as the human-derived protein. Immunoglobulin orthologs include any mammalian ortholog of IgG, IgA, IgM, IgD, IgE inclusive of the ortholog in humans and other primates, experimental mammals (such as mice, rats, hamsters and guinea pigs), mammals of commercial significance (such as horses, cows, camels, pigs and sheep), and also companion mammals (such as domestic animals, e.g., rabbits, ferrets, dogs, and cats), or a camel, llama, or shark.

In some embodiments, the peptide linker can comprise a portion of fragment crystallization (Fc) region of an immunoglobulin or a modified thereof. In such embodiments, the portion of the Fc region that can be used as a linker can comprise at least one region selected from the group consisting of a hinge region, a CH2 region, a CH3 region, and any combinations thereof. By way of example, in some embodiments, a CH2 region can be excluded from the portion of the Fc region as a linker. In one embodiment, Fc linker comprises a hinge region, a CH2 domain and a CH3 domain, *e*.*g*., Fc IgG. Such Fc linker can be used to facilitate expression and purification of the engineered microbe-binding molecules described herein. The N terminal Fc has been shown to improve expression levels, protein folding and secretion of the fusion partner. In addition, the Fc has a staphylococcal protein A binding site, which can be used for one-step purification protein A affinity chromatography. See Lo KM et al. (1998) Protein Eng. 11: 495-500. Further, the protein A binding site can be used to facilitate binding of protein A-expressing or protein G-expressing microbes in the absence of calcium ions. Such binding capability can be used to develop methods for distinguishing protein A-expressing microbes (*e*.*g*., *S. aureus*) from non-protein A-expressing or non-protein G-expressing microbes (*e.g., E. coli*) present in a test sample, and various embodiments of such methods will be described in detail later. Further, such Fc linker have a molecule weight above a renal threshold of about 45kDa, thus reducing the possibility of engineered microbe-binding molecules being removed by glomerular filtration. Additionally, the Fc linker can allow dimerization of two engineered microbe-binding molecules to form a dimer, *e*.*g*., the dimeric engineered MBL molecule.

In one embodiment, the amino acid sequence of a Fc region comprises SEQ ID NO: 56.

SEQ ID NO: 56 depicts the amino acid sequence of a Fc domain:

1 MWGWKCLLFW AVLVTATLCT ARPAPTLPEQ AQQSTRADLG PGEPKSCDKT HTCPPCPAPE
61 LLGGPSVFLF PPKPKDTLMI SRTPEVTCVV VDVSHEDPEV KFNWYVDGVE VHNAKTKPRE
121 EQYNSTYRVV SVLTVLHQDW LNGKEYKCKV SNKALPAPIE KTISKAKGQP REPQVYTLPP
181 SRDELTKNQV SLTCLVKGFY PSDIAVEWES NGQPENNYKT TPPVLDSDGS FFLYSKLTVD
241 KSRWQQGNVF SCSVMHEALH NHYTQKSLSL SPGK

In some embodiments where the linker comprises a Fc region or a fragment thereof, the Fc region or a fragment thereof can comprise at least one mutation, *e*.*g*., to modify the performance of the engineered microbe-binding molecules. For example, in some embodiments, a half-life of the engineered microbe-binding molecules described herein can be increased, *e*.*g*., by mutating an amino acid lysine (K) at the residue 232 of SEQ ID NO. 9 to alanine (A). Other mutations, e.g., located at the interface between the CH2 and CH3 domains shown in Hinton et al (2004) J Biol Chem. 279:6213-6216 and Vaccaro C. et al. (2005) Nat Biotechnol. 23: 1283-1288, can be also used to increase the half-life of the IgG1 and thus the engineered microbe-binding molecules.

In some embodiments, the linker can be albumin, transferrin or a fragment thereof. Such linkers can be used to extend the plasma half-life of the engineered microbe-binding molecules and thus are good for *in vivo* administration. *See* Schmidt SR (2009) Curr Opin Drug Discov Devel. 12: 284.

When the engineered microbe-binding molecules are used as therapeutics *in vivo,* the linker can be further modified to modulate the effector function such as antibody-dependent cellular cytotoxicity (ADCC) and complement-dependent cytotoxicity (CDC). By way of example only, the Fc region for use as a linker can mediate ADCC and CDC. In ADCC, the Fc region can generally bind to Fc receptors on the surface of immune effector cells such as natural killers and macrophages, leading to the phagocytosis or lysis of a targeted cell. In CDC, the Fc region can generally trigger the complement cascade at the cell surface to kill the targeted cell. Accordingly, modulating effector functions can be achieved by engineering the Fc region to either increase or decrease their binding to the Fc receptors on the surface of the immune effector cells or the complement factors. For example, numerous mutations within a Fc region for modulating ADCC and CDC are well known to a skilled artisan, *e*.*g*., see Armour KL. et al. (1999) Eur J Immmunol 29: 2613-2624; Shields RL. et al. (2001) J Biol Chem. 276: 6591-6604; Idusogie EE. et al. (2001) J Immunol. 166: 2571-2575; Idusogie EE. et al. (2000) J Immunol. 155: 1165-1174; and Steurer W. et al. (1995) J ImmunoL 155: 1165-1674. In one embodiment, the amino acid asparagine (N) at the residue 82 of the SEQ ID NO. 6 can be mutated to aspartic acid (D), *e*.*g*., to remove the glycosylation of Fc and thus, in turn, reduce ADCC and CDC functions.

In various embodiments, the N-terminus or the C-terminus of the linker, e.g., the portion of the Fc region, can be modified. By way of example only, the N-terminus or the C-terminus of the linker can be extended by at least one additional linker described herein, e.g., to provide further flexibility, or to attach additional molecules. In some embodiments, the N-terminus of the linker can be linked directly or indirectly (via an additional linker) with a substrate-binding domain adapted for orienting the carbohydrate recognition domain away from the capture element Exemplary Fc linked MBL (FcMBL and Akt-FcMBL) are described in PCT application no. PCT/US2011/021603, filed January 19, 2011, and U.S. Provisional Application Nos. 61/508,957 filed July 18, 2011, and 61/605,081 filed February 29, 2012, the contents of which are incorporated herein by reference.

In some embodiments, the linker can be embodied as part of the microbe surface-binding domain, or part of the carbohydrate-binding protein (e.g., MBL and/or the neck region thereof).

In some embodiments, the distance between the microbe surface-binding domain and the capture element can range from about 50 angstroms to about 5000 angstroms, from about 100 angstroms to about 2500 angstroms, or from about 200 angstroms to about 1000 angstroms.

The linkers can be of any shape. In some embodiments, the linkers can be linear. In some embodiments, the linkers can be folded. In some embodiments, the linkers can be branched. For branched linkers, each branch of a microbe surface-binding domain can comprise at least one microbe surface-binding domain. In other embodiments, the linker adopts the shape of the physical capture element.

In some embodiments, the linker is a polypeptide comprising the amino acid sequence

EPKSSDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSN
KALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYT
QKSLSLSPGA (SEQ ID NO: 9).

In some embodiments, the linker is a polypeptide comprising the amino acid sequence

EPKSSDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA
PIEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
(SEQ ID NO: 55).

In some embodiments provided herein, the linker can further comprise a detectable label. In some embodiments, the detectable label can be a chromogenic or fluorogenic microbe enzyme capture element so that when a microbe binds to the engineered microbe-binding molecule, the enzyme that the microbe releases can interact with the detectable label to induce a color change. Examples of such microbe enzyme capture element can include, but are not limited to, indoxyl butyrate, indoxyl glucoside, esculin, magneta glucoside, red-β-glucuronide, 2-methoxy-4-(2-nitrovinyl) phenyl β-D-glu-copyranoside, 2-methoxy-4-(2-nitrovinyl) phenyl β-D-cetamindo-2-deoxyglucopyranoside, and any other art-recognized microbe enzyme capture elements. Such embodiments can act as an indicator for the presence of a microbe or pathogen.

As used herein, the term "detectable label" refers to a composition capable of producing a detectable signal indicative of the presence of a target Detectable labels include any composition detectable by spectroscopic, photochemical, biochemical, immunochemical, electrical, optical or chemical means. Suitable labels include fluorescent molecules, radioisotopes, nucleotide chromophores, enzymes, substrates, chemiluminescent moieties, bioluminescent moieties, and the like. As such, a label is any composition detectable by spectroscopic, photochemical, biochemical, immunochemical, electrical, optical or chemical means needed for the methods and devices described herein.

A wide variety of fluorescent reporter dyes are known in the art. Typically, the fluorophore is an aromatic or heteroaromatic compound and can be a pyrene, anthracene, naphthalene, acridine, stilbene, indole, benzindole, oxazole, thiazole, benzothiazole, cyanine, carbocyanine, salicylate, anthranilate, coumarin, fluorescein, rhodamine or other like compound.

Exemplary fluorophores include, but are not limited to, 1,5 IAEDANS; 1,8-ANS ; 4-Methylumbelliferone; 5-carboxy-2,7-dichlorofluorescein; 5-Carboxyfluorescein (5-FAM); 5-Carboxynapthofluorescein (pH 10); 5-Carboxytetramethylrhodamine (5-TAMRA); 5-FAM (5-Carboxyfluorescein); 5-Hydroxy Tryptamine (HAT); 5-ROX (carboxy-X-rhodamine); 5-TAMRA (5-Carboxytetramethylrhodamine); 6-Carboxyrhodamine 6G; 6-CR 6G; 6-JOE; 7-Amino-4-methylcoumarin; 7-Aminoactinomycin D (7-AAD); 7-Hydroxy-4-methylcoumarin; 9-Amino-6-chloro-2-methoxyacridine; ABQ; Acid Fuchsin; ACMA (9-Amino-6-chloro-2-methoxyacridine); Acridine Orange; Acridine Red; Acridine Yellow; Acriflavin; Acriflavin Feulgen SITSA; Aequorin (Photoprotein); Alexa Fluor 350™; Alexa Fluor 430™; Alexa Fluor 488™; Alexa Fluor 532™; Alexa Fluor 546™; Alexa Fluor 568™; Alexa Fluor 594™; Alexa Fluor 633™; Alexa Fluor 647™; Alexa Fluor 660™; Alexa Fluor 680™; Alizarin Complexon; Alizarin Red; Allophycocyanin (APC); AMC, AMCA-S; AMCA (Aminomethylcoumarin); AMCA-X; Aminoactinomycin D; Aminocoumarin; Anilin Blue; Anthrocyl stearate; APC-Cy7; APTS; Astrazon Brilliant Red 4G; Astrazon Orange R; Astrazon Red 6B; Astrazon Yellow 7 GLL; Atabrine; ATTO-TAG™ CBQCA; ATTO-TAG™ FQ; Auramine; Aurophosphine G; Aurophosphine; BAO 9 (Bisaminophenyloxadiazole); BCECF (high pH); BCECF (low pH); Berberine Sulphate; Beta Lactamase; BFP blue shifted GFP (Y66H); BG-647; Bimane; Bisbenzamide; Blancophor FFG; Blancophor SV; BOBO™ -1; BOBO™ -3; Bodipy 492/515; Bodipy 493/503; Bodipy 500/510; Bodipy 505/515; Bodipy 530/550; Bodipy 542/563; Bodipy 558/568; Bodipy 564/570; Bodipy 576/589; Bodipy 581/591; Bodipy 630/650-X; Bodipy 650/665-X; Bodipy 665/676; Bodipy Fl; Bodipy FL ATP; Bodipy Fl-Ceramide; Bodipy R6G SE; Bodipy TMR; Bodipy TMR-X conjugate; Bodipy TMR-X, SE; Bodipy TR; Bodipy TR ATP; Bodipy TR-X SE; BO-PRO™ -1; BO-PRO™ -3; Brilliant Sulphoflavin FF; Calcein; Calcein Blue; Calcium Crimson™; Calcium Green; Calcium Green-1 Ca²⁺ Dye; Calcium Green-2 Ca²⁺; Calcium Green-5N Ca²⁺; Calcium Green-C18 Ca²⁺; Calcium Orange; Calcofluor White; Carboxy-X-rhodamine (5-ROX); Cascade Blue™; Cascade Yellow; Catecholamine; CFDA; CFP - Cyan Fluorescent Protein; Chlorophyll; Chromomycin A; Chromomycin A; CMFDA; Coelenterazine ; Coelenterazine cp; Coelenterazine f; Coelenterazine fcp; Coelenterazine h; Coelenterazine hep; Coelenterazine ip; Coelenterazine O; Coumarin Phalloidin; CPM Methylcoumarin; CTC; Cy2™; Cy3.1 8; Cy3.5™; Cy3™; Cy5.1 8; Cy5.5™; Cy5™; Cy7™; Cyan GFP; cyclic AMP Fluorosensor (FiCRhR); d2; Dabcyl; Dansyl; Dansyl Amine; Dansyl Cadaverine; Dansyl Chloride; Dansyl DHPE; Dansyl fluoride; DAPI; Dapoxyl; Dapoxyl 2; Dapoxyl 3; DCFDA; DCFH (Dichlorodihydrofluorescein Diacetate); DDAO; DHR (Dihydorhodamine 123); Di-4-ANEPPS; Di-8-ANEPPS (non-ratio); DiA (4-Di-16-ASP); DIDS; Dihydorhodamine 123 (DHR); DiO (DiOC18(3)); DiR; DiR (DiIC18(7)); Dopamine; DsRed; DTAF; DY-630-NHS; DY-635-NHS; EBFP; ECFP; EGFP; ELF 97; Eosin; Erythrosin; Erythrosin ITC; Ethidium homodimer-1 (EthD-1); Euchrysin; Europium (III) chloride; Europium; EYFP; Fast Blue; FDA; Feulgen (Pararosaniline); FITC; FL-645; Flazo Orange; Fluo-3; Fluo-4; Fluorescein Diacetate; Fluoro-Emerald; Fluoro-Gold (Hydroxystilbamidine); Fluor-Ruby; FluorX; FM 1-43™; FM 4-46; Fura Red™ (high pH); Fura-2, high calcium; Fura-2, low calcium; Genacryl Brilliant Red B; Genacryl Brilliant Yellow 10GF; Genacryl Pink 3G; Genacryl Yellow 5GF; GFP (S65T); GFP red shifted (rsGFP); GFP wild type, non-UV excitation (wtGFP); GFP wild type, UV excitation (wtGFP); GFPuv; Gloxalic Acid; Granular Blue; Haematoporphyrin; Hoechst 33258; Hoechst 33342; Hoechst 34580; HPTS; Hydroxycoumarin; Hydroxystilbamidine (FluoroGold); Hydroxytryptamine; Indodicarbocyanine (DiD); Indotricarbocyanine (DiR); Intrawhite Cf; JC-1; JO-JO-1; JO-PRO-1; LaserPro; Laurodan; LDS 751; Leucophor PAF; Leucophor SF; Leucophor WS; Lissamine Rhodamine; Lissamine Rhodamine B; LOLO-1; LO-PRO-1; Lucifer Yellow; Mag Green; Magdala Red (Phloxin B); Magnesium Green; Magnesium Orange; Malachite Green; Marina Blue; Maxilon Brilliant Flavin 10 GFF; Maxilon Brilliant Flavin 8 GFF; Merocyanin; Methoxycoumarin; Mitotracker Green FM; Mitotracker Orange; Mitotracker Red; Mitramycin; Monobromobimane; Monobromobimane (mBBr-GSH); Monochlorobimane; MPS (Methyl Green Pyronine Stilbene); NBD; NBD Amine; Nile Red; Nitrobenzoxadidole; Noradrenaline; Nuclear Fast Red; Nuclear Yellow; Nylosan Brilliant Iavin E8G; Oregon Green™; Oregon Green 488-X; Oregon Green™ 488; Oregon Green™ 500; Oregon Green™ 514; Pacific Blue; Pararosaniline (Feulgen); PE-Cy5; PE-Cy7; PerCP; PerCP-Cy5.5; PE-TexasRed (Red 613); Phloxin B (Magdala Red); Phorwite AR; Phorwite BKL; Phorwite Rev; Phorwite RPA; Phosphine 3R; PhotoResist; Phycoerythrin B [PE]; Phycoerythrin R [PE]; PKH26 ; PKH67; PMIA; Pontochrome Blue Black; POPO-1; POPO-3; PO-PRO-1; PO-PRO-3; Primuline; Procion Yellow; Propidium Iodid (PI); PyMPO; Pyrene; Pyronine; Pyronine B; Pyrozal Brilliant Flavin 7GF; QSY 7; Quinacrine Mustard; Resorufin; RH 414; Rhod-2; Rhodamine; Rhodamine 110; Rhodamine 123; Rhodamine 5 GLD; Rhodamine 6G; Rhodamine B 540; Rhodamine B 200 ; Rhodamine B extra; Rhodamine BB; Rhodamine BG; Rhodamine Green; Rhodamine Phallicidine; Rhodamine Phalloidine; Rhodamine Red; Rhodamine WT; Rose Bengal; R-phycoerythrin (PE); red shifted GFP (rsGFP, S65T); S65A; S65C; S65L; S65T; Sapphire GFP; Serotonin; Sevron Brilliant Red 2B; Sevron Brilliant Red 4G; Sevron Brilliant Red B; Sevron Orange; Sevron Yellow L; sgBFP™; sgBFP™ (super glow BFP); sgGFP™; sgGFP™ (super glow GFP); SITS; SITS (Primuline); SITS (Stilbene Isothiosulphonic Acid); SPQ (6-methoxy-N-(3-sulfopropyl)-quinolinium); Stilbene; Sulphorhodamine B can C; Sulphorhodamine G Extra; Tetracycline; Tetramethylrhodamine; Texas Red™; Texas Red-X™ conjugate; Thiadicarbocyanine (DiSC3); Thiazine Red R; Thiazole Orange; Thioflavin 5; Thioflavin S; Thioflavin TCN; Thiolyte; Thiozole Orange; Tinopol CBS (Calcofluor White); TMR; TO-PRO-1; TO-PRO-3; TO-PRO-5; TOTO-1; TOTO-3; TriColor (PE-Cy5); TRITC
(TetramethylRodamineIsoThioCyanate); True Blue; TruRed; Ultralite; Uranine B; Uvitex SFC; wt GFP; WW 781; XL665; X-Rhodamine; XRITC; Xylene Orange; Y66F; Y66H; Y66W; Yellow GFP; YFP; YO-PRO-1; YO-PRO-3; YOYO-1; and YOYO-3. Many suitable forms of these fluorescent compounds are available and can be used.

Other exemplary detectable labels include luminescent and bioluminescent markers (*e.g.,* biotin, luciferase (*e.g.,* bacterial, firefly, click beetle and the like), luciferin, and aequorin), radiolabels (*e*.*g*., 3H, 1251, 35S, 14C, or 32P), enzymes (*e*.*g*., galactosidases, glucorinidases, phosphatases (*e.g*., alkaline phosphatase), peroxidases (*e.g*., horseradish peroxidase), and cholinesterases), and calorimetric labels such as colloidal gold or colored glass or plastic (*e.g*., polystyrene, polypropylene, and latex) beads. Patents teaching the use of such labels include U.S. Pat. Nos. 3,817,837, 3,850,752, 3,939,350, 3,996,345, 4,277,437, 4,275,149, and 4,366,241, each of which is incorporated herein by reference.

Means of detecting such labels are well known to those of skill in the art. Thus, for example, radiolabels can be detected using photographic film or scintillation counters, fluorescent markers can be detected using a photo-detector to detect emitted light. Enzymatic labels are typically detected by providing the enzyme with an enzyme substrate and detecting the reaction product produced by the action of the enzyme on the enzyme substrate, and calorimetric labels can be detected by visualizing the colored label.

In some embodiments, the detectable label is a fluorophore or a quantum dot. Without wishing to be bound by a theory, using a fluorescent reagent can reduce signal-to-noise in the imaging/readout, thus maintaining sensitivity. Accordingly, in some embodiments, prior to detection, the microbes isolated from or remained bound on the microbe-binding substrate can be stained with at least one stain, *e.g*., at least one fluorescent staining reagent comprising a microbe-binding molecule, wherein the microbe-binding molecule comprises a fluorophore or a quantum dot. Examples of fluorescent stains include, but are not limited to, any microbe-binding element (*e.g*., microbe-specific antibodies or any microbe-binding proteins or peptides or oligonucleotides) typically conjugated with a fluorophore or quantum dot, and any fluorescent stains used for detection as described herein.

In some embodiments, a labeling molecule can be configured to include a "smart label", which is undetectable when conjugated to the microbe-binding molecules, but produces a color change when released from the engineered molecules in the presence of a microbe enzyme. Thus, when a microbe binds to the engineered microbe-binding molecules, the microbe releases enzymes that release the detectable label from the engineered molecules. An observation of a color change indicates presence of the microbe in the sample.

In some embodiments, the microbe-binding substrate can be conjugated with a label, such as a detectable label or a biotin label.

In some embodiments, the labeling molecule can comprise a wild-type microbe-binding molecule (e.g. MBL) or a microbe-binding molecule described herein. In some embodiment, the labeling molecule comprises FcMBL. Without wishing to be bound by a theory, labeling molecules based on microbe-binding molecules described herein and MBL (e.g., FcMBL) attach selectively to a broad range of microbes, and so they enable the method described herein to detect the majority of blood-borne microbes with high sensitivity and specificity.

Any method known in the art for detecting the particular label can be used for detection. Exemplary methods include, but are not limited to, spectrometry, fluorometry, microscopy imaging, immunoassay, and the like. While the microbe capture step can specifically capture microbes, it can be beneficial to use a labeling molecule that can enhance this specificity. If imaging, *e.g.,* microscopic imaging, is to be used for detecting the label, the staining can be done either prior to or after the microbes have been laid out for microscopic imaging. Additionally, imaging analysis can be performed via automated image acquisition and analysis.

For optical detection, including fluorescent detection, more than one stain or dye can be used to enhance the detection or identification of the microbe. For example, a first dye or stain can be used that can bind with a genus of microbes, and a second dye or strain can be used that can bind with a specific microbe. Colocalization of the two dyes then provides enhanced detection or identification of the microbe by reducing false positive detection of microbes.

In some embodiments, microscopic imaging can be used to detect signals from label on the labeling agent. Generally, the microbes in the subsample are stained with a staining reagent and one or more images taken from which an artisan can easily count the number of cells present in a field of view.

In particular embodiments, microbe can be detected through use of one or more enzyme assays, *e*.*g*., enzyme-linked assay (ELISA). Numerous enzyme assays can be used to provide for detection. Examples of such enzyme assays include, but are not limited to, beta-galactosidase assays, peroxidase assays, catalase assays, alkaline phosphatase assays, and the like. In some embodiments, enzyme assays can be configured such that an enzyme will catalyze a reaction involving an enzyme substrate that produces a fluorescent product. Enzymes and fluorescent enzyme substrates are known and are commercially available (*e.g.,* Sigma-Aldrich, St. Louis, Mo.). In some embodiments, enzyme assays can be configured as binding assays that provide for detection of microbe. For example, in some embodiments, a labeling molecule can be conjugated with an enzyme for use in the enzyme assay. An enzyme substrate can then be introduced to the one or more immobilized enzymes such that the enzymes are able to catalyze a reaction involving the enzyme substrate to produce a detectable signal.

In some embodiments, an enzyme-linked assay (ELISA) can be used to detect signals from the labeling molecule. In ELISA, the labeling molecule can comprise an enzyme as the detectable label. Each labeling molecule can comprise one or more (*e.g*., 1, 2, 3, 4, 5, 6, 7, 8, 9, 10 or more) enzymes. Additionally, each labeling molecule can comprise one or more (*e.g.,* 1, 2, 3, 4, 5, 6, 7, 8, 9, 10 or more) sites for binding with a microbe. Without wishing to be bound by a theory, presence of multimeric probe molecules can enhance ELISA signal.

For ELISA, any labeling molecule conjugated to an enzyme can be used. Exemplary labeling molecules include those comprising a microbe-binding molecule described herein. Other exemplary labeling molecules include those comprising MBL, FcMBL, AKT-FcMBL, wheat germ agglutinin, lectins, antibodies (*e.g*., gram-negative antibodies or gram-positive antibodies), antigen binding fragments of antibodies, aptamers, ligands (agonists or antagonists) of cell-surface receptors and the like.

In some embodiments, the labeling molecule can comprise MBL or FcMBL labeled with a detectable label.

Similarly, a variety of enzymes can be used, with either colorimetric or fluorogenic substrates. In some embodiments, the reporter-enzyme produces a calorimetric change which can be measured as light absorption at a particular wavelength. Exemplary enzymes include, but are not limited to, beta-galactosidases, peroxidases, catalases, alkaline phosphatases, and the like.

In some embodiments, the enzyme is a horseradish peroxidase (HRP).

In some embodiments, the enzyme is an alkaline peroxidase (AP).

A microbe-binding molecule and the enzyme can be linked to each other by a linker. In some embodiments, the linker between the microbe-binding molecule and the enzyme is an amide bond. In some embodiments, the linker between the microbe-binding molecule and the enzyme is a disulfide (S-S) bond. When the microbe-binding molecule is a peptide, polypeptide or a protein, the enzyme can be linked at the N-terminus, the C-terminus, or at an internal position of the microbe-binding molecule. Similarly, the enzyme can be linked by its N-terminus, C-terminus, or an internal position.

In one embodiment, the ELISA probe molecule can comprise a MBL or a portion there of or a FcMBL molecule linked to a HRP. Conjugation of HRP to any proteins and antibodies are known in the art. In one embodiment, FcMBL-HRP construct is generated by direct coupling HRP to FcMBL using any commercially-available HRP conjugation kit. In some embodiments, the microbes isolated from or remained bound on the microbe-binding substrate can be incubated with the HRP-labeled microbe-binding molecules, *e.g.,* MBL or a portion thereof, or a FcMBL molecule linked to a HRP for a period of time, *e*.*g*., at least about 5 mins, at least about 10 mins, at least about 15 mins, at least about 20 mins, at least about 25 mins, at least about 30 mins. The typical concentrations of HRP-labeled molecules used in the ELISA assay can range from about 1: 500 to about 1:20,000 dilutions. In one embodiment, the concentration of HRP-labeled microbe-binding molecules, *e*.*g*., MBL or a portion thereof, or a FcMBL molecule linked to a HRP molecule, can be about 1:1000 to about 1:10000 dilutions.

In one embodiment, the ELISA probe molecule can comprise a MBL or a portion thereof, or a FcMBL molecule linked to a AP. Conjugation of AP to any proteins and antibodies are known in the art. In one embodiment, FcMBL-AP construct is generated by direct coupling AP to FcMBL using any commercially-available AP conjugation kit. In some embodiments, the microbes isolated from or remained bound on the microbe-binding substrate can be incubated with the AP-labeled microbe-binding molecule, *e*.*g*., MBL or a portion thereof, or a FcMBL molecule linked to a AP for a period of time, *e*.*g*., at least about 5 mins, at least about 10 mins, at least about 15 mins, at least about 20 mins, at least about 25 mins, at least about 30 mins. The typical concentrations of AP-labeled molecules used in the ELISA assay can range from about 1: 1000 to about 1:20,000 dilutions. In one embodiment, the concentration of AP- labeled microbe-binding molecules, *e*.*g*., MBL or a portion thereof, or a FcMBL molecule linked to a AP molecule, can be about 1:5000 to about 1:10000 dilutions.

Following incubation with the ELISA probe molecules, the sample can be washed with a wash buffer one or more (*e.g*., 1, 2, 3, 4, 5 or more) times to remove any unbound probes. An appropriate substrate for the enzyme (*e.g*., HRP or AP) can be added to develop the assay. Chromogenic substrates for the enzymes (*e.g*., HRP or AP) are known to one of skill in the art. A skilled artisan can select appropriate chromogenic substrates for the enzyme, *e.g.,* TMB substrate for the HRP enzyme, or BCIP/NBT for the AP enzyme. In some embodiments, the wash buffer used after incubation with an ELISA probe molecule can contain calcium ions at a concentration of about at least about 0.01 mM, at least about 0.05 mM, at least about 0.1 mM, at least about 0.5 mM, at least about 1 mM, at least about 2.5 mM, at least about 5 mM, at least about 10 mM, at least about 20 mM, at least about 30 mM, at least about 40 mM, at least about 50 mM or more. In alternative embodiments, the wash buffer used after incubation with an ELISA probe molecule can contain no calcium ions. In some embodiments, the wash buffer used after incubation with an ELISA probe molecule can contain a chelating agent A wash buffer can be any art-recognized buffer used for washing between incubations with antibodies and/or labeling molecules. An exemplary wash buffer can include, but is not limited to, TBST.

In some embodiments, without wishing to be bound by theory, it can be desirable to use a wash buffer without a surfactant or a detergent for the last wash before addition of a chromogenic substrate, because a surfactant or detergent may have adverse effect to the enzymatic reaction with a chromogenic substrate.

One advantage of the ELISA-based approach is that the solid substrate does not need to be dispersed or dissociated from the microbe before binding the secondary reagents. This is in contrast to microscopic techniques, in which excess residual solid substrate may obscure the microbe during imaging. Furthermore, the optical readout components for ELISA are likely cheaper than in the microscopy case, and there is no need for focusing or for demanding that the sample be on the same focal plane. A further advantage of the ELISA-based approach is that it can take advantage of commercially available laboratory equipment. In particular, when the solid substrate is magnetic, magnetic separation can be automated using the KINGFISHER® system, the brief culture can be performed using an airlift fermenter, and the colorimetric/fluorescent readout can be attained using a standard plate reader.

Further amplification of the ELISA signal can be obtained by multimerizing the recognition molecule (*e*.*g*., the microbe-binding molecule) or by multimerizing the detection enzyme (HRP, etc.). For instance, phage expression can be used to yield multimerized MBL and provide a scaffold to increase the concentration of HRP (either through direct coupling of HRP to the phage particles or using an HRP-antiM13 conjugated antibody).

In some embodiments, microbe can be detected through use of immunoassay. Numerous types of detection methods may be used in combination with immunoassay based methods.

Without limitations, detection of microbes in a sample can also be carried out using light microscopy with phase contrast imaging based on the characteristic size (5 um diameter), shape (spherical to elliptical) and refractile characteristics of target components such as microbes that are distinct from all normal blood cells. Greater specificity can be obtained using optical imaging with fluorescent or cytochemical stains that are specific for all microbes or specific subclasses (*e.g*. calcofluor (1 µM to 100 µM) for chitin in fungi, fluorescent antibodies directed against fungal surface molecules, gram stains, acid-fast stains, fluorescent MBL, fluorescent Fc-MBL, etc.).

Microbe detection can also be carried out using an epifluorescent microscope to identify the characteristic size (5 um diameter), shape (spherical to elliptical) and staining characteristics of microbes. For example, fungi stain differently from all normal blood cells, strongly binding calcofluor (1 µM to 100 µM) and having a rigid ellipsoid shape not found in any other normal blood cells.

In some embodiments, a microbe can be detected through use of spectroscopy. Numerous types of spectroscopic methods can be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Pat. No. 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, a microbe can be detected through use of fluorescence anisotropy. Fluorescence anisotropy is based on measuring the steady state polarization of sample fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel-Intensity perpendicular)/ (Intensity parallel+2(Intensity perpendicular)) where the I terms indicate intensity measurements parallel and perpendicular to the incident polarization. Fluorescence anisotropy detection of fluorescent molecules has been described. Accordingly, fluorescence anisotropy can be coupled to numerous fluorescent labels as have been described herein and as have been described in the art.

In some embodiments, microbe can be detected through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect captured microbe. For example, in some embodiments, a first labeling molecule can be labeled with a fluorescent donor and second labeling molecule can be labeled with a fluorescent acceptor. Accordingly, such labeled first and second labeling molecules can be used within competition assays to detect the presence and/or concentration of microbe in a sample. Numerous combinations of fluorescent donors and fluorescent acceptors can be used for detection.

In some embodiments, a microbe can be detected through use of polynucleotide analysis. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described, for example, in U.S. Pat. No. 7,118, 910 and No. 6,960,437; herein incorporated by reference). Such methods can be adapted to provide for detection of one or more microbe nucleic acids. In some embodiments, fluorescence quenching, molecular beacons, electron transfer, electrical conductivity, and the like can be used to analyze polynucleotide interaction. Such methods are known and have been described, for example, in Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al, Proc. Natl. Acad. Sci, 100:7605-7610 (2003); Wang et al. Anal. Chem, 75:3941-3945 (2003); and Fan et al, Proc. Natl. Acad. Sci, 100:9134-9137 (2003) and in U.S. Pat. No. 6,958,216; No. 5,093,268; and 6,090,545, the content of all of which is incorporated herein by reference. In some embodiments, the polynucleotide analysis is by polymerase chain reaction (PCR). The fundamentals of PCR are well-known to the skilled artisan, see, *e.g.* McPherson, et al., PCR, A Practical Approach, IRL Press, Oxford, Eng. (1991), hereby incorporated by reference.

In some embodiments, a metabolic assay is used to determine the relative number of microbes in a sample compared to a control. As will be apparent to one of ordinary skill in the art any metabolic indicator that can be associated with cells can be used, such as but not limited to, turbidity, fluorescent dyes, and redox indicators such as, but not limited to, Alamar Blue, MTT, XTT, MTS, and WST. Metabolic indicators can be components inherent to the cells or components added to the environment of the cells. In some embodiments, changes in or the state of the metabolic indicator can result in alteration of ability of the media containing the sample to absorb or reflect particular wavelengths of radiation.

Exemplary metabolic assays include, but are not limited to, ATP Luminescence, reactive oxygen species (ROS) assays, Resazurin assays, Luminol, MTT-metabolic assays, and the like. Further, as one of skill in the art is well aware, kits and methods for carrying out metabolic assays are commercially available. For example, 2-(N-(7-Nitrobenz-2-oxa-1,3-diazol-4-yl)Amino)-2-Deoxyglucose (2-NBDG), ATP Determination Kit, AMPLEX® Red Galactose/Galactose Oxidase Assay Kit, AMPLEX® Red Glucose/Glucose Oxidase Assay Kit, AMPLEX® Red Glutamic AcidGlutamate Oxidase Assay Kit, AMPLEX® Red Hydrogen Peroxide/Peroxidase Assay Kit, AMPLEX® Red Monoamine Oxidase Assay Kit, AMPLEX® Red Neuraminidase (Sialidase) Assay Kit, AMPLEX® Red Phosphatidylcholine-Specific Phospholipase C Assay Kit, AMPLEX® Red Sphingomyelinase Assay kit, AMPLEX® Red Uric Acid/Uricase Assay Kit, AMPLEX® Red Xanthino/Xanthine Oxidase Assay Kit, THIOLTRACKER™ Violet (Glutathione Detection Reagent), THIOLTRACKER™ Violet (Glutathione Detection Reagent), and VYBRANT® Cell Metabolic Assay Kit from Invitrogen; Adenosine 5'-triphospahte (ATP) Luminescence Assay Kit (ENLITEN® from Promega; ATPLITETM from PerkinElmer Life Sciences; ATP Bioluminescence Assay kit HS II from Boehringer Mannheim, Germany; Adenosine 5'-triphosphate (ATP) Luminescence Assay Kit from EMD Millipore; Reactive Oxygen Species (ROS) Assays from Cell BioLabs, Inc.; Cellular Reactive Oxygen Species Detection Assay Kit from ABCAM®; hROS Detection Kit from Cell Technology, Inc.; and ABTS Antioxidant Assay Kit, ORAC Antioxidant Assay Kit, OxiSelect HORAC Activity Assay Kit, OxiSelect *In vitro* ROS/RNS Assay Kit (Green Fluorescence), OxiSelect Intracellular ROS Assay Kit (Green Fluorescence), OxiSelect ORAC Activity Assay Kit, OxiSelect Total Antioxidant Capacity (TAC) Assay Kit, and Total Antioxidant Capacity Assay Kit from BioCat.

In some embodiments, microbes isolated from or remained bound on microbe-binding substrate can be labeled with nucleic acid barcodes for subsequent detection and/or multiplexing detection. Nucleic acid barcoding methods for detection of one or more analytes in a sample are well known in the art.

In other embodiments, the captured microbe can be analyzed and/or detected in the capture chamber or capture and visualization chamber of a rapid microbe diagnostic device described in the Int. Pat. App. No. Int. Pat App. No. WO 2011/091037, filed January 19, 2011, content of which is incorporated herein by reference. Alternatively, the captured microbe can be recovered (*i.e.,* removed) and analyzed and/or detected.

In some embodiments, the captured microbe is recovered and analyzed and/or detected using a particle on membrane assay as described in U.S. Patent No. 7,781,226, content of which is incorporated herein by reference. A particle on membrane assay as described in U.S. Patent No. 7,781,226 can be operably linked with a rapid microbe diagnostic device of the Int. Pat. App. No. Int. Pat. App. No. WO 2011/091037 to reduce the number of sample handling steps, automate the process and/or integrate the capture, separation and analysis/detection steps into a microfluidic device.

In some embodiments, microbe capture, separation and analysis can be done using a hybrid microfluidic SPR and molecular imagining device as described in U.S. Pat. App. Pub. No. US 2011/0039280.

In some embodiments, the processes or assays described herein can detect the presence or absence of a microbe and/or identify a microbe in a test sample in less than 24 hours, less than 12 hours, less than 10 hours, less than 8 hours, less than 6 hours, less than 4 hours, less than 3 hours, less than 2 hours, less than 1 hour, or lower. In some embodiments, the processes or assays described herein can detect the presence or absence of a microbe and/or identify a microbe in a test sample in less than 6 hours, less than 4 hours, less than 3 hours, less than 2 hours, less than 1 hour, or lower.

In certain embodiments, the binding groups can be derived from arginylglycylaspartic acid, which is a tripeptide composed of L-arginine, glycine, and L-aspartic acid (RGD peptide).

In certain embodiments, in addition to the FcMBL exemplified herein, the binding groups that can selectively bind to desired moieties include groups that can be used to conjugate proteins e.g. antibodies & peptides, nucleic acids e.g. DNA, RNA, polymers e.g. PEG, PNA to the surface, including: secreted proteins, signaling molecules, embedded proteins, nucleic acid/protein complexes, nucleic acid precipitants, chromosomes, nuclei, mitochondria, chloroplasts, flagella, biominerals, protein complexes, and minicells.

The surface of a solid substrate can be functionalized to include coupling molecules described herein. As used herein, the term "coupling molecule" refers to any molecule or any functional group that is capable of selectively binding with an engineered microbe surface-binding domain described herein. Representative examples of coupling molecules include, but are not limited to, antibodies, antigens, lectins, proteins, peptides, nucleic acids (DNA, RNA, PNA and nucleic acids that are mixtures thereof or that include nucleotide derivatives or analogs); receptor molecules, such as the insulin receptor; ligands for receptors (e.g., insulin for the insulin receptor); and biological, chemical or other molecules that have affinity for another molecule, such as biotin and avidin. The coupling molecules need not comprise an entire naturally occurring molecule but may consist of only a portion, fragment or subunit of a naturally or non-naturally occurring molecule, as for example the Fab fragment of an antibody. The coupling molecule can further comprise a detectable label. The coupling molecule can also encompass various functional groups that can couple the solid substrate to the engineered microbe surface-binding domains. Examples of such functional groups include, but are not limited to, an amino group, a carboxylic acid group, an epoxy group, and a tosyl group.

In some embodiments, the microbe-binding molecule can be conjugated to a solid substrate surface through a covalent or non-covalent interaction. The microbe-binding molecule and/or coupling molecule can be conjugated to the surface of a solid substrate covalently or non-covalently using any of the methods known to those of skill in the art For example, covalent immobilization can be accomplished through, for example, silane coupling. See, *e*.*g*., Weetall, 15 Adv. Mol. Cell Bio. 161 (2008); Weetall, 44 Meths. Enzymol. 134 (1976). The covalent interaction between the microbe-binding molecule and/or coupling molecule and the surface can also be mediated by other art-recognized chemical reactions, such as NHS reaction or a conjugation agent. The non-covalent interaction between the microbe-binding molecule and/or coupling molecule and the surface can be formed based on ionic interactions, van der Waals interactions, dipole-dipole interactions, hydrogen bonds, electrostatic interactions, and/or shape recognition interactions.

Without limitations, conjugation can include either a stable or a labile (*e.g*. cleavable) bond or conjugation agent. Exemplary conjugations include, but are not limited to, covalent bond, amide bond, additions to carbon-carbon multiple bonds, azide alkyne Huisgen cycloaddition, Diels-Alder reaction, disulfide linkage, ester bond, Michael additions, silane bond, urethane, nucleophilic ring opening reactions: epoxides, non-aldol carbonyl chemistry, cycloaddition reactions: 1,3-dipolar cycloaddition, temperature sensitive, radiation (IR, near-IR, UV) sensitive bond or conjugation agent, pH-sensitive bond or conjugation agent, non-covalent bonds (*e.g*., ionic charge complex formation, hydrogen bonding, pi-pi interactions, cyclodextrin/adamantly host guest interaction) and the like. In some embodiments, conjugation includes a stable bond or conjugation agent.

As used herein, the term "conjugation agent" means an organic moiety that connects two parts of a compound. Linkers typically comprise a direct bond or an atom such as oxygen or sulfur, a unit such as NR¹, C(O), C(O)NH, SO, SO₂, SO₂NH or a chain of atoms, such as substituted or unsubstituted alkyl, substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocyclylalkyl, heterocyclylalkenyl, heterocyclylalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroaiylalkyl, alkylheteroarylalkenyl, alkylheteroarylalkynyl, alkenylheteroarylalkyl, alkenylheteroarylalkenyl, alkenylheteroarylalkynyl, alkynylheteroarylalkyl, alkynylheteroarylalkenyl, alkynylheteroarylalkynyl, alkylheterocyclylalkyl, alkylheterocyclylalkenyl, alkylhererocyclylalkynyl, alkenylheterocyclylalkyl, alkenylheterocyclylalkenyl, alkenylheterocyclylalkynyl, alkynylheterocyclylalkyl, alkynylheterocyclylalkenyl, alkynylheterocyclylalkynyl, alkylaryl, alkenylaryl, alkynylaryl, alkylheteroaryl, alkenylheteroaryl, alkynylhereroaryl, where one or more methylenes can be interrupted or terminated by O, S, S(O), SO₂, NH, C(O)N(R¹)₂, C(O), cleavable linking group, substituted or unsubstituted aryl, substituted or unsubstituted heteroaryl, substituted or unsubstituted heterocyclic; where R¹ is hydrogen, acyl, aliphatic or substituted aliphatic.

Without limitations, any conjugation chemistry known in the art for conjugating two molecules or different parts of a composition together can be used for linking at least one microbe-binding molecule to a solid substrate. Exemplary coupling molecules and/or functional groups for conjugating at least one microbe-binding molecule to a solid substrate include, but are not limited to, a polyethylene glycol (PEG, NH₂-PEGx-COOH which can have a PEG spacer arm of various lengths X, where 1 < X < 100, *e*.*g*., PEG-2K, PEG-5K, PEG-10K, PEG-12K, PEG-15K, PEG-20K, PEG-40K, and the like), maleimide conjugation agent, PASylation, HESylation, Bis(sulfosuccinimidyl) suberate conjugation agent, DNA conjugation agent, peptide conjugation agent, silane conjugation agent, polysaccharide conjugation agent, hydrolyzable conjugation agent, and any combinations thereof.

In alternative embodiments, the microbe-binding molecule can be conjugated onto the surface of the solid substrate by a coupling molecule pair. The terms "coupling molecule pair" and "coupling pair" as used interchangeably herein refer to the first and second molecules that specifically bind to each other. One member of the binding pair is conjugated with the solid substrate while the second member is conjugated with the substrate-binding domain of a microbe-binding domain. As used herein, the phrase "first and second molecules that specifically bind to each other" refers to binding of the first member of the coupling pair to the second member of the coupling pair with greater affinity and specificity than to other molecules.

Exemplary coupling molecule pairs include, without limitations, any haptenic or antigenic compound in combination with a corresponding antibody or binding portion or fragment thereof (*e.g*., digoxigenin and anti-digoxigenin; mouse immunoglobulin and goat antimouse immunoglobulin) and nonimmunological binding pairs (*e.g*., biotin-avidin, biotin-streptavidin), hormone (*e.g*., thyroxine and cortisol-hormone binding protein), receptor-receptor agonist, receptor-receptor antagonist (*e.g.*, acetylcholine receptor-acetylcholine or an analog thereof), IgG-protein A, lectin-carbohydrate, enzyme-enzyme cofactor, enzyme-enzyme inhibitor, and complementary oligonucleotide pairs capable of forming nucleic acid duplexes). The coupling molecule pair can also include a first molecule that is negatively charged and a second molecule that is positively charged.

One example of using coupling pair conjugation is the biotin-avidin or biotin-streptavidin conjugation. In this approach, one of the members of the coupling pair (*e.g*., a portion of the microbe-binding molecule such as substrate-binding domain, or a solid substrate) is biotinylated and the other (*e.g*., a solid substrate or the microbe-binding molecule) is conjugated with avidin or streptavidin. Many commercial kits are also available for biotinylating molecules, such as proteins. For example, an aminooxy-biotin (AOB) can be used to covalently attach biotin to a molecule with an aldehyde or ketone group. In one embodiment, AOB is attached to the substrate-binding domain (*e.g*., comprising AKT oligopeptide) of the microbe-binding molecule.

One non-limiting example of using conjugation with a coupling molecule pair is the biotin-sandwich method. See, *e*.*g*., Davis et al., 103 PNAS 8155 (2006). The two molecules to be conjugated together are biotinylated and then conjugated together using tetravalent streptavidin. In addition, a peptide can be coupled to the 15-amino acid sequence of an acceptor peptide for biotinylation (referred to as AP; Chen et al., 2 Nat. Methods 99 (2005)). The acceptor peptide sequence allows site-specific biotinylation by the *E. coli* enzyme biotin ligase (BirA; *Id*.). An engineered microbe surface-binding domain can be similarly biotinylated for conjugation with a solid capture element. Many commercial kits are also available for biotinylating proteins. Another example for conjugation to a solid surface would be to use PLP -mediated bioconjugation. See, *e*.*g*., Witus et al., 132 JACS 16812 (2010).

Still another example of using coupling pair conjugation is double-stranded nucleic acid conjugation. In this approach, one of the members of the coupling pair (*e.g.*, a portion of the microbe-binding molecule such as substrate-binding domain, or a solid substrate) can be conjugated with a first strand of the double-stranded nucleic acid and the other (*e.g*., a solid substrate, or a microbe-binding molecule) is conjugated with the second strand of the double-stranded nucleic acid. Nucleic acids can include, without limitation, defined sequence segments and sequences comprising nucleotides, ribonucleotides, deoxyribonucleotides, nucleotide analogs, modified nucleotides and nucleotides comprising backbone modifications, branchpoints and nonnucleotide residues, groups or bridges.

Activation agents can be used to activate the components to be conjugated together (*e.g*., surface of a solid substrate). Without limitations, any process and/or reagent known in the art for conjugation activation can be used. Exemplary surface activation method or reagents include, but are not limited to, 1-Ethyl-3-[3-dimethylaminopropyl]carbodiimide hydrochloride (EDC or EDAC), hydroxybenzotriazole (HOBT), N-Hydroxysuccinimide (NHS), 2-(1H-7-Azabenzotriazol-1-yl)-1,1,3,3-tetramethyl uronium hexafluorophosphate methanaminium (HATU), silanization, surface activation through plasma treatment, and the like. In one embodiment, EDC is used to conjugate a microbe-binding molecule (e.g., FcMBL) to a solid substrate surface.

Again, without limitations, any art known reactive group can be used for coupling. For example, various surface reactive groups can be used for surface coupling including, but not limited to, alkyl halide, aldehyde, amino, bromo or iodoacetyl, carboxyl, hydroxyl, epoxy, ester, silane, thiol, and the like.

### Patterning

Desired patterns of the anchoring molecules and binding molecules onto the solid substrate can be carried out in a number of different ways. For example, desired anchoring molecules can be deposited onto the substrate using any number of techniques, such as vapor deposition, solvent deposition, contact printing, inkjet printing, spray printing, gravure printing, silkscreen printing, and the like. If desired, additional steps can be carried to form a desired pattern, through techniques such as photolithography, etching, selective heating, and the like.

In certain embodiments, anchoring molecules and/or binding molecules can be deposited onto surface using a soft lithography technique. For example, desired stamp patterns can be printed on a mask followed by fabrication of a mold using standard photolithographic techniques. Then, stamps can be produced using the fabricated mold. The produced stamps can then be used to print a pattern of desired anchoring molecules onto a substrate.

In certain embodiments, anchoring molecules can be deposited on the remaining regions after the deposition of binding molecules using any suitable techniques. The anchoring molecule may have functional groups that have an affinity with the lubricating liquid. Particularly, when the microcontact printed binding molecules are adhered to the substrate without a strong chemical bond, use of gentle deposition processes, such as vapor deposition, should be carried out In such instances, carrying out a more vigorous form of deposition, such as solvent deposition, of the anchoring molecule may wash away the microcontact printed binding molecules.

In certain embodiments, the anchoring molecules that have been printed onto the substrate can have functional groups that bind with another functional group that is present on other materials, such as colloidal particles. For example, a pattern of streptavidin anchoring molecules can be contact printed onto a substrate and colloidal particles that have been functionalized with biotin can be introduced to the substrate to bind the colloidal particles onto the substrate. While a streptavidin-biotin reaction has been provided as an example, other types of reactions, such as amine-APTES reactions and the like, can be utilized. Thereafter, a second type of anchoring molecules having affinity with the lubricating liquid may be deposited onto the remaining regions of the substrate.

In certain embodiments, the functional groups present on the colloidal particles that have been deposited onto the substrate can be utilized to provide any other desired functionality to the colloidal particle. For example, FcMBL that have been functionalized with streptavidin can be utilized to provide colloidal particles having FcMBL functional groups.

### Lubricating Layer

The lubricating liquid used to form the lubricating layer is applied to the anchoring layer. Thus, the lubricating layer, which flows readily over the substrate, should stably, but non-covalently bind the functional groups of the anchoring layer to form a continuous, repellant layer. The lubricating layer can be selected based on its ability to repel immiscible materials. In one or more embodiments, the lubricating layer is inert with respect to the solid substrate and material to be repelled

The lubricating layer can be selected from a variety of fluids. These fluids can be selected, e.g., based on their biocompatibility, level of toxicity, anti-coagulation properties, and chemical stability under physiologic conditions. For example, compounds that are approved for use in biomedical applications can be used in accordance with the present disclosure. In some aspects, the lubricating layer is perfluorinated liquids, non-limiting examples of which include perfluorinated hydrocarbon oils such as FC-43, FC-70, perfluoropropane, perfluorobutane, perfluoropentane, perfluoroperhydrophenanthrene perfluorotripentylamine, perfluorotributylamine, perfluorotripropylamine, perfluorotriethylamine, perfluorotrimethylamine, perfluorodecalin, perfluorooctane, perfluorohexane, perfluoroheptane, perfluorononane, perfluorodecane, perfluorododecane, perfluorooctyl bromide, perfluoro(2-butyl-tetrahydrofurane), perfluoro-2-methylpentane, perfluoromethylcyclohexane, perfluoro-1,3-dimethylcyclohexane, perfluoro-2-methyl-3-ethylpentane, perfluoro-2,4-dimethyl-3-ethylpentane, perfluoromethyldecalin, perfluoroperhydrofluorene, perfluorinated polyether hydrocarbons such as Krytox 100, 103, and combinations thereof. In some aspects, the lubricating layer is fluorinated oils, non-limiting examples of which include fluorinated hydrocarbon oils such as methoxy-nonafluorobutane, ethyl nonafluoroisobutyl ether, ethyl nonafluorobutyl ether, 3-ethoxy-1,1,1,2,3,4,4,5,5,6,6,6-dodecafluoro-2-trifluoromethyl-hexane, polyvinylidene fluoride, polychlorotrifluoroethylene, and partially fluorinated olefins, 2,3,3,3-tetrafluoropropene, 1,3,3,3-tetrafluoropropene, 2-chloro-2-(difluoromethoxy)-1,1,1-trifluoro-ethane, 1,1,1,3,3,3-hexafluoro-2-(fluoromethoxy)propane, 1-Chloro-3,3,3-trifluoropropene and combinations thereof. In other aspects, the lubricating layer is hydrocarbon oil, non-limiting examples of which include oils such as alkanes (e.g., butane, pentane, hexane, cyclohexane, heptane, octane, nonane, decane, dodecane, hexadecane, octadecane), triacylglycerides, mineral oil, alkenes, cholesterol, aromatic hydrocarbons (e.g., benzene, phenol, naphthalene, naphthol,) and combinations thereof. In other aspects, the lubricating layer is a hydrophilic liquid, non-limiting examples of which include water, aqueous solutions (e.g., acids, bases, salts, polymers, buffers), ethanol, methanol, glycerol, ionic liquids (e.g., ethylammonium nitrate, ethylmethylimidazolium hexafluorophosphate, 1-butyl-3-methylimidazolium hexafluorophosphate), and combinations thereof.

In some aspects, the lubricating layer has a low freezing temperature, such as less than -5 °C, -25 °C, or -50 °C. A lubricating layer with a low freezing temperature allows the layer to remain liquid in low temperatures to maintain the ability of the combination of the lubricating layer and functionalized surface to repel a variety of liquids or solidified fluids, such as ice and the like. In other aspects, the lubricating layer has a high boiling temperature, such as more than 50 °C, 100 °C, 200°C, or 300 °C. A lubricating layer with a high boiling point allows the layer to remain liquid in high temperatures to maintain the ability of the combination of lubricating layer and functionalized surface to repel a variety of liquids or liquefied solids, such as butter and the like.

In some aspects, the lubricating layer has a low evaporation rate or a low vapor pressure. For example, the vapor pressure of the lubricating liquid can be less than 10 mmHg at 25 °C, less than 5 mmHg at 25 °C, less than 2 mmHg at 25 °C, less than 1 mmHg at 25 °C, less than 0.5 mmHg at 25 °C, or less than 0.1 mmHg at 25 °C. The lubricating layer can be applied in a thickness sufficient to cover the anchoring layer. In some embodiments, the lubricating layer is applied at a thickness sufficient to form a monomolecular layer on the substrate. In other embodiments, the lubricating layer is applied at a thickness of 10 nm to 10 µm on the substrate. In other embodiments, the lubricating layer is applied at a thickness of 10 µm to 10 mm on the substrate. The lubricating layer applied in a typical thickness, assumed to be at least a monomolecular layer, can remain liquid repellant for a long period without requiring replenishing. By way of example, the surface can remain liquid repellant for a period longer than 1 hour, or longer than 6 hours, or longer than 24 hours, longer than a week, or longer than a year or more.

The lubricating liquid can be sprayed, cast, or drawn onto the substrate either once or repeatedly. In certain embodiments, the lubricating layer can be applied to the surface by spinning coating, pipetting drops of lubricating liquid onto the surface, or dipping the surface into a reservoir or channel containing the lubricating liquid, through microscale holes in the wall of the underlying substrate, or by presaturating the surface with lubricating liquid to form a lubricating layer. The lubricating liquid can also be applied by absorption, wicking, thin layer deposition, or by intermittent passing of volumes of lubricating liquid over the surface (e.g., small plugs or bubbles flowing in a catheter). In some embodiments, any excess lubricating liquid can be removed by spinning the coated article or by drawing a squeegee across the surface.

In some embodiments, the lifetime of the liquid repellant surface can be extended by reapplying the lubricating layer at a certain interval. For example, a pump can be used to periodically send plugs of PFC oil through PDMS tubing. In some aspects, the lubricating layer can be replenished every 1, 5, 10, 15, 20, 30, 40, 50, or 60 seconds. In other aspects, the lubricating layer can be replenished every 5, 10, 15, 20, 30, 40, 50, or 60 minutes. In still other aspects, the lubricating layer can be replenished every 2, 4, 6, 8, 10, 12, 24, 48, 60, or 72 hours or more. In other embodiments, the surface can be replenished with lubricating liquid from a reservoir **200** housed below the substrate **110** as shown in **FIGS.** 1A, **1B****,** **1E****,** **1F****,** **1G****,** and **1I****.** The lubricating liquid is drawn through micropassages **210** to replenish lubricating liquid lost to the environment

In one embodiment, perfluorocarbon ("PFC") oil is used as the lubricating liquid. The PFC oil is retained on the surface by a "Teflon-like" layer on the surface, *e*.*g*., a fluorous surface, which serves as the anchoring layer. The treated surface containing fluoro and/or amino groups has an affinity for other fluorocarbons, and thus when PFC oil is applied to the treated surface, the surface is wetted by and retains a thin layer of PFC oil that resists adhesion of liquids and repels materials. *See*, e*.g*. Wong, T. S. et.al., Nature 447, 443-447 (2011). ***Uses***

In certain embodiments, the surfaces having simultaneous repellent characteristics while selectively binding desired moieties can have applications in assays where the use of the SLIPS surface may significantly reduce the background binding of undesired moieties (e.g., non-specific sticking of DNA, protein, antibodies, etc.) and therefore improve the signal to noise ratio and improve sensitivity and specificity of desired moieties (e.g., specific DNA, proteins, antibodies, etc.). Such assays may be useful in medicine, where measure analytes in complex, messy fluids can be accurately and quickly determined.

In one or more embodiments, any arbitrary liquid (e.g., a biological fluid), and solid particulates contained therein, may be strongly repelled from the surfaces modified in accordance with the present disclosure while targeted moieties are bound to the surface.

In one embodiment, surfaces modified according to the present disclosure can repel a fluid without causing surface adhesion, surface-mediated clot formation, coagulation or aggregation while selectively binding desired targeted moieties. Non-limiting examples of biological fluids include water, whole blood, plasma, serum, sweat, feces, urine, saliva, tears, vaginal fluid, prostatic fluid, gingival fluid, amniotic fluid, intraocular fluid, cerebrospinal fluid, seminal fluid, sputum, ascites fluid, pus, nasopharengal fluid, wound exudate fluid, aqueous humour, vitreous humour, bile, cerumen, endolymph, perilymph, gastric juice, mucus, peritoneal fluid, pleural fluid, sebum, vomit, synthetic fluid (e.g., synthetic blood, hormones, nutrients), and combinations thereof.

In another embodiment, surfaces modified according to the present disclosure can repel various types of bacteria while selectively binding desired targeted moieties. In one embodiment, the type of bacteria repelled and/or selective bound by these surfaces is gram positive bacteria. In another embodiment, the type of bacteria repelled and/or selective bound by the disclosed modified surfaces is a gram negative bacterium. Non-limiting examples of bacteria repelled and/or selective bound by surfaces modified in accordance with the present disclosure include members of the genus selected from the group consisting of *Actinobacillus (e.g., Actinobacillus actinomycetemcomitans*), *Acinetobacter* (*e.g., Acinetobacter baumannii*), *Aeromonas, Bordetella* (*e*.*g*., *Bordetella pertussis, Bordetella bronchiseptica,* and *Bordetella parapertussis*), *Brevibacillus, Brucella, Bacteroides (e.g., Bacteroides fragilis), Burkholderia* (*e.g., Burkholderia cepacia* and *Burkholderia pseudomallei*), *Borelia* (*e.g., Borelia burgdorfen*), *Bacillus (e.g., Bacillus anthracis* and *Bacillus subtilis*), *Campylobacter* (*e.g., Campylobacter jejuni*), *Capnocytophaga, Cardiobacterium* (*e.g., Cardiobacterium hominis*), *Citrobacter, Clostridium* (*e.g., Clostridium tetani* or *Clostridium difficile*), *Chlamydia* (*e.g., Chlamydia trachomatis, Chlamydia pneumoniae,* and *Chlamydia psiffaci*), *Eikenella* (*e*.*g*., *Eikenella corrodens*), *Enterobacter, Escherichia (e.g., Escherichia coli*), *Francisella* (*e.g., Francisella tularensis), Fusobacterium, Flavobacterium, Haemophilus (e.g., Haemophilus ducreyi* or *Haemophilus influenzae*), *Helicobacter* (*e.g., Helicobacter pylori*), *Kingella* (*e.g., Kingella kingae), Klebsiella (e.g., Klebsiella pneumoniae), Legionella (e.g., Legionella pneumophila), Listeria (e.g., Listeria monocytogenes), Leptospirae, Moraxella (e.g., Moraxella catarrhalis*), *Morganella, Mycoplasma* (*e.g., Mycoplasma hominis* and *Mycoplasma pneumoniae*), *Mycobacterium* (*e.g., Mycobacterium tuberculosis* or *Mycobacterium leprae*), *Neisseria* (*e*.*g*., *Neisseria gonorrhoeae* or *Neisseria meningitidis*), *Pasteurella* (*e.g., Pasteurella multocida*), *Proteus* (*e.g., Proteus vulgaris* and *Proteus mirablis*), *Prevotella, Plesiomonas (e.g., Plesiomonas shigelloides), Pseudomonas (e.g., Pseudomonas aeruginosa), Providencia, Rickettsia* (*e.g., Rickettsia rickettsii* and *Rickettsia typhi*), *Stenotrophomonas* (*e.g., Stenotrophomonas maltophila*), *Staphylococcus* (*e.g., Staphylococcus aureus* and *Staphylococcus epidermidis*), *Streptococcus* (*e.g., Streptococcus viridans, Streptococcus pyogenes* (group A), *Streptococcus agalactiae* (group B), *Streptococcus bovis,* and *Streptococcus pneumoniae), Streptomyces (e.g., Streptomyces hygroscopicus), Salmonella* (*e.g., Salmonella enteriditis, Salmonella typhi,* and *Salmonella typhimurium*), *Serratia* (*e.g., Serratia marcescens), Shigella, Spirillum (e.g., Spirillum minus), Treponema (e.g., Treponema pallidum*), *Veillonella, Vibrio (e.g., Vibrio cholerae, Vibrio parahaemolyticus,* and *Vibrio vulnificus*), *Yersinia* (*e*.*g*., *Yersinia enterocolitica, Yersinia pestis,* and *Yersinia pseudotuberculosis*), *Xanthomonas* (*e.g., Xanthomonas maltophilia*) and combinations thereof.

Particularly, non-limiting examples of bacteria repelled and/or selective bound by surfaces modified in accordance with the present disclosure include *Actinobacillus, Acinetobacter* (*e.g., Acinetobacter baumannii*)*,* Aeromonas, *Bordetella, Brevibacillus, Brucella, Bacteroides, Burkholderia, Borelia, Bacillus, Campylobacter, Capnocytophaga, Cardiobacterium, Citrobacter, Clostridium, Chlamydia, Eikenella, Enterobacter,* Enterococcus, *Escherichia, Francisella, Fusobacterium, Flavobacterium, Haemophilus, Helicobacter, Kingella, Klebsiella,* Lacobacillus, *Legionella, Listeria, Leptospirae, Moraxella, Morganella, Mycoplasma, Mycobacterium, Neisseria,* Nocardia, *Pasteurella, Proteus, Prevotella, Plesiomonas, Pseudomonas, Providencia, Rickettsia,* Salmonella, Serratia, Shigella, *Stenotrophomonas, Staphylococcus, Streptococcus* (group A), *Streptococcus agalactiae* (group B), *Streptococcus bovis, Streptococcus pneumoniae, Streptomyces, Salmonella, Serratia, Shigella, Spirillum, Treponema, Veillonella, Vibrio, Yersinia, Xanthomonas,* and combinations thereof

Surfaces modified according to the present disclosure can repel and/or selectively bind various types of fungi. Non-limiting examples of fungi repelled and/or selective bound by modified surfaces include members of the genus *Aspergillus* (*e.g., Aspergillus flavus, Aspergillus fumigatus, Aspergillus glaucus, Aspergillus nidulans, Aspergillus niger,* and *Aspergillus terreus), Blastomyces dermatitidis, Candida (e.g., Candida albicans, Candida glabrata, Candida tropicalis, Candida parapsilosis, Candida krusei,* and *Candida guillermondii*)*, Coccidioides immitis, Cryptococcus (e.g., Cryptococcus neoformans, Cryptococcus albidus,* and *Cryptococcus laurentii*), *Fusarium, Histoplasma capsulatum* var. *capsulatum, Histoplasma capsulatum* var. *duboisii, Mucor, Paracoccidioides brasiliensis, Pneumocystis, Saccharomyces, Sporothrix schenckii, Absidia corymbifera; Rhizomucor pusillus, Rhizopus arrhizous,* and combinations thereof.

Surfaces modified according to the present disclosure can also repel and/or selective bind various types of viruses and virus-like particles. In one or more embodiments, the virus repelled and/or selective bound by these surfaces is selected from the group consisting of dsDNA viruses, ssDNA viruses, dsRNA viruses, (+)ssRNA viruses, (-)ssRNA viruses, ssRNA-RT viruses, dsDNA-RT viruses, and combinations thereof. Non-limiting examples of viruses repelled and/or selective bound by surfaces modified in accordance with the present disclosure include cytomegalovirus (CMV), dengue, Epstein-Barr, Hantavirus, human T-cell lymphotropic virus (HTLV I/II), Parvovirus, hepatitides (e.g., hepatitis A, hepatitis B, and hepatitis C), human papillomavirus (HPV), human immunodeficiency virus (HIV), acquired immunodeficiency syndrome (AIDS), respiratory syncytial virus (RSV), Varicella zoster, West Nile, herpes, polio, smallpox, yellow fever, rhinovirus, coronavirus, Orthomyxoviridae (influenza viruses) (e.g., Influenzavirus A, Influenzavirus B, Influenzavirus C, Isavirus and Thogotovirus), and combinations thereof.

In still another embodiment, surfaces modified according to the present disclosure are capable of repelling and/or selectively binding particles in suspension or solution without causing surface adhesion, surface-mediated clot formation, coagulation, fouling, or aggregation. The omniphobic nature of the disclosed modified surfaces allows them to protect materials from a wide range of contaminants. Non-limiting examples of a particles in suspension or solution include cells (e.g., normal cells, diseased cells, parasitized cells, cancer cells, foreign cells, stem cells, and infected cells), microorganisms (e.g., viruses, virus-like particles, bacteria, bacteriophages), proteins and cellular components (e.g., cell organelles, cell fragments, cell membranes, cell membrane fragments, viruses, virus-like particles, bacteriophage, cytosolic proteins, secreted proteins, signaling molecules, embedded proteins, nucleic acid/protein complexes, nucleic acid precipitants, chromosomes, nuclei, mitochondria, chloroplasts, flagella, biominerals, protein complexes, and minicells).

In still other embodiments, other applications such as molecular origami, where specific patterns and morphologies of DNA, proteins, antibodies can be formed on surfaces and/or in three-dimensions are possible.

### Devices

Described herein are devices for capturing a target moiety, such as a soluble or suspended target moiety in a liquid. Non-limiting examples include a microbe and/or microbial matter present in a bodily fluid, e.g., blood; a fluid obtained from an environmental source, e.g., a pond, a river or a reservoir, or a test sample (e.g., a food sample; DNA, immunoglobulins or cytokines in bodily fluid, e.g. fetal DNA in maternal blood, IgE, IL-6; a forensic sample, e.g. semen or semen components (e.g., DNA) in a rape kit vaginal/rectal swab; a drug test, e.g. anabolic steroids in urine; a clinical test, e.g., creatinine in urine; a point of care test, e.g., human chorionic gonadotropin in urine and/or blood to detect pregnancy). The devices can bind or capture at least one target moiety, such as an intact microbe, and/or "microbial matter." In certain embodiments, the devices can bind or capture at least one target moiety while other non-targeted moieties are repelled away from the surface.

Described herein are diagnostic devices for capturing a target moiety, such as a microbe and/or microbial matter present in a bodily fluid, e.g., blood, a fluid obtained from an environmental source, e.g., a pond, a river or a reservoir, or a test sample. The diagnostic devices can bind or capture at least one target moiety, *e*.*g*., an intact microbe, and/or "microbial matter" while other non-targeted moieties are repelled away from the surface. The diagnostic devices described herein provide a signal-to-noise ratio that exceeds 3, 5, 10, 100, 1000, 10,000, 100,000, or 1,000,000.

In one aspect, provided herein is a device for capturing a microbe, a microbial matter and/or target molecule comprising (i) a chamber with an inlet and an outlet, (ii) at least one capture element disposed in the chamber between the inlet and outlet, wherein the capture element has on its surface at least one microbe-binding molecule described herein. By way of example only, **FIG. 4** shows one embodiment of a capture element **400** (e.g., a spiral mixer) coated with lubricating layer **140** and binding molecules **120.** As described in detail below, binding molecule **120** can be any molecule that binds to a microbe. However, in some embodiments, the binding molecules **120** can comprise (a) at least one microbe surface-binding domain; and (b) at least a portion of a Fc region of an immunoglobulin. In one embodiment, the microbe surface-binding domain can be derived from a mannose-binding lectin (MBL) or a fragment thereof.

Depending on the surface property of the capture element **400,** in some embodiments, the surface of the capture element **400** can be modified to provide a lubricating layer **140,** e.g., to reduce binding of the microbe and/or microbial matter onto the material surface using any of the surface modification methods described herein. In one embodiment, the surface of the capture element **400** is modified such that microbes preferentially bind to the microbe-binding molecules **120.** As used herein, the term "preferential bind" refers to a microbe binding to a microbe-binding molecule with a higher likelihood or probability than to a capture element material surface. For example, the likelihood or probability for a microbe to bind to a microbe-binding molecule can be higher, e.g., by at least about 10%, at least about 20%, at least about 30%, at least about 40%, at least about 50%, at least about 60%, at least about 70%, at least about 80%, at least about 90%, at least about 95% or more, than binding to a capture element material surface. In some embodiments, the term "preferential bind" can refer to a microbe binding to a microbe-binding molecule with a likelihood or probability above a threshold level. For example, the binding of a microbe to a microbe-binding molecule with a likelihood or probability of at least about 50%, at least about 60%, at least about 70%, at least about 80%, at least about 90%, at least about 95%, at least about 98%, or more, can be considered as a preferential binding. In some embodiments, a microbe can bind to a microbe-binding molecule only, but not to a capture element material surface.

**FIGS. 4B** and **4C** show different exemplary embodiments of a microbe-binding device described herein. For example, the microbe-binding device **410** can comprise one or more capture elements **400** disposed in a chamber **420.** In some embodiments, the microbe-binding device **410** can comprise at least 1, at least 2, at least 3, at least 4, at least 5, at least 6, at least 7, at least 8, at least 9, at least 10 or more capture elements **400.** In some embodiments where the capture element is a mixing element, e.g., a spiral mixer **400** as shown in **FIGS. 4B-****4C,** the mixing element(s) can be disposed in the chamber **420** in any arrangement, e.g., placed in parallel to the flow (**FIG. 4B**) or transverse to the flow (**FIG. 4C**).

The size and/or volume of the chamber **420** can vary depending on a number of factors, including, but not limited to, volume of a fluid to be processed, and/or types of applications. In general, a larger chamber can be used to process a larger volume of a fluid. However, a small chamber can be desirable if portability is desirable.

While **FIGS. 4B** and **4C** illustrate a chamber **420** having a circular cross-section, a person having ordinary skill in the art readily appreciates that the chamber can have a cross-section of any shape, e.g., rectangular, square, oval, triangular, polygonal or any irregular-shaped. The chamber **420** can be made of any material, e.g., any biocompatible material that is inert to the fluid to be processed. An exemplary material that can be used in a chamber can include, but is not limited to, plastic.

In some embodiments, the device described herein can be integrated with a shunt system or adapted to connect to a shunt system. By way of example only, as shown in **FIG. 5****,** the device **410** can be adapted to connect to a shunt system **510.** The shunt system **510** can comprise a first end **520,** e.g., for collecting a fluid such as blood, and a second end **530,** e.g., for returning the filtered fluid such as blood to a patient. In such embodiments, a fluid flowing through the device **410** can have any microbes, if present, bound to the microbe-binding molecules of the capture element, and get filtered before returning to a patient This can be designed to be portable and used anywhere, e.g., for emergency applications such as military field applications. A standard shunt can be inserted into a jugular vein or femoral vein with a device **410** attached to the shunt. The device **410** can be disposable such that a patient can change the device **410** regularly to maintain microbe-capture effiency until he/she is transported to a hospital for treatment.

In addition, the device described herein can be integrated into any system for capturing and/or detecting a microbe. In some embodiments, the surface modification of the capture element surface can reduce background binding and thus, increase the detection sensitivity of the microbial detection assay.

### Capture Elements

A capture element employed in the device described herein can be of any structure, shape, and/or dimension.

A capture element can be made from a wide variety of materials and in a variety of formats. For example, the capture element can be utilized in the form of beads (including polymer microbeads, magnetic microbeads, superparamagnetic microbeads, superparamagnetic nanoparticles, and the like), filters, fibers, screens, mesh, tubes, hollow fibers, scaffolds, plates, channels, other substrates commonly utilized in assay formats, and any combinations thereof. Examples of capture elements can include, but are not limited to, nucleic acid scaffolds, protein scaffolds, lipid scaffolds, dendrimers, microparticles or microbeads, nanotubes, medical apparatuses (e.g., needles or catheters) or implants, microchips, filtration devices or membranes, hollow-fiber reactors, microfluidic devices, extracorporeal devices, and mixing elements (e.g., impellers, or mixers).

The capture element can be made of any material, e.g., any material that is compatible to a fluid to be processed. For example, the capture element can be made of any biocompatible material known in the art, e.g., but not limited to, TEFLON®, polysulfone, polypropylene, polystyrene, metal, metal alloy, polymer, plastic, glass, fabric, hydrogels, proteins, peptides, nucleic acids, and any combinations thereof. In some embodiments, the capture element can be made of a material that is resistant and/or inert to an organic solvent, if present, in the fluid to be processed.

In one embodiment, the capture element includes one or more mixing elements. As used herein, the term "mixing element" refers to any structural component constructed to faciliate mixing a fluid (e.g., to increase contact with microbe-binding molecules conjugated on the capture element). The mixing element can have any shape, depending on applications such as low-shear mixing (e.g., to prevent cell lysis or hemolysis) or high-shearing mixing (e.g., to facilitate lysis or homogenizing a component in a fluid). The size of the mixing element can vary with a number of factors, including but not limited to, size and/or design of the device housing, volume and/or viscosity of a fluid to be processed, number of mixing elements present in the device, and/or desirable fluid dynamics. In some embodiments, a plurality of smaller mixing elements (e.g., 2, 3, 4, 5, 6, 7, 8, 9, 10 or more mixing elements) can be used instead of a large single mixing element to provide a more controlled mixing.

The mixing element can be used to continually mix a wide variety and combination of fluids, solids and gases. The mixing element can be configured or designed to provide mixing for low-shear applications and high-shear turbulent mixing efficiency applications. As used herein, the term "low-shear mixing" generally means a laminar-flow type of mixing. In some embodiments, the term "low-shear mixing" with respect to a physiological range (e.g., in a human being) refers to a mixing with a shear stress of less than 1 dyne/cm². As used herein, the term "high-shear mixing" generally means a turbulent-flow type of mixing. In some embodiments, the term "high-shear mixing" with respect to a physiological range (e.g., in a human being) refers to a mixing with a shear stress of higher than 1 dyne/cm² and less than 15 dynes/cm². In some embodiments, the mixing element is constructed to provide low-shear mixing. One of skill in the art can design a low-shear mixing element, e.g., using computational modeling to predict the shear stress to be produced by the designed mixing element at a certain flow rate of a fluid.

In some embodiments, the mixing element can include an impeller. The term "impeller" as used herein refers to any structures that can be caused to move and in turn cause molecules locating proximate the impeller to move in response to the motion of the impeller. In some embodiments, the impeller can include rotary impeller.

In some embodiments, the mixing element can include a mixer, e.g., spiral mixer or a static mixer. A static mixer is generally an assembly of one or more elements such as fins or manifolds disposed in a flow conduit that disturb the flow to cause 'folding' or 'mixing' of the fluidic content, or subdividing and recombining the flow. **FIGS. 6A-6E** illustrate different exemplary embodiments of static mixers that can be subjected to optional surface modification described herein and coated with microbe-binding molecules described herein. **FIG. 6A** shows a Kenics mixer (right twist-left twist; angle of blade twist 180 °). **FIG.** 6B shows a Ross LPD (right rotation- left rotation; crossing angle θ = 90°). **FIG. 6C** shows a standard Sulzer SMX (n, Nₚ, Nₓ) = (number of crosses over the height, number of parallel bars/fins over the length, number of crossing bars/fins over the width) = (2, 3, 8). Two examples of the more efficient SMX(n) (n, Nₚ, Nₓ) = (n, 2n-1, 3n) are shown: rectangular version of the "working" horse (n=1) in **FIG. 6D** and the compact version (n=3) in **FIG. 6E****.**

In some embodiments, the capture element and/or mixing element can include a plurality of posts or pillars disposed in a flow conduit that disturb the flow of a fluid. For example, there can be an array of posts or pillars disposed in a channel through which the fluid flows. When the fluid is in contact with the posts or pillars, which include on their surfaces a plurality of microbe-binding molecules, any microbes, if present, in the fluid bind to the posts or pillars, and thus get removed from the fluid.

In some embodiments, the capture element and/or mixing element can include magnetic microbeads, which can be used, e.g., in combination with two electromagnets placed opposite on either side of the device or housing to form a cycling electromagnetic mixer.

In various embodiments, the surface of the capture element can further comprise at least one scaffold or dendrimer molecule (e.g., nucleic acid or peptide scaffold). Such embodiments can be desired for increasing the surface area available for conjugation with more microbe-binding molecules.

### Test Sample

In accordance with various embodiments described herein, a test sample or sample, including any fluid or specimen (processed or unprocessed), that is suspected of comprising a microbe and/or microbial matter can be subjected to an assay or method, kit and system described herein. The test sample or fluid can be liquid, supercritical fluid, solutions, suspensions, gases, gels, slurries, and combinations thereof. The test sample or fluid can be aqueous or non-aqueous.

In some embodiments, the test sample can be an aqueous fluid. As used herein, the term "aqueous fluid" refers to any flowable water-containing material that is suspected of comprising a microbe and/or microbial matter.

In some embodiments, the test sample can include a biological fluid obtained from a subject. Exemplary biological fluids obtained from a subject can include, but are not limited to, blood (including whole blood, plasma, cord blood and serum), lactation products (e.g., milk), amniotic fluids, sputum, saliva, urine, semen, cerebrospinal fluid, bronchial aspirate, perspiration, mucus, liquefied feces, synovial fluid, lymphatic fluid, tears, tracheal aspirate, and fractions thereof. In some embodiments, a biological fluid can include a homogenate of a tissue specimen (*e*.*g*., biopsy) from a subject.

In some embodiments, the biological fluid sample obtained from a subject, *e*.*g*., a mammalian subject such as a human subject or a domestic pet such as a cat or dog, can contain cells from the subject. In other embodiments, the biological fluid sample can contain non-cellular biological material, such as non-cellular fractions of blood, saliva, or urine, which can be used to measure plasma/serum biomarker expression levels.

The biological fluid sample can be freshly collected from a subject or a previously collected sample. In some embodiments, the biological fluid sample used in the assays and/or methods described herein can be collected from a subject no more than 24 hours, no more than 12 hours, no more than 6 hours, no more than 3 hours, no more than 2 hours, no more than 1 hour, no more than 30 mins or shorter.

In some embodiments, the biological fluid sample or any fluid sample described herein can be treated with a chemical and/or biological reagent described herein prior to use with the assays and/or methods described herein. In some embodiments, at least one of the chemical and/or biological reagents can be present in the sample container before a fluid sample is added to the sample container. For example, blood can be collected into a blood collection tube such as VACUTAINER®, which has already contained heparin. Examples of the chemical and/or biological reagents can include, without limitations, surfactants and detergents, salts, cell lysing reagents, anticoagulants, degradative enzymes (*e.g*., proteases, lipases, nucleases, collagenases, cellulases, amylases), and solvents such as buffer solutions.

In some embodiments, the test sample can include a fluid or specimen obtained from an environmental source, *e*.*g*., but not limited to, water supplies (including wastewater), ponds, rivers, reservoirs, swimming pools, soils, food processing and/or packaging plants, agricultural places, hydrocultures (including hydroponic food farms), pharmaceutical manufacturing plants, animal colony facilities, and any combinations thereof.

In some embodiments, the test sample can include a fluid (*e.g*., culture medium) from a biological culture. Examples of a fluid (*e.g*., culture medium) obtained from a biological culture includes the one obtained from culturing or fermentation, for example, of single- or multi-cell organisms, including prokaryotes (*e*.*g*., bacteria) and eukaryotes (*e.g*., animal cells, plant cells, yeasts, fungi), and including fractions thereof. In some embodiments, the test sample can include a fluid from a blood culture. In some embodiments, the culture medium can be obtained from any source, *e*.*g*., without limitations, research laboratories, pharmaceutical manufacturing plants, hydrocultures (*e.g.*, hydroponic food farms), diagnostic testing facilities, clinical settings, and any combinations thereof.

In some embodiments, the test sample can include a media or reagent solution used in a laboratory or clinical setting, such as for biomedical and molecular biology applications. As used herein, the term "media" refers to a medium for maintaining a tissue, an organism, or a cell population, or refers to a medium for culturing a tissue, an organism, or a cell population, which contains nutrients that maintain viability of the tissue, organism, or cell population, and support proliferation and growth.

As used herein, the term "reagent" refers to any solution used in a laboratory or clinical setting for biomedical and molecular biology applications. Reagents include, but are not limited to, saline solutions, PBS solutions, buffered solutions, such as phosphate buffers, EDTA, Tris solutions, and any combinations thereof. Reagent solutions can be used to create other reagent solutions. For example, Tris solutions and EDTA solutions are combined in specific ratios to create "TE" reagents for use in molecular biology applications.

In some embodiments, the test sample can be a non-biological fluid. As used herein, the term "non-biological fluid" refers to any fluid that is not a biological fluid as the term is defined herein. Exemplary non-biological fluids include, but are not limited to, water, salt water, brine, buffered solutions, saline solutions, sugar solutions, carbohydrate solutions, lipid solutions, nucleic acid solutions, hydrocarbons (*e*.*g*. liquid hydrocarbons), acids, gasoline, petroleum, liquefied samples (*e*.*g*., liquefied samples), and mixtures thereof.

### Exemplary Microbes or Pathogens

As used herein, the term "microbes" or "microbe" generally refers to microorganism(s), including bacteria, fungi, protozoan, archaea, protists, *e*.*g*., algae, and a combination thereof. The term "microbes" encompasses both live and dead microbes. The term "microbes" also includes pathogenic microbes or pathogens, *e*.*g*., bacteria causing diseases such as plague, tuberculosis and anthrax; protozoa causing diseases such as malaria, sleeping sickness and toxoplasmosis; fungi causing diseases such as ringworm, candidiasis or histoplasmosis; and bacteria causing diseases such as sepsis.

***Microbe-induced diseases:*** In some other embodiments, the engineered microbe-binding molecules or capture elements, products and kits described herein can be used to detect or bind to the following microbes that causes diseases and/or associated microbial matter: *Bartonella henselae, Borrelia burgdorferi, Campylobacter jejuni, Campylobacterfetus, Chlamydia trachomatis, Chlamydia pneumoniae, Chylamydia psittaci, Simkania negevensis, Escherichia coli* (*e.g.,* O157:H7 and K88), *Ehrlichia chafeensis, Clostridium botulinum, Clostridium perfringens, Clostridium tetani, Enterococcus faecalis, Haemophilius influenzae, Haemophilius ducreyi, Coccidioides immitis, Bordetella pertussis, Coxiella burnetii, Ureaplasma urealyticum, Mycoplasma genitalium, Trichomatis vaginalis, Helicobacter pylori, Helicobacter hepaticus, Legionella pneumophila, Mycobacterium tuberculosis, Mycobacterium bovis, Mycobacterium africanum, Mycobacterium leprae, Mycobacterium asiaticum, Mycobacterium avium, Mycobacterium celatum, Mycobacterium celonae, Mycobacterium fortuitum, Mycobacterium genavense, Mycobacterium haemophilum, Mycobacterium intracellulare, Mycobacterium kansasii, Mycobacterium malmoense, Mycobacterium marinum, Mycobacterium scrofulaceum, Mycobacterium simiae, Mycobacterium szulgai, Mycobacterium ulcerans, Mycobacterium xenopi, Corynebacterium diptheriae, Rhodococcus equi, Rickettsia aeschlimannii, Rickettsia africae, Rickettsia conorii, Arcanobacterium haemolyticum, Bacillus anthracis, Bacillus cereus, hysteria monocytogenes, Yersinia pestis, Yersinia enterocolitica, Shigella dysenteriae, Neisseria meningitides, Neisseria gonorrhoeae, Streptococcus bovis, Streptococcus hemolyticus, Streptococcus mutans. Streptococcus pyogenes, Streptococcus pneumoniae, Staphylococcus aureus, Staphylococcus epidermidis, Staphylococcus pneumoniae, Staphylococcus saprophyticus, Vibrio cholerae, Vibrio parahaemolyticus, Salmonella typhi, Salmonella paratyphi, Salmonella enteritidis, Treponema pallidum, Human rhinovirus, Human coronavirus, Dengue virus, Filoviruses (e.g., Marburg and Ebola viruses), Hantavirus, Rift Valley virus, Hepatitis B, C, and E, Human Immunodeficiency Virus (e.g., HIV-1, HIV-2), HHV-8, Human papillomavirus, Herpes virus (e.g., HV-I and HV-II), Human T-cell lymphotrophic viruses (e.g., HTLV-I and HTLV-II), Bovine leukemia virus. Influenza virus, Guanarito virus, Lassa virus, Measles virus, Rubella virus, Mumps virus, Chickenpox (Varicella virus), Monkey pox, Epstein Bahr virus, Norwalk (and Norwalk-like)* viruses, *Rotavirus, Parvovirus B19, Hantaan virus, Sin Nombre virus, Venezuelan equine encephalitis, Sabia virus, West Nile virus, Yellow Fever virus, causative agents of transmissible spongiform encephalopathies, Creutzfeldt-Jakob disease agent, variant Creutzfeldt-Jakob disease agent, Candida, Cryptcooccus, Cryptosporidium, Giardia lamblia, Microsporidia, Plasmodium vivax, Pneumocystis carinii, Toxoplasma gondii, Trichophyton mentagrophytes, Enterocytozoon bieneusi, Cyclospora cayetanensis, Encephalitozoon hellem, Encephalitozoon cuniculi,* among other viruses, bacteria, archaea, protozoa, and fungi).

In some embodiments, the engineered microbe-binding molecules or capture elements, products and kits described herein can be used to differentiate a protein A-expressing or protein G-expressing microbe from protein A- and protein G-negative microbes (*e.g.*, *E. coli*) by employing the methods or assays described herein.

In some embodiments, a protein A-expressing microbe includes *Staphylococcus* species. Examples of *Staphylococcus* species include, but are not limited to, *S. aureus* group (*e.g., S. aureus, S. simiae*), *S. auricularis* group (*e.g., S. auricularis*), *S. carnosus* group (*e.g., S. carnosus, S. condimenti, S. massiliensis, S. piscifermentans, S. simulans*), *S. epidermidis* group (*e*.*g*., *S. capitis, S. caprae, S. epidermidis, S. saccharolyticus*), *S. haemolyticus* group (*e.g., S. devriesei, S. haemolyticus, S. hominis*)*, S. hyicus-intermedius* group *(eg., S. chromogenes, S. felis, S. delphini, S. hyicus, S. intermedius, S. lutrae, S. microti, S. muscae, S. pseudintermedius, S. rostri, S. schleiferi*), *S. lugdunensis* group (*e.g., S. lugdunensis*), *S. saprophyticus* group (*e.g., S. arlettae, S. cohnii, S. equorum, S. gallinarum, S. kloosii, S. leei, S. nepalensis, S. saprophyticus, S. succinus, S. xylosus*), *S. sciuri* group (*e.g., S. fleurettii, S. lentus, S. sciuri, S. stepanovicii, S. vitulinus*), *S. simulans* group (*e.g., S. simulans*), *and S. warneri* group (*e.g., S. pasteuri, S. warneri*).

In some embodiments, *S. aureus* can be differentiated from a protein A- and protein G-negative microbe (*e.g., E. coli*) using the assays and/or methods described herein.

In some embodiments, *S. aureus* can be differentiated from *S*. *epidermidis* using the assays and/or methods described herein.

In some embodiments, *S. epidermidis* cannot be differentiated from a protein A- and protein G-negative microbe (*e.g., E, coli*) using the assays and/or methods described herein.

In some embodiments, a protein G-expressing microbe includes *Streptococcus* species. Examples of *Streptococcus* species can include, but are not limited to, alpha-hemolytic including *Pneumococci* (*e.g., S. pneumonia*), and the Viridans group (*e.g., S. mutans, S. mitis, S. sanguinis, S. salivarius, S. salivarius ssp. thermophilus, S. constellatus*); and beta-hemolytic including Group A (*e.g., S. pyogenes*), Group B (*e.g., S. agalactiae*), Group C (*e.g., S. equi,* and *S. zooepidemicus*), Group D (*e.g., enterococci, Streptococcus bovis and Streptococcus equinus*), Group F *streptococci,* and Group G *streptococci.*

In some embodiments, a protein G-expressing microbe includes Group C and Group G *streptococci.*

One skilled in the art can understand that the engineered microbe-binding molecules or capture elements, products and kits described herein can be used to target any microorganism with a microbe surface-binding domain described herein modified for each microorganism of interest. A skilled artisan can determine the cell-surface proteins or carbohydrates for each microorganism of interest using any microbiology techniques known in the art.

***Biofilm:*** Accordingly, in some embodiments, the microbe-binding molecules or capture elements, products and kits herein can be used to detect microbes and/or assocatied microbial matter present in a biofilm or to treat equipment surfaces to prevent or inhibit formation of a biofilm. For example, *Listeria monocytogenes* can form biofilms on a variety of materials used in food processing equipment and other food and non-food contact surfaces (Blackman, J Food Prot 1996; 59:827-31; Frank, J Food Prot 1990; 53:550-4; Krysinski, J Food Prot 1992; 55:246-51; Ronner, J Food Prot 1993; 56:750-8). Biofilms can be broadly defined as microbial cells attached to a surface, and which are embedded in a matrix of extracellular polymeric substances produced by the microorganisms. Biofilms are known to occur in many environments and frequently lead to a wide diversity of undesirable effects. For example, biofilms cause fouling of industrial equipment such as heat exchangers, pipelines, and ship hulls, resulting in reduced heat transfer, energy loss, increased fluid frictional resistance, and accelerated corrosion. Biofilm accumulation on teeth and gums, urinary and intestinal tracts, and implanted medical devices such as catheters and prostheses frequently lead to infections (Characklis W G. Biofilm processes. In: Characklis W G and Marshall K C eds. New York: John Wiley & Sons, 1990:195-231; Costerton et al., Annu Rev Microbiol 1995; 49:711-45). In some embodiments, the engineered microbe-binding microparticles, *e*.*g*., encapsulating a drug or a chemical for treatment of a biofilm, can be sprayed on contaminated equipment surfaces. The bacteria present in the biofilm bind to the microbe-binding microparticles, which release the drug to treat the bacteria for targeted drug delivery.

In addition, *L. monocytogenes* attached to surfaces such as stainless steel and rubber, materials commonly used in food processing environments, can survive for prolonged periods (Helke and Wong, J Food Prot 1994; 57:963-8). This would partially explain their ability to persist in the processing plant. Common sources of *L. monocytogenes* in processing facilities include equipment, conveyors, product contact surfaces, hand tools, cleaning utensils, floors, drains, walls, and condensate (Tomkin et al., Dairy, Food Environ Sanit 1999; 19:551-62; Welbourn and Williams, Dairy, Food Environ Sanit 1999; 19:399-401). In some embodiments, the engineered microbe-binding molecules can be configured to include a "smart label", which is undetectable when conjugated to the engineered microbe-binding molecules, but produces a color change when released from the engineered molecules in the presence of a microbe enzyme. Thus, when a microbe binds to the engineered microbe-binding molecules, the microbe releases enzymes that release the detectable label from the engineered molecules. An observation of a color change indicates a risk for bacteria contamination on a particular surface, and thus some embodiments of the engineered microbe-binding molecules and products can be used for early detection of biofilm formation.

***Plant microbes:*** In still further embodiments, the engineered microbe-binding molecules or capture elements and products described herein can be used to target plant microbes and/or associated microbial matter. Plant fungi have caused major epidemics with huge societal impacts. Examples of plant fungi include, but are not limited to, *Phytophthora infestans, Crinipellis perniciosa,* frosty pod (*Moniliophthora roreri*), oomycete *Phytophthora capsici, Mycosphaerella fijiensis, Fusarium Ganoderma spp* fungi and *Phylophihora.* An exemplary plant bacterium includes *Burkholderia cepacia.* Exemplary plant viruses include, but are not limited to, soybean mosaic virus, bean pod mottle virus, tobacco ring spot virus, barley yellow dwarf virus, wheat spindle streak virus, soil born mosaic virus, wheat streak virus in maize, maize dwarf mosaic virus, maize chlorotic dwarf virus, cucumber mosaic virus, tobacco mosaic virus, alfalfa mosaic virus, potato virus X, potato virus Y, potato leaf roll virus and tomato golden mosaic virus.

***Military and bioterrorism applicadons:*** In yet other embodiments, the engineered microbe-binding molecules and product comprising thereof can be used to detect or combat bioterror agents (*e.g., B. Anthracis,* and smallpox).

In accordance with some embodiments described herein, an engineered microbe-binding molecule or microbe-binding capture element can be modified to bind to any of the microbes, *e*.*g*., the ones described herein, including the associated microbial matter (e.g., but not limited to, fragments of cell wall, microbial nucleic acid and endotoxin).

### EXAMPLES

The following examples are presented for the purpose of illustration only and are not intended to be limiting.

### Example 1

0.1 mm glass beads were coated with a combination of perfluorcarbon silane (Trichloro(1H,1H,2H,2H-perfluorooctyl)silane) and aminosilane (3-aminopropyltrimethoxysilane).

The treated glass beads were cleaned in soapy water, rinsed with distilled deionized water and treated with 1 molar sodium hydroxide for 1 hour to clean and activate the surface of the beads. Then, the beads were rinsed with distilled, deionized water, rinsed with anhydrous ethanol, and finally dried at 80 °C for 48 hours.

Then, 5.0 g of beads were weighed out into four separate 50-mL conical vials. Anhydrous ethanol was added to the 10 mL mark in each vial and 500 microliters of silane was added to each vial according to the volume ratio listed below.
Bead A = beads silanized with a 1:1 ratio of amine containing and perfluorocarbon containing silanes.
Bead B = beads silanized with a 1:10 ratio of amine containing and perfluorocarbon containing silanes.
Bead C = beads silanized with a 1:100 ratio of amine containing and perfluorocarbon containing silanes.
Bead D= beads silanized with a 1:1000 ratio of amine containing and perfluorocarbon containing silanes.
Bead E = beads silanized with pure perfluorocarbon containing silane.

The different beads were the incubated at room temperature for 1 hour, rinsed three times with 50 mL of anhydrous ethanol, the supernatant decanted, and then dried in oven overnight at 80 °C. The resulting glass beads are schematically illustrated in the top of FIG. 7A.

Then, each of the vials containing the different beads (Beads A through E) were reacted with FcMBL, which is schematically shown in the middle of FIG. 7A, followed by filling with perflurocarbon oil, followed by addition of mineral oil layer. The resulting glass beads having a SLIPS surface with FcMBL that can selectively bind to a target (e.g., *S*. *aureus*) is schematically illustrated in the bottom of **FIG. 7A****.**

Then, 12 mL of untreated blood was added followed by a wash with PFC oil/PBS.

A control experiment was carried out with glass beads that do not contain any silanization where 12 mL of untreated blood was added followed by a wash with PBS.

As shown in **FIG. 7B****,** much less blood remained on the columns that contained beads with increasing amounts of perfluorocarbon containing silanes. For example, the column containing no perfluorocarbon containing silanes (left, Control) was heavily stained with blood whereas the column that contained Beads E had little to no discernible amounts of blood remaining. In other words, the mixed silanization can still tolerate a certain amount of non-perfluorocarbon functionalization that would be expected to diminish the repellency without losing its repellency. Without wishing to be bound by theory, it is expected that the PFC oil wash covers the amine groups that allows the glass beads to maintain its repellency.

Beads A through D were then each placed inside a test tube. Then, per 1 mg of the glass beads, 1 microgram of FcMBL (FcMBL stock at 1.7 mg/ml in phosphate buffered saline, pH 7.4) and 14.8 microgram of EDC I-ethyl-3-(3-dimethylaminopropyl) carbodiimide] (EDC stock at 40mg/ml in MES buffered saline, pH 4.5) were added and mixed together.

The bead solutions were vortexed to force the beads into solution at room temperature for 40 minutes. Then 500 microliters of TBS was added to quench the reaction.

The solution was then spun down in a centrifuge and the supernatant was removed.

1 mL of PBS was then added, spun down and the supernatant removed again. The PBS wash was repeated twice.

The resulting materials were each resuspended in 1 mL of PBS with 1% BSA.

The following protocol was followed to determining whether *S. aureus* selectively binds to the treated glass beads
- Grow *S. aureus* overnight in 2XYT
- Spin down and wash 1mL of culture with TBS-T w/ 5mM CaCl2
- Resuspend in 1mL of TBS-T w/ 5mM CaCl2
- Mix beads and add 20-30 microliters to tube
- Remove excess water
- If appropriate:
- Add 200 microliters FC-70 and shake at 400 rpm for 20 minutes
- Remove excess FC-70
- If appropriate:
- Add 200 microliters TBS-T with 5mM Calcium
- Add 10 microliters of washed overnight culture of S. aureus
- Mix at 800 rpm for 20 minutes.
- Mix on Hula mixer ( Life Technologies) for 20 minutes
- Wash beads 2X in TBS-T w/ 5mM CaC12 and 1X with TBS w/ 5mM CaC12
- Add 200 microliters of HRP-FcMBL (1:5000 dilution of the stock). HRP Fc MBL is FcMBL (Sequence 6 that has been conjugated with Horseradish peroxidase enzyme for use in colorimetric assays e.g. ELISA
- Mix for 30 minutes at 400rpm
- Remove excess HRP-FcMBL
- Wash 2X with TBS-T w/ CaC12
- Wash 2X with TBS w/ CaC12
- Remove excess liquid
- Add 100 microliters of TMB substrate and let sit for 2.5 minutes
- Add 100 microliters 1M H2S04 to stop the enzymatic reaction.
- Transfer 150 microliters to 96 well plate and Read Absorbance at 450nm

ELISA was performed using SLIPS, FcMBL conjugated glass beads. As control experiments, the following experiments were carried out.

As a first control, Bead A with FcMBL was used, but with no FC-70 and no *S*. *aureus* added. As shown in **FIG. 7C****,** this first control is indicated as "No Microbe Control" and shows background noise of the ELISA spectrum.

As a second control, Bead A with FcMBL was used, but no FC-70 was added. As shown in **FIG. 7C****,** this second control is indicated as "Bead A no oil" and the presence of FcMBL selectively bound *S. aureus* as evidenced by the strong absorbance at 450 nm. It should be noted that the absorbance saturation of the ELISA detector was at about 3, demonstrating a very strong signal-to-noise ratio.

Moreover, as shown in **FIG. 7C****,** beads functionalized with FcMBL shows saturated absorbance signals for all Beads A through D (bar graph corresponding to "+FcMBL"). This demonstrates that with FcMBL, selective binding of *S*. *aureus* occurred while other undesired moieties were repelled, leading to a very high signal-to-noise ratio.

Moreover, as shown in **FIG. 7C****,** beads that were *not* functionalized with FcMBL shows a continuous decrease in the absorbance peak with increasing amounts of the perfluorocarbon containing silanes. That is, as described in Example 19, Bead D contained the highest relatively amount of the perfluorocarbon containing silanes. Importantly, even with a 1:10 ratio of amine containing and perfluorocarbon containing silanes, most of the undesired moieties (and desired moieties) were repelled from the glass beads as evidenced by the absorbance value being similar to the "No Microbe Control" absorbance value.

### Example 2

Pieces of acrylic were first plasma cleaned. Plasma clean acrylic surfaces were then treated with various silanization solutions (silane 5% v/v in ethanol) for 1 hour at room temperature. The following different silanization solutions were utilized.
- 100% (3-aminopropyl)trimethoxysilane-tetramethoxysilane (APTMS)
- 50% APTMS/50% Trichloro(1H,1H,2H,2H-perfluorooctyl)silane (6C-PFC)
- 10% APTMS/90% 6C-PFC
- 1% APTMS/99% 6C-PFC
- 100% 6C-PFC

The surfaces were then washed in 100% ethanol and then in water.

FcMBL conjugation solutions were made of 1 part FcMBL (stock concentration = 1.75 mg/ml) in phosphate buffered saline (PBS) and 1 part EDC (stock concentration = 20mg/ml) in MES buffered saline (MBS) and spotted on to half of the silanized acrylic surfaces. The conjugation reaction was allowed to proceed for 1.5 hours and then the reaction was quenched with Tris buffered saline (TBS) and then washed in PBS.

Fluorinert FC-70 was then applied to the surface. The surfaces were tilted to allow excess FC-70 to flow off and the edges were lightly touched with a ChemWipe to remove any residual excess FC-70. The resulting surfaces are shown in **FIG. 8A****.**

75ul of Fresh Whole Blood with 0.25 units of the anticoagulant heparin per ml was added to each surface. The spots of blood were allowed to sit for 2-5 minutes and then each surface was tilted to see if the blood would be repelled by the surfaces.

Photos were taken after the blood was initially spotted on to the surfaces and then again after the tilt test to remove unbound blood.

**FIG. 8B** shows images of acrylic surfaces that have been plasma cleaned, but not silanized or conjugated with FcMBL. The surface on the right was oiled with FC-70, while the surface on the left was not. Both surfaces show the expected result of blood sticking to surfaces that had not been treated with a perfluorocarbon silane group (6C-PFC silane). As shown, blood adheres to the control surfaces. While the blood adheres to the entire acrylic surface for a plasma treated surface, blood adheres where at the spot where the blood was deposited. Without wishing to be bound by theory, on the right, it is thought that blood displaces the FC-70 oil and adheres to the underlying plasma treated acrylic surface.

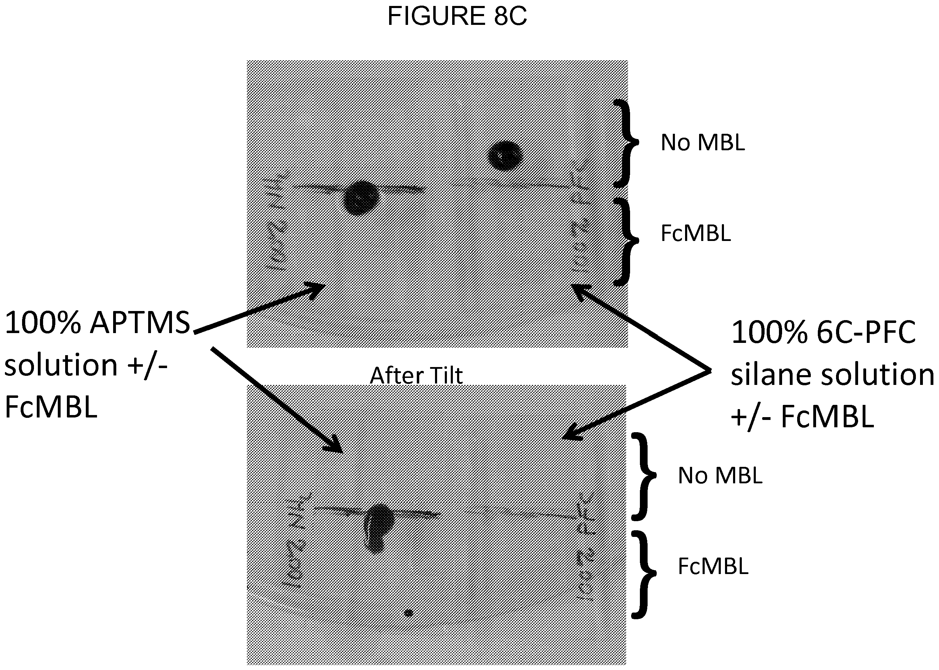

**FIG. 8C** shows images of acrylic surfaces that have been plasma cleaned and treated with a 100% APTMS (left surface) or a 100% 6C-PFC (right surface) silane solution. Part of the acrylic surface marked below the line was exposed to an FcMBL conjugation solution. FC-70 was applied to both surfaces prior to addition of blood. As expected the surface treated with a silane solution free of the perfluorocarbon silane (6C-PFC) did not repel blood. Because there are no free amine groups on the acrylic surface treated with the 100% 6C-PFC silane solution, FcMBL will not actually be conjugated to the surface, rather this step was included for consistency purposes. Without wishing to be bound by theory, on the left image, it is thought that blood displaces the FC-70 oil and adheres to the underlying 6C-PFC silane groups.

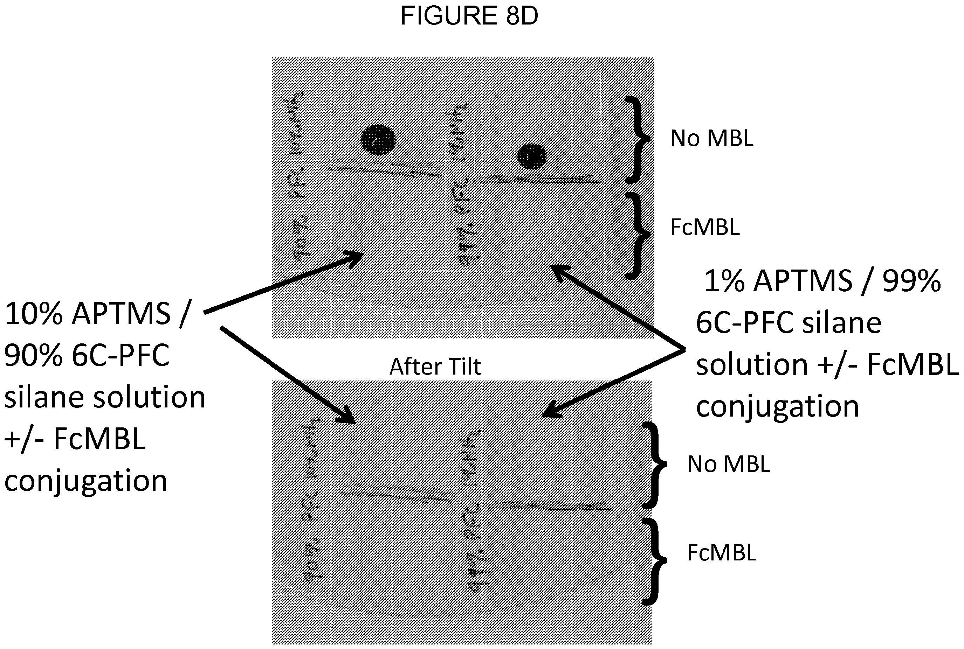

**FIG. 8D** shows images of acrylic surfaces that have been plasma cleaned and treated with a 10% APTMS / 90% 6C-PFC (left surface) or a 1% APTMS / 99% 6C-PFC (right surface) silane solution and exposed to an FcMBL conjugation solution. FC-70 was applied to both surfaces prior to addition of blood. As shown, blood does not adhere to either surfaces, demonstrating that slippery surfaces can be formed even up to 10% APTMS.

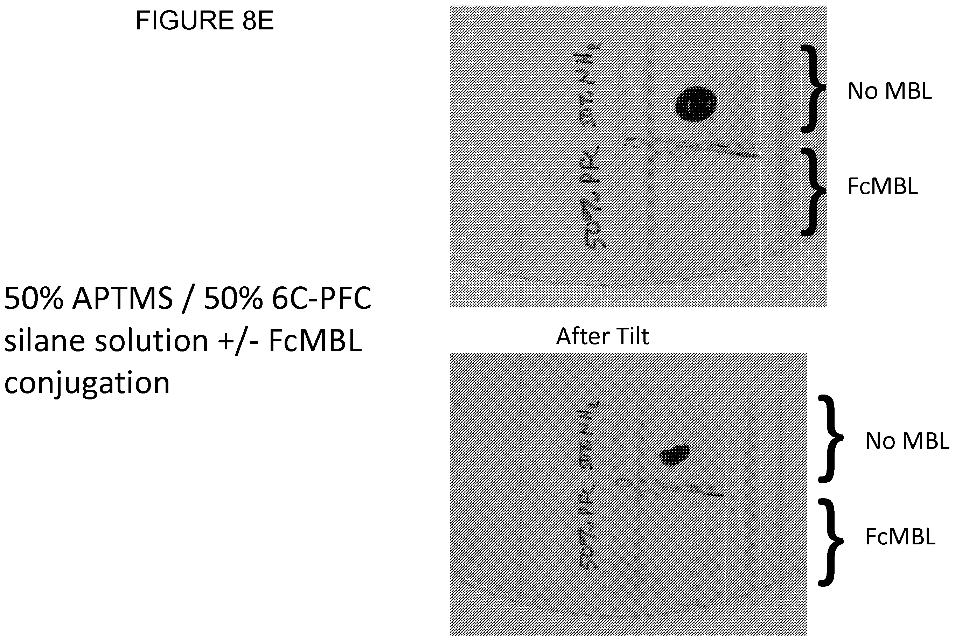

**FIG. 8E** shows images of acrylic surfaces that have been plasma cleaned and treated with a 50% APTMS / 50% 6C-PFC silane solution and exposed to an FcMBL conjugation solution. FC-70 was applied to the surface. As shown, surfaces with about 50% APTMS begins to lose some of the slippery characteristics as some blood adheres to the surface. While not shown in **FIG. 8E****,** some other experiments under the same conditions show surfaces that do not adhere blood.

### Example 3

Patterned surfaces having regions that have proteins and regions that have ultra slippery surfaces were produced as follows.

First, desired stamp patterns were printed on a chrome mask followed by fabrication of a silicon-based mold with SU-8 photoresist patterns through standard photolithography in a clean room.

Soft lithography was then implemented to produce PDMS stamps using the fabricated mold. **FIGS. 9A** and **9B** show optical microscope images of the fabricated PDMS stamps. The produced stamps were used to micro-contact print desired molecules (Proteins/Silane etc) onto a glass surface.

For micro-contact printing, different PDMS stamp designs were used to micro-contact biomolecules onto the substrate (glass, PDMS PMMA ...). Substrates were cleaned and extensively rinsed with DI water, and dried under nitrogen. PDMS stamps were exposed to UV light for 20 min and rinsed with 70% ethanol. Each PDMS stamp was covered with 15 µl of the desired bimolecule (20 µg/ml) at room temperature. A plasma treated cover slip (60 s, 200 W, 200 mTorr O2) was placed on the stamp for 10 min, to help spread the antibody solution as well as avoid evaporation. After rinsing with PBS and distilled water (10 s each) and drying under nitrogen, the stamp was gently brought into contact with the glass substrate for 60 s. The micro-contact printed surfaces can be stored at 4°C for up to 4 weeks and would keep bio-functionality at 37°C for at least three weeks.

Patterned surface was then silanized (trichlorosilane) in a desiccator under vacuum for 5 hours. Vapor deposition here was critical to preserve patterned biomolecules as in wet conditions applying ethanol were destructive to the patterns.

A lubricating liquid (FC70) was then applied on the surface. Functionality of the patterned surfaces was investigated using an immunoassay on patterned proteins after generating the slippery surface.

**FIGS. 9C, 9D and 9E** show the patterned superhydrophobic slippery surfaces with human IgG primary antibody that were fluorescently labeled with secondary human IgG. As shown, the produced surfaces behaved as ultra slippery surfaces with super hydrophobic properties while the patterned primary antibodies remained functional and captured the fluorescently labeled secondary antibodies. Moreover, biofilm formation and blood clotting were not observed on these surfaces.

**FIGS. 10A** through **10D** show capture and detection of *S. aureus* on from whole blood using the linearly patterned superhydrophobic slippery surfaces using FcMBL as the biomolecule. As shown, **FIGS. 10A** and **10C** represent fluorescently labeled capture molecule (FcMBL) patterned on a slippery surface. **FIGS. 10B** and **10D** show pathogen (S. aureus) capture on the patterned surface shown in **FIGS. 10A** and **10C****,** respectively. This confirms selected capture and detection of desired species (pathogen) using a slippery surface.

**FIGS. 11A** through **11D** show capture and detection of *Candida* on from whole blood using the dot patterned superhydrophobic slippery surfaces using FcMBL as the biomolecule. As shown, **FIG. 11A** represents fluorescently labeled capture molecule (FcMBL) patterned on a slippery surface. **FIG. 11B** shows detection and capture of fluorescently labeled pathogen (*Candida*) on the patterned surface shown in **FIG. 11A. FIGS. 11C** and **11D** show 3D fluorescence intensities of capture molecule and captured pathogen on the slippery surface shown in **FIG. 11A** and **FIG. 11B****,** respectively.

**FIG. 12** shows the images of pathogen capture from whole blood on bio-functional slippery surface versus a bio-functional surface, which was used as the control sample. As shown, blood does not clot or spread on the bio-functional slippery surface while on the bio-functional surface, blood spreads on the surface and clots rapidly.

The graph shown in **FIG. 13** compares the non-specific adhesion/capture of pathogens on bio-functional slippery surfaces versus bio-functional control surfaces. As shown, non-specific adhesion drastically decreases using bio-functional slippery surfaces. This provides tremendous advantages for diagnostic systems. The images from **FIGS. 10A-10D** and **FIG. 11** were further analyzed to find out non-specific binding of pathogens using image J softeware. Two different fluorescence dyes were used to stain FcMBL and pathogens. The areas that had pathogen without the presence of fluorescence FcMBL were considered non-specific binding by the software. The quantitavie results for non-specific binding of pathogens have been shown in **FIG. 13**

### Example 4

Surfaces that have been patterned with bio-functional micro-beads were also produced. Such bio-functional micro-beads can enhance the surface to volume ratio on the surface while avoiding non-specific adhesion by the presence of the ultra-slippery surface.

Six different steps were followed to produce the slippery beads: design and fabrication of the PDMS stamps, immobilizing and patterning biotin onto the interface using micro-contact printing, attaching streptavidin conjugated beads to the biotin pattered interface, silanizing the surface and beads at the same time, functionalizing the beads with the capture molecule (biotinylated Fc-MBL) and finally applying a lubricating liquid.

First, desired stamp patterns were printed on a chrome mask followed by fabrication of a silicon-based mold with SU-8 photoresist patterns through standard photolithography in a clean room.

Second, soft lithography was then implemented to produce PDMS stamps using the fabricated mold. The produced stamps were used to micro-contact print a biotin conjugated biomolecule (biotinylated human IgG) to a substrate (glass). Then, micro-contact printing was carried out. Different PDMS stamp designs were used to micro-contact biomolecules onto the substrate (glass, PDMS PMMA ...). Substrates were cleaned and extensively rinsed with DI water, and dried under nitrogen. PDMS stamps were exposed to UV light for 20 min and rinsed with 70% ethanol. Each PDMS stamp was covered with 15 µl of the desired bimolecule (20 µg/ml) at room temperature. A plasma treated cover slip (60 s, 200 W, 200 mTorr O2) was placed on the stamp for 10 min, to help spread the antibody solution as well as avoid evaporation. After rinsing with PBS and distilled water (10 s each) and drying under nitrogen, the stamp was gently brought into contact with the glass substrate for 60 s. The micro-contact printed surfaces can be stored at 4°C for up to 4 weeks and would keep bio-functionality at 37°C for at least three weeks.

Third, streptavidin conjugated beads (1 µm) were incubated on the patterned surfaces for 15-20 min to attach the beads to the patterned biotin on the surface.

Fourth, patterned surfaces along with attached beads, were then silanized (trichlorosilane) in a desiccator under vacuum for 5 hours.

Fifth, biotinylated capture molecule (Fc-MBL) was then incubated with the surface for 15-20 min to functionalize the streptavidin-conjugated beads with the capture bio-molecule.

Finally and right before testing the surfaces a lubricating liquid (FC70) was applied to the interface containing the beads. Produced surfaces behaved as ultra-slippery surfaces with superhydrophobic properties.

As shown in **FIG. 14A****,** contact angle measurement confirm the changes in the contact angle as an indication of producing biofunctional slippery beads. As shown, contact angles were measured after adding the lubricating liquid to beads functionalized with silane ("After Silane") and beads that were not functionalized with silane ("No Silane").

Moreover, three different conditions were examined to show the effect of slippery beads.

First, beads were deposited on silanized surfaces (without any particular patterning) and were used as control (see top row in **FIG. 14B**).

Second, lubricating oil was added to the silanized surfaces with patterned beads (see middle row in **FIG. 14B**).

Finally, beads were also silanized together with their flat substrate and then the lubricating oil was added (see bottom row in **FIG. 14B**).

**FIG. 14B** shows the fluorescent microscope images of the produced surfaces after exposure to E. coli (left columns) and S. aureus (right columns). The circular donut shape gray cells in the images are red blood cells. As shown in the images, the number of red blood cells decreases significantly when bio-functional slippery surfaces were used (middle row) and even more when bio-functional slippery beads were used (bottom row) on slippery flat surfaces. Pathogens are also fluorescently labeled and as shown have been captured by beads (small black dots) in the images. Hence, these results demonstrate that only the pathogens are captured without capturing other species, such as red blood cells. Therefore, this provides significant benefits for diagnostic purposes because the non-specific adhesion of red blood cell is not desired whereas the specific attachment of pathogens is being carried out.

**FIG. 14C** shows a graph comparing the percentage of captured red blood cells for S. aureus and E. coli for the three different surfaces. This graph represents the non-specific adhesion/capture of red blood cells on bio-functional slippery flat surface versus bio-functional slippery beads and the control where the surface is not slippery. Bio-functional slippery beads immobilized on a flat slippery surface could successfully capture pathogens (E. coli and S. aureus) but more importantly non-specific adhesion of red blood cells was significantly decreased when slippery beads were used.

As shown, the produced bio-functional slippery beads remained biofunctional and could capture pathogens from fresh whole blood. More importantly nonspecific adhesion of red blood cells was significantly decreased by using bio-functional slippery beads patterned on slippery flat surfaces.

As will be apparent to one of ordinary skill in the art from a reading of this disclosure, aspects of the present disclosure can be embodied in forms other than those specifically disclosed above. The particular embodiments described above are, therefore, to be considered as illustrative and not restrictive. Those skilled in the art will recognize, or be able to ascertain, using no more than routine experimentation, numerous equivalents to the specific embodiments described herein. The scope of the invention is as set forth in the appended claims and equivalents thereof, rather than being limited to the examples contained in the foregoing description.

## Claims

1. An article comprising:
a substrate;
anchoring molecules comprising a head group attached to the substrate and a tail group directly or indirectly attached to the head group, wherein the tail group has an affinity with a lubricating liquid;
a lubricating liquid layer immobilized over the substrate surface comprising said lubricating liquid having an affinity with the tail group of said anchoring molecules, wherein the anchoring molecules and the lubricating liquid are held together by non-covalent attractive forces,
a binding group directly or indirectly secured to the substrate and extending over the surface of the lubricating liquid layer or retained within the lubricating liquid layer having an affinity with a target moiety;
wherein the anchoring molecules and the lubricating liquid layer form a slippery surface configured and arranged for contact with a material that is immiscible with the lubricating liquid,
wherein said slippery surface resists adhesion and repels the material that is immiscible with the lubricating liquid but selectively binds the target moiety to the binding group.

2. The article of claim 1, wherein the head group is covalently attached to the surface; or
wherein the head group is adsorbed onto the surface; and/or
wherein the anchoring layer forms a monomolecular layer on the surface.

3. The article of any preceding claim, wherein the slippery surface is hydrophobic, optionally wherein the tail group comprises a hydrocarbon; or
wherein the surface is hydrophilic; or
wherein the surface is omniphobic, optionally
wherein the tail group is a perfluorocarbon.

4. The article of any preceding claim, wherein said binding group comprises at least one microbe-binding domain; optionally
wherein the microbe-binding domain is indirectly secured to said substrate via at least one linker; further optionally
further comprising a substrate-binding domain adapted for orienting the microbe-binding domain away from said substrate; and/or
further comprising a substrate-binding domain and said at least one linker is located between the microbe-binding domain and the substrate-binding domain; and/or
wherein the linker comprises a portion of a Fc region of an immunoglobulin; optionally
wherein the immunoglobulin is selected from the group consisting of IgG, IgA, IgM, IgD, and IgE.

5. The article of claim 4, wherein the microbe-binding domain is indirectly secured to said substrate via at least one linker and wherein the microbe-binding domain comprises a carbohydrate recognition domain (CRD) or a fragment thereof.

6. The article of claim 5, wherein the CRD or fragment thereof further comprises at least a portion of a carbohydrate-binding protein; optionally
the portion of the carbohydrate-binding protein excludes at least one of complement and coagulation activation region; and/or
wherein the CRD or fragment thereof comprises a lectin, a ficolin, or a fragment thereof; and/or
wherein carbohydrate-binding protein comprises a lectin, a ficolin, or a fragment thereof; optionally
wherein the lectin is C-type lectin, or a fragment thereof; further optionally
wherein the C-type lectin is collectin, or a fragment thereof; and yet further optionally
wherein the collectin is mannose-binding lectin (MBL) or a fragment thereof.

7. The article of claim 5, wherein the CRD comprises SEQ ID NO: 4; and/or
wherein the CRD comprises a neck region of the carbohydrate-binding protein or a fragment thereof.

8. The article of claim 4, wherein said binding group comprises at least one microbe-binding domain, wherein the microbe-binding domain is indirectly secured to said substrate via at least one linker, further comprising a substrate-binding domain adapted for orienting the microbe-binding domain away from said substrate and wherein the substrate-binding domain comprises at least one amine; and/or
wherein the substrate-binding domain comprises at least one oligopeptide comprising an amino acid sequence of AKT.

9. The article of any preceding claim, wherein the binding group comprises a receptor molecule, an adhesion molecule, or a fragment thereof; optionally
wherein the receptor molecule is selected from the group consisting of an extracellular receptor molecule, an intracellular receptor molecule, an immune receptor molecule, a G protein coupled receptor molecule, a virus receptor molecule, and an iron scavenging receptor molecule; and/or
wherein the adhesion molecule is selected from the group consisting of a cell adhesion molecule, a Synaptic cell adhesion molecule (SynCAM), a Neural cell adhesion molecules (NCAM), an Intercellular Cell Adhesion Molecule (ICAM), a Vascular Cell Adhesion Molecule (VCAM), and a Platelet-endothelial Cell Adhesion Molecules (PECAM).

10. The article of any preceding claim, wherein the substrate is selected from the group consisting of glass, polymers, polymers with plasticizers, metals, metal alloys, metalloids, metal oxides, metalloid oxides, non-metals, non-metal oxides, carbon, plastics, paper, ceramics, and combinations thereof.

11. The article of any of the preceding claims, wherein the substrate is selected from the group consisting of poly(dimethyl siloxane) (PDMS), acrylic, polystyrene, tissue-culture polystyrene, metal, polypropylene, acrylic adhesive, silicon wafer, polysulfone, and soda lime glass, the anchoring layer comprises a silyl group covalently attached to a perfluorocarbon tail, and the lubricating liquid layer comprises perfluorocarbon oil, wherein the anchoring layer and the lubricating liquid layer form an omniphobic surface that resists adhesions and repels a material that is immiscible with the lubricating liquid but binds the target moiety to the binding group.

12. A method of preventing adhesion, adsorption, surface-mediated clot formation, or coagulation of a material while selectively binding a target moiety thereon, comprising:
providing a slippery surface comprising a substrate; anchoring molecules comprising a head group attached to the substrate and a tail group directly or indirectly attached to the head group, wherein the tail group has an affinity with a lubricating liquid; a lubricating liquid layer immobilized over the substrate surface comprising said lubricating liquid having an affinity with the tail group of said anchoring molecules, wherein the anchoring molecules and the lubricating liquid are held together by non-covalent attractive forces, a binding group directly or indirectly secured to the substrate and extending over the surface of the lubricating liquid layer or retained within the lubricating liquid layer having an affinity with a target moiety; wherein the anchoring molecules and the lubricating liquid layer form a surface configured and arranged for contact with a material that is immiscible with the lubricating liquid, wherein said slippery surface resists adhesion and repels the material that is immiscible with the lubricating liquid but selectively binds the target moiety to the binding group; and
contacting said material that is immiscible with the lubricating liquid_to the surface and selectively binding said target moiety to the binding group.

13. The method of claim 12, wherein the substrate is treated to activate the surface of the substrate prior to exposure to the anchoring molecules; optionally
wherein activation comprises acid treatment, base treatment, oxidization, ammonization, plasma, heat, electromagnetic, photon, ion, electron, electromagnetic, ultraviolet, microwave treatment or combinations thereof.

14. The method of any of claims 12 to 13, wherein the material that is immiscible with the lubricating liquid is selected from the group consisting of water, whole blood, plasma, serum, sweat, feces, urine, saliva, tears, vaginal fluid, prostatic fluid, gingival fluid, amniotic fluid, intraocular fluid, cerebrospinal fluid, seminal fluid, sputum, ascites fluid, pus, nasopharengal fluid, wound exudate fluid, aqueous humour, vitreous humour, bile, cerumen, endolymph, perilymph, gastric juice, mucus, peritoneal fluid, pleural fluid, sebum, vomit, synthetic fluid (e.g., synthetic blood, hormones, nutrients), and combinations thereof.

15. The method of any of claims 12 to 14, wherein the material that is immiscible with the lubricating liquid_is a solution or suspension containing bacteria selected from the group consisting of *Actinobacillus, Acinetobacter, Aeromonas, Bordetella, Brevibacillus, Brucella, Bacteroides, Burkholderia, Borelia, Bacillus, Campylobacter, Capnocytophaga, Cardiobacterium, Citrobacter, Clostridium, Chlamydia, Eikenella, Enterobacter, Enterococcus, Escherichia, Francisella, Fusobacterium, Flavobacterium, Haemophilus, Helicobacter, Kingella, Klebsiella, Lacobacillus, Legionella, Listeria, Leptospirae, Moraxella, Morganella, Mycoplasma, Mycobacterium, Neisseria, Nocardia, Pasteurella, Proteus, Prevotella, Plesiomonas, Pseudomonas, Providencia, Rickettsia, Salmonella, Serratia, Shigella, Stenotrophomonas, Staphylococcus, Streptococcus (group A), Streptococcus agalactiae (group B), Streptococcus bovis, Streptococcus pneumoniae, Streptomyces, Salmonella, Serratia, Shigella, Spirillum, Treponema, Veillonella, Vibrio, Yersinia, Xanthomonas,* and combinations thereof; and/or
wherein the material that is immiscible with the lubricating liquid_is a solution or suspension containing fungi selected from the group consisting of a member of the genus *Aspergillus, Blastomyces dermatitidis, Candida, Coccidioides immitis, Cryptococcus, Fusarium, Histoplasma capsulatum var. capsulatum, Histoplasma capsulatum var. duboisii, Mucor, Paracoccidioides brasiliensis, Pneumocystis, Saccharomyces, Sporothrix schenckii, Absidia corymbifera; Rhizomucor pusillus, Rhizopus arrhizous,* and combinations thereof; and/or
wherein the material that is immiscible with the lubricating liquid_is a solution or suspension containing viruses selected from the group consisting of cytomegalovirus (CMV), dengue, Epstein-Barr, Hantavirus, human T-cell lymphotropic virus (HTLV I/II), Parvovirus, hepatitides, human papillomavirus (HPV), human immunodeficiency virus (HIV), acquired immunodeficiency syndrome (AIDS), respiratory syncytial virus (RSV), Varicella zoster, West Nile, herpes, polio, smallpox, yellow fever, rhinovirus, coronavirus, Orthomyxoviridae (influenza viruses), and combinations thereof; and/orwherein the material that is immiscible with the lubricating liquid_is a solution or suspension containing particles selected from the group consisting of normal cells, diseased cells, parasitized cells, cancer cells, foreign cells, stem cells, and infected cells, microorganisms, viruses, virus-like particles, bacteria, bacteriophages, proteins, cellular components, cell organelles, cell fragments, cell membranes, cell membrane fragments, viruses, virus-like particles, bacteriophage, cytosolic proteins, secreted proteins, signaling molecules, embedded proteins, nucleic acid/protein complexes, nucleic acid precipitants, chromosomes, nuclei, mitochondria, chloroplasts, flagella, biominerals, protein complexes, and minicells.

16. A method of making an article having a slippery surface that selectively binds a target moiety, the method comprising:
contacting a substrate with anchoring molecules, anchoring molecules comprising a head group attached to the substrate and a tail group directly or indirectly attached to the head group, wherein the tail group has an affinity with a lubricating liquid;
contacting the anchoring layer with a lubricating liquid having an affinity for the tail group to form a lubricating liquid layer immobilized over the substrate; and
providing a binding group directly or indirectly secured to the substrate and extending over the surface of the lubricating liquid layer or retained within the lubricating liquid layer having an affinity with a target moiety;
wherein the anchoring molecules and the lubricating liquid layer form a surface configured and arranged for contact with a material that is immiscible with the lubricating liquid, wherein said slippery surface resists adhesion and repels the material that is immiscible with the lubricating liquid but selectively binds the target moiety to the binding group, and
wherein the anchoring molecules and the lubricating layer are held together by non-covalent attractive forces.

17. The method of claim 16, wherein the substrate comprises a reservoir through which the lubricating liquid is replenished.

## Patentansprüche

1. Artikel, umfassend:
ein Substrat;
Ankermoleküle, die eine an dem Substrat angebrachte Kopfgruppe und eine direkt oder indirekt an der Kopfgruppe angebrachte Schwanzgruppe umfassen, wobei die Schwanzgruppe eine Affinität mit einer Schmierflüssigkeit aufweist;
eine über die Substratoberfläche immobilisierte Schmierflüssigkeitsschicht, umfassend die Schmierflüssigkeit mit einer Affinität mit der Schwanzgruppe der Ankermoleküle, wobei die Ankermoleküle und die Schmierflüssigkeit durch nichtkovalente Anziehungskräfte zusammengehalten werden;
eine Bindungsgruppe, die direkt oder indirekt an dem Substrat gesichert ist und sich über die Oberfläche der Schmierflüssigkeitsschicht erstreckt oder in der Schmierflüssigkeitsschicht mit einer Affinität zu einem Zielanteil gehalten wird;
wobei die Ankermoleküle und die Schmierflüssigkeitsschicht eine rutschige Oberfläche bilden, die für einen Kontakt mit einer Substanz gestaltet und angeordnet ist, die mit der Schmierflüssigkeit nicht mischbar ist,
wobei die rutschige Oberfläche einer Adhäsion widersteht und die mit der Schmierflüssigkeit nicht mischbare Substanz abstößt, jedoch selektiv den Zielanteil mit der Bindungsgruppe bindet.

2. Artikel nach Anspruch 1, wobei die Kopfgruppe kovalent an der Oberfläche angebracht ist; oder wobei die Kopfgruppe auf der Oberfläche anlagert; und/oder
wobei die Ankerschicht eine monomolekulare Schicht auf der Oberfläche bildet.

3. Artikel nach einem der vorstehenden Ansprüche, wobei die rutschige Oberfläche hydrophob ist,
wobei die Schwanzgruppe optional einen Kohlenwasserstoff umfasst; oder wobei die Oberfläche hydrophil ist; oder
wobei die Oberfläche omniphob ist;
wobei die Schwanzgruppe optional ein Perfluorkohlenwasserstoff ist.

4. Artikel nach einem der vorstehenden Ansprüche, wobei die Bindungsgruppe mindestens eine Mikrobenbindungsdomäne umfasst;
wobei optional die Mikrobenbindungsdomäne über mindestens einen Linker indirekt an dem Substrat gesichert ist;
ferner optional eine Substratbindungsdomäne umfassend, welche die Mikrobenbindungsdomäne von dem Substrat weg ausrichten kann; und/oder
ferner umfassend eine Substratbindungsdomäne, und wobei der wenigstens eine Linker zwischen der Mikrobenbindungsdomäne und der Substratbindungsdomäne angeordnet ist; und/oder
wobei der Linker einen Teil einer Fc-Region eines Immunglobulins umfasst;
wobei das Immunglobulin optional ausgewählt ist aus der Gruppe bestehend aus IgG, IgA, IgM, IgD und IgE.

5. Artikel nach Anspruch 4, wobei die Mikrobenbindungsdomäne über wenigstens einen Linker indirekt mit dem Substrat gesichert ist, und wobei die Mikrobenbindungsdomäne eine Kohlenhydraterkennungsdomäne (Carbohydrate Recognition Domain, CRD) oder ein Fragment davon umfasst.

6. Artikel nach Anspruch 5, wobei die CRD oder das Fragment davon ferner mindestens einen Teil eines Kohlenhydratbindungsproteins umfasst; wobei optional
der Teil des Kohlenhydratbindungsproteins wenigstens ein Komplement oder eine Koagulationsaktivierungsregion ausschließt; und/oder
wobei die CRD oder das Fragment davon ein Lektin, ein Ficolin oder ein Fragment davon umfasst; und/oder
wobei das Kohlenhydratbindungsprotein ein Lektin, ein Ficolin oder ein Fragment davon umfasst; wobei optional
das Lektin ein C-Typ-Lektin oder ein Fragment davon ist, wobei ferner optional
das C-Typ-Lektin Collectin oder ein Fragment davon ist; und wobei ferner optional
das Collectin Mannose-Bindungs-Lektin (MBL) oder ein Fragment davon ist.

7. Artikel nach Anspruch 5, wobei die CRD SEQ ID NO: 4 umfasst; und/oder
wobei die CRD eine Halsregion des Kohlenhydratbindungsproteins oder ein Fragment davon umfasst.

8. Artikel nach Anspruch 4, wobei die Bindungsgruppe wenigstens eine Mikrobenbindungsdomäne umfasst, wobei die Mikrobenbindungsdomäne über wenigstens einen Linker indirekt an dem Substrat gesichert ist, ferner umfassend eine Substratbindungsdomäne, welche die Mikrobenbindungsdomäne von dem Substrat weg ausrichten kann, und wobei die Substratbindungsdomäne wenigstens ein Amin umfasst; und/oder
wobei die Substratbindungsdomäne wenigstens ein Oligopeptid umfasst, eine Aminosäuresequenz von AKT umfassend.

9. Artikel nach einem der vorstehenden Ansprüche, wobei die Bindungsgruppe ein Rezeptormolekül, ein Adhäsionsmolekül oder ein Fragment davon umfasst;
wobei das Rezeptormolekül ausgewählt ist aus der Gruppe bestehend aus einem extrazellulären Rezeptormolekül, einem intrazellulären Rezeptormolekül, einem Immunrezeptormolekül, einem an ein G-Protein gekoppelten Rezeptormolekül, einem Virusrezeptormolekül und einem Eisenfängerrezeptormolekül; und/oder
wobei das Adhäsionsmolekül ausgewählt ist aus der Gruppe bestehend aus einem Zelladhäsionsmolekül, einem synaptischen Zelladhäsionsmolekül (SynCAM), Nervenzelladhäsionsmolekülen (NCAM), einem interzellulären Zelladhäsionsmolekül (ICAM), einem vaskulären Zelladhäsionsmolekül (VCAM) und einem Plättchen-Endothel-Zelladhäsionsmolekül (PECAM).

10. Artikel nach einem der vorstehenden Ansprüche, wobei das Substrat ausgewählt ist aus der Gruppe bestehend aus Glas, Polymeren, Polymeren mit Weichmachern, Metallen, Metalllegierungen, Metalloiden, Metalloxiden, Metalloidoxiden, Nicht-Metallen, Nicht-Metalloxiden, Kohlenstoff, Kunststoffen, Papier, Keramikwerkstoffen und Kombinationen davon.

11. Artikel nach einem der vorstehenden Ansprüche, wobei das Substrat ausgewählt ist aus der Gruppe bestehend aus Poly(dimethylsiloxan) (PDMS), Acryl, Polystyrol, Gewebekultur-Polystyrol, Metall, Polypropylen, Acryladhäsionsmittel, Siliziumwafer, Polysulfon und Kalknatronglas, wobei die Ankerschicht eine Silylgruppe umfasst, die kovalent an einem Perfluorkohlenstoffschwanz angebracht ist, und wobei die Schmierflüssigkeitsschicht Perfluorkohlenstofföl umfasst, wobei die Ankerschicht und die Schmierflüssigkeitsschicht eine omniphobe Oberfläche bilden, die Adhäsionen widersteht und eine Substanz abstößt, die mit der Schmiermittelflüssigkeit nicht mischbar ist, jedoch den Zielanteil an die Bindungsgruppe bindet.

12. Verfahren zur Prävention von Adhäsion, Adsorption, oberflächenvermittelter Klumpenbildung oder Koagulation einer Substanz, während selektiv ein Zielanteil daran gebunden wird, umfassend:
Bereitstellen einer rutschigen Oberfläche, umfassend ein Substrat; Ankermoleküle, die eine an dem Substrat angebrachte Kopfgruppe und eine direkt oder indirekt an der Kopfgruppe angebrachte Schwanzgruppe umfassen, wobei die Schwanzgruppe eine Affinität mit einer Schmierflüssigkeit aufweist; eine über die Substratoberfläche immobilisierte Schmierflüssigkeitsschicht, umfassend die Schmierflüssigkeit mit einer Affinität mit der Schwanzgruppe der Ankermoleküle, wobei die Ankermoleküle und die Schmierflüssigkeit durch nichtkovalente Anziehungskräfte zusammengehalten werden; eine Bindungsgruppe, die direkt oder indirekt an dem Substrat gesichert ist und sich über die Oberfläche der Schmierflüssigkeitsschicht erstreckt oder in der Schmierflüssigkeitsschicht mit einer Affinität zu einem Zielanteil gehalten wird; wobei die Ankermoleküle und die Schmierflüssigkeitsschicht eine rutschige Oberfläche bilden, die für einen Kontakt mit einer Substanz gestaltet und angeordnet ist, die mit der Schmierflüssigkeit nicht mischbar ist,
wobei die rutschige Oberfläche einer Adhäsion widersteht und die mit der Schmierflüssigkeit nicht mischbare Substanz abstößt, jedoch selektiv den Zielanteil mit der Bindungsgruppe bindet; und
Inkontaktbringen der Substanz, die mit der Schmierflüssigkeit nicht mischbar ist, mit der Oberfläche und selektive Bindung des Zielanteils an die Bindungsgruppe.

13. Verfahren nach Anspruch 12, wobei das Substrat behandelt ist, um die Oberfläche des Substrats zu aktivieren, bevor sie den Ankermolekülen ausgesetzt wird;
wobei die Aktivierung optional Säurebehandlung, Basenbehandlung, Oxidation, Ammonisierung, Plasma-, Wärme-, elektromagnetische, Photonen-, Ionen-, Elektronen-, elektromagnetische, UV-, Mikrowellenbehandlung oder Kombinationen davon umfasst.

14. Verfahren nach Anspruch 12 oder 13, wobei die Substanz, die mit der Schmierflüssigkeit nicht mischbar ist, ausgewählt ist aus der Gruppe bestehend aus Wasser, Vollblut, Plasma, Serum, Schweiß, Fäzes, Urin, Speichel, Tränen, Vaginalflüssigkeit, Prostataflüssigkeit, gingivaler Flüssigkeit, Fruchtwasser, intraokularer Flüssigkeit, Zerebrospinalflüssigkeit, Samenflüssigkeit, Sputum, Bauchwasser, Eiter, Nasopharyngealflüssigkeit, Wundnässe, Kammerwasser, Glaskörper, Gallenflüssigkeit, Cerumen, Endolymphe, Perilymphe, Magensaft, Schleim, Peritonealflüssigkeit, Pleuralflüssigkeit, Sebum, Erbrochenem, synthetischer Flüssigkeit (z.B. synthetisches Blut, Hormone, Nährstoffe) und Kombinationen davon.

15. Verfahren nach einem der Ansprüche 12 bis 14, wobei die Substanz, die mit der Schmierflüssigkeit nicht mischbar ist, eine Lösung oder Suspension ist, die Bakterien enthält, ausgewählt aus der Gruppe bestehend aus *Actinobacillus, Acinetobacter, Aeromonas, Bordetella, Brevibacillus, Brucella, Bacteroides, Burkholderia, Borelia, Bacillus, Campylobacter, Capnocytophaga, Cardiobacterium, Citrobacter, Clostridium, Chlamydia, Eikenella, Enterobacter, Enterococcus, Escherichia, Francisella, Fusobacterium, Flavobacterium, Haemophilus, Helicobacter, Kingella, Klebsiella, Lacobacillus, Legionella, Listeria, Leptospirae, Moraxella, Morganella, Mycoplasma, Mycobacterium, Neisseria, Nocardia, Pasteurella, Proteus, Prevotella, Plesiomonas, Pseudomonas, Providencia, Rickettsia, Salmonella, Serratia, Shigella, Stenotrophomonas, Staphylococcus, Streptococcus (Gruppe A), Streptococcus agalactiae (Gruppe B), Streptococcus bovis, Streptococcus pneumoniae, Streptomyces, Salmonella, Serratia, Shigella, Spirillum, Treponema, Veillonella, Vibrio, Yersinia, Xanthomonas* und Kombinationen davon; und/oder
wobei die Substanz, die mit der Schmierflüssigkeit nicht mischbar ist, eine Lösung oder Suspension ist, die Pilze enthält, ausgewählt aus der Gruppe bestehend aus einem Mitglied des Genus *Aspergillus, Blastomyces dermatitidis, Candida, Coccidioides immitis, Cryptococcus, Fusarium, Histoplasma capsulatum var. capsulatum, Histoplasma capsulatum var. duboisii, Mucor, Paracoccidioides brasiliensis, Pneumocystis, Saccharomyces, Sporothrix schenckii, Absidia corymbifera; Rhizomucor pusillus, Rhizopus arrhizous* und Kombinationen davon; und/oder
wobei die Substanz, die mit der Schmierflüssigkeit nicht mischbar ist, eine Lösung oder Suspension ist, die Viren enthält, ausgewählt aus der Gruppe bestehend aus Cytomegalievirus (CMV), Dengue-Virus, Epstein-Barr-Virus, Hantavirus, humanem T-lymphotropem Virus (HTLVI/II), Parvovirus, Hepatitiden, humanem Papillomavirus (HPV), humanem Immundefizienz-Virus (HIV), Acquired Immunodeficiency Syndrome (AIDS), respiratorischem Syncytial-Virus (RSV), Varizella-Zoster-Virus, West-Nil-Virus, Herpes, Polio, Pocken, Gelbfieber, Rhinovirus, Coronavirus, Orthomyxoviridae (Influenza-Viren) und Kombinationen davon; und/oder wobei die Substanz, die mit der Schmierflüssigkeit nicht mischbar ist, eine Lösung oder Suspension ist, die Viren enthält, ausgewählt aus der Gruppe bestehend aus gesunden Zellen, erkrankten Zellen, parasitierten Zellen, Krebszellen, körperfremden Zellen, Stammzellen und infizierten Zellen, Mikroorganismen, Viren, Virus-ähnlichen Partikeln, Bakterien, Bakteriophagen, Proteinen, Zellbestandteilen, Zellfragmenten, Zellmembranen, Zellmembranfragmenten, Viren, Virus-ähnlichen Partikeln, Bakteriophagen, zytosolischen Proteinen, sezernierten Proteinen, Signalmolekülen, eingebetteten Proteinen, Nukleinsäure/Protein-Komplexen, Nukleinsäurefällungen, Chromosomen, Kernen, Mitochondrien, Chloroplasten, Flagellen, Biomineralen, Proteinkomplexen und Minizellen.

16. Verfahren zur Herstellung eines Artikels mit einer rutschigen Oberfläche, die selektiv einen Zielanteil bindet,
wobei das Verfahren folgendes umfasst:
Inkontaktbringen eines Substrats mit Ankermolekülen, wobei die Ankermoleküle eine Kopfgruppe umfassen, die an dem Substrat angebracht ist, und eine direkt oder indirekt an der Kopfgruppe angebrachte Schwanzgruppe, wobei die Schwanzgruppe eine Affinität mit einer Schmierflüssigkeit aufweist;
Inkontaktbringen der Ankerschicht mit einer Schmierflüssigkeit mit einer Affinität für die Schwanzgruppe, um eine über dem Substrat immobilisierte Schmierflüssigkeitsschicht zu bilden; und
Bereitstellen einer Bindungsgruppe, die direkt oder indirekt an dem Substrat gesichert ist und sich über die Oberfläche der Schmiermittelflüssigkeit erstreckt oder in der Schmierflüssigkeitsschicht mit einer Affinität zu einem Zielanteil gehalten wird;
wobei die Ankermoleküle und die Schmierflüssigkeitsschicht eine Oberfläche bilden, die für einen Kontakt mit einer Substanz gestaltet und angeordnet ist, die mit der Schmierflüssigkeit nicht mischbar ist, wobei die rutschige Oberfläche
einer Adhäsion widersteht und die mit der Schmierflüssigkeit nicht mischbare Substanz abstößt, jedoch selektiv den Zielanteil mit der Bindungsgruppe bindet, und
wobei die wobei die Ankermoleküle und die Schmierflüssigkeit durch nichtkovalente Anziehungskräfte zusammengehalten werden.

17. Verfahren nach Anspruch 16, wobei das Substrat einen Speicher umfasst, durch welchen die Schmierflüssigkeit aufgefüllt wird.

## Revendications

1. Article, comprenant :
un substrat ;
des molécules d'ancrage comprenant un groupe de tête lié au substrat et un groupe de queue directement ou indirectement lié au groupe de tête, le groupe de queue ayant une affinité avec un liquide de lubrification ;
une couche de liquide de lubrification immobilisée sur la surface du substrat comprenant ledit liquide de lubrification ayant une affinité avec le groupe de queue desdites molécules d'ancrage, les molécules d'ancrage et le liquide de lubrification étant maintenus ensemble par des forces d'attraction non covalentes,
un groupe de liaison directement ou indirectement fixé au substrat et s'étendant sur la surface de la couche de liquide de lubrification ou maintenu dans la couche de liquide de lubrification ayant une affinité avec une fraction cible ;
les molécules d'ancrage et la couche de liquide de lubrification formant une surface glissante conçue et disposée pour un contact avec un matériau qui est non miscible avec le liquide de lubrification,
ladite surface glissante résistant à l'adhérence et repoussant le matériau qui est non miscible avec le liquide de lubrification mais liant de façon sélective la fraction cible au groupe de liaison.

2. Article selon la revendication 1, le groupe de tête étant lié de façon covalente à la surface ; ou
le groupe de tête étant adsorbé sur la surface ; et/ou
la couche d'ancrage formant une couche monomoléculaire sur la surface.

3. Article selon l'une quelconque des revendications précédentes, la surface glissante étant hydrophobe, éventuellement
le groupe de queue comprenant un hydrocarbure ; ou
la surface étant hydrophile ; ou
la surface étant omniphobe, éventuellement
le groupe de queue étant un perfluorocarbure.

4. Article selon l'une quelconque des revendications précédentes, ledit groupe de liaison comprenant au moins un domaine de liaison de microbe ; éventuellement
le domaine de liaison de microbe étant indirectement fixé audit substrat par l'intermédiaire d'au moins un lieur ; éventuellement
comprenant en outre un domaine de liaison de substrat conçu pour orienter le domaine de liaison de microbe à l'opposé dudit substrat ; et/ou
comprenant en outre un domaine de liaison de substrat et ledit au moins un lieur étant situé entre le domaine de liaison de microbe et le domaine de liaison de substrat ; et/ou
le lieur comprenant une partie d'une région Fc d'une immunoglobuline ; éventuellement
l'immunoglobuline étant choisie dans le groupe constitué par IgG, IgA, IgM, IgD et IgE.

5. Article selon la revendication 4, le domaine de liaison de microbe étant indirectement fixé audit substrat par l'intermédiaire d'au moins un lieur et le domaine de liaison de microbe comprenant un domaine de reconnaissance de glucide (CRD) ou un fragment de celui-ci.

6. Article selon la revendication 5, le CRD ou fragment de celui-ci comprenant en outre au moins une partie d'une protéine de liaison aux glucides ; éventuellement
la partie de la protéine de liaison aux glucides excluant au moins l'une parmi les régions d'activation de coagulation ou de complément ; et/ou
le CRD ou un fragment de celui-ci comprenant une lectine, une ficoline, ou un fragment de celles-ci ; et/ou
une protéine de liaison aux glucides comprenant une lectine, une ficoline, ou un fragment de celles-ci ; éventuellement
la lectine étant une lectine de type C, ou un fragment de celle-ci ; éventuellement
la lectine de type C étant une collectine, ou un fragment de celle-ci ; et encore éventuellement
la collectine étant une lectine de liaison au mannose (MBL) ou un fragment de celle-ci.

7. Article selon la revendication 5, le CRD comprenant SEQ ID No 4 ; et/ou
le CRD comprenant une région de col de la protéine de liaison aux glucides ou un fragment de celle-ci.

8. Article selon la revendication 4, ledit groupe de liaison comprenant au moins un domaine de liaison de microbe, le domaine de liaison de microbe étant indirectement fixé audit substrat par l'intermédiaire d'au moins un lieur, comprenant en outre un domaine de liaison de substrat conçu pour orienter le domaine de liaison de microbe à l'opposé dudit substrat et le domaine de liaison de substrat comprenant au moins une amine ; et/ou
le domaine de liaison de substrat comprenant au moins un oligopeptide comprenant une séquence d'acides aminés de Akt.

9. Article selon l'une quelconque des revendications précédentes, le groupe de liaison comprenant une molécule réceptrice, une molécule d'adhérence, ou un fragment de celles-ci ; éventuellement
la molécule réceptrice étant choisie dans le groupe constitué par une molécule réceptrice extracellulaire, une molécule réceptrice intracellulaire, une molécule réceptrice immunitaire, une molécule réceptrice couplée à la protéine G, une molécule réceptrice de virus, et une molécule réceptrice de piégeage de fer ; et/ou
la molécule d'adhérence étant choisie dans le groupe constitué par une molécule d'adhérence cellulaire, une molécule d'adhérence cellulaire synaptique (SynCAM), une molécule d'adhérence cellulaire neurale (NCAM), une molécule d'adhérence cellulaire intercellulaire (ICAM), une molécule d'adhérence des cellules vasculaire (VCAM), et une molécule d'adhérence des cellules plaquettaires-endothéliales (PECAM).

10. Article selon l'une quelconque des revendications précédentes, le substrat étant choisi dans le groupe constitué par le verre, les polymères, les polymères avec plastifiants, les métaux, les alliages métalliques, les métalloïdes, les oxydes métalliques, les oxydes métalloïdes, les non-métaux, les oxydes non-métalliques, le carbone, le plastique, le papier, la céramique, et des combinaisons de ceux-ci.

11. Article selon l'une quelconque des revendications précédentes, le substrat étant choisi dans le groupe constitué par poly(diméthyl siloxane) (PDMS), acrylique, polystyrène, polystyrène de culture de tissus, métal, polypropylène, adhésif acrylique, tranche de silicium, polysulfone, et verre sodo-calcique, la couche d'ancrage comprenant un groupe silyle lié de façon covalente à une queue de perfluorocarbone, et la couche de liquide de lubrification comprenant de l'huile de perfluorocarbone, la couche d'ancrage et la couche de liquide de lubrification formant une surface omniphobe qui résiste aux adhérences et repousse un matériau qui est non miscible avec le liquide de lubrification mais lie la fraction cible au groupe de liaison.

12. Procédé pour empêcher l'adhérence, l'adsorption, la formation de caillots médiée en surface, ou la coagulation d'un matériau tout en liant sélectivement une fraction cible dessus, comprenant les étapes consistant à :
fournir une surface glissante comprenant un substrat ; des molécules d'ancrage comprenant un groupe de tête lié au substrat et un groupe de queue directement ou indirectement lié au groupe de tête, le groupe de queue ayant une affinité avec un liquide de lubrification ; une couche de liquide de lubrification immobilisée sur la surface du substrat comprenant ledit liquide de lubrification ayant une affinité avec le groupe de queue desdites molécules d'ancrage, les molécules d'ancrage et le liquide de lubrification étant maintenus ensemble par des forces d'attraction non covalentes, un groupe de liaison directement ou indirectement fixé au substrat et s'étendant sur la surface de la couche de liquide de lubrification ou maintenu dans la couche de liquide de lubrification ayant une affinité avec une fraction cible ; les molécules d'ancrage et la couche de liquide de lubrification formant une surface conçue et disposée pour être en contact avec un matériau qui est non miscible avec le liquide de lubrification, ladite surface glissante résistant à l'adhérence et repoussant le matériau qui est non miscible avec le liquide de lubrification mais liant sélectivement la fraction cible au groupe de liaison ; et
mettre en contact ledit matériau qui est non miscible avec le liquide de lubrification sur la surface et lier sélectivement ladite fraction cible au groupe de liaison.

13. Procédé selon la revendication 12, le substrat étant traité pour activer la surface du substrat avant exposition aux molécules d'ancrage ; éventuellement
l'activation comprenant un traitement acide, un traitement basique, une oxydation, une ammonisation, un traitement au plasma, thermique, électromagnétique, aux photons, aux ions, aux électrons, électromagnétique, ultraviolet, aux micro-ondes ou des combinaisons de ceux-ci.

14. Procédé selon la revendication 12 ou 13, le matériau qui est non miscible avec le liquide de lubrification étant choisi dans le groupe constitué par eau, sang entier, plasma, sérum, sueur, excréments, urine, salive, larmes, sécrétions vaginales, liquide prostatique, liquide gingival, liquide amniotique, liquide intraoculaire, liquide céphalorachidien, liquide séminal, crachats, liquide d'ascite, pus, fluide nasopharengal, fluide d'exsudat de plaie, humeur aqueuse, humeur vitrée, bile, cérumen, endolymphe, périlymphe, suc gastrique, mucus, liquide péritonéal, liquide pleural, sébum, vomi, fluide synthétique (p. ex., sang synthétique, hormones, éléments nutritifs), et combinaisons de ceux-ci.

15. Procédé selon l'une quelconque des revendications 12 à 14, le matériau qui est non miscible avec le liquide de lubrification étant une solution ou une suspension contenant les bactéries sélectionnées dans le groupe constitué par *Actinobacillus, Acinetobacter, Aeromonas, Bordetella, Brevibacillus, Brucella, Bacteroides, Burkholderia, Borelia, Bacillus, Campylobacter, Capnocytophaga, Cardiobacterium, Citrobacter, Clostridium, Chlamydia, Eikenella, Enterobacter, Enterococcus, Escherichia, Francisella, Fusobacterium, Flavobacterium, Haemophilus, Helicobacter, Kingella, Klebsiella, Lacobacillus, Legionella, Listeria, Leptospirae, Moraxella, Morganella, Mycoplasma, Mycobacterium, Neisseria, Nocardia, Pasteurella, Proteus, Prevotella, Plesiomonas, Pseudomonas, Providencia, Rickettsia, Salmonella, Serratia, Shigella, Stenotrophomonas, Staphylococcus, Streptococcus (groupe A), Streptococcus agalactiae (groupe B), Streptococcus bovis, Streptococcus pneumoniae, Streptomyces, Salmonella, Serratia, Shigella, Spirillum, Treponema, Veillonella, Vibrio, Yersinia, Xanthomonas,* et des combinaisons de celles-ci ; et/ou
le matériau qui est non miscible avec le liquide de lubrification étant une solution ou une suspension contenant des champignons choisis dans le groupe constitué un un membre du genre *Aspergillus, Blastomyces dermatitidis, Candida, Coccidioides immitis, Cryptococcus, Fusarium, Histoplasma capsulatum var. capsulatum, Histoplasma capsulatum var. duboisii, Mucor, Paracoccidioides brasiliensis, Pneumocystis, Saccharomyces, Sporothrix schenckii, Absidia corymbifera; Rhizomucorpusillus, Rhizopus arrhizous,* et des combinaisons de ceux-ci ; et/ou
le matériau qui est non miscible avec le liquide de lubrification étant une solution ou une suspension contenant des virus choisis dans le groupe constitué par cytomégalovirus (CMV), dengue, Epstein-Barr, Hantavirus, virus humain lymphotrope des cellules T (HTLV I/II), parvovirus, hépatitides, papillovirus humain (VPH), virus d'immunodéficience humaine (VIH), syndrome d'immunodéficience acquise (SIDA), virus respiratoire syncytial (VRS), varicelle-zona, Nil occidental, herpès, polio, variole, fièvre jaune, rhinovirus, coronavirus, orthomyxoviridés (virus de l'influenza), et des combinaisons de ceux-ci ; et/ou matériau qui est non miscible avec le liquide de lubrification étant une solution ou une suspension contenant des particules choisies dans le groupe constitué par cellules normales, cellules malades, cellules parasitées, cellules cancéreuses, cellules étrangères, cellules-souches, et cellules infectées, micro-organismes, virus, particules de type viral, bactéries, bactériophages, protéines, composants cellulaires, organites cellulaires, fragments cellulaires, membranes cellulaires, fragments de membrane cellulaire, virus, particules de type viral, bactériophage, protéines cytosoliques, protéines sécrétées, molécules de signalisation, protéines intégrées, complexes acide nucléique/protéine, précipitants des acides nucléiques, chromosomes, noyaux, mitochondries, chloroplastes, flagelles, biominéraux, complexes protéiques et minicellules.

16. Procédé de fabrication d'un article ayant une surface glissante qui lie sélectivement une fraction cible, le procédé comprenant les étapes consistant :
mettre en contact un substrat avec des molécules d'ancrage, les molécules d'ancrage comprenant un groupe de tête lié au substrat et un groupe de queue directement ou indirectement lié au groupe de tête, le groupe de queue ayant une affinité avec un liquide de lubrification ;
mettre en contact la couche d'ancrage avec un liquide de lubrification ayant une affinité pour le groupe de queue pour former une couche de liquide de lubrification immobilisée sur le substrat ; et
fournir un groupe de liaison directement ou indirectement fixé au substrat et s'étendant sur la surface de la couche de liquide de lubrification ou maintenu dans la couche de liquide de lubrification ayant une affinité avec une fraction cible ;
les molécules d'ancrage et la couche de liquide de lubrification formant une surface conçue et disposée pour entrer en contact avec un matériau qui est non miscible avec le liquide de lubrification, ladite surface glissante résistant à l'adhérence et repoussant le matériau qui est non miscible avec le liquide de lubrification mais liant sélectivement la fraction cible au groupe de liaison, et
les molécules d'ancrage et la couche lubrifiante étant maintenues ensemble par des forces d'attractions non covalentes.

17. Procédé selon la revendication 16, le substrat comprenant un réservoir à travers lequel le liquide de lubrification est réapprovisionné.
